# EUROPÄISCHE PATENTSCHRIFT

(11) **EP 3 638 666 B1**
(45) Veröffentlichungstag und Bekanntmachung des Hinweises auf die Patenterteilung: **21.07.2021**
(21) Anmeldenummer: 18728669.5
(22) Anmeldetag: 11.06.2018
(51) Int. Cl.: C07D 413/12, A01N 43/80

(54) **HERBIZID WIRKSAME 3-PHENYLISOXAZOLIN-5-CARBOXAMIDE VON TETRAHYDRO- UND DIHYDROFURANCARBONSÄUREAMIDEN**
HERBICIDAL 3-PHENYLISOXAZOLINE-5-CARBOXAMIDES OF TETRAHYDRO AND DIHYDROFURAN CARBOXYLIC ACIDS AMIDES
HERBICIDES EFFICACES A BASE DE 3-PHENYLISOXAZOLINE-5-CARBOXAMIDES D'AMIDES D'ACIDE CARBOXYLIQUE DE TÉTRAHYDRO ET DIHYDROFURANE

(30) Priorität: 13.06.2017 EP 17175780
(43) Veröffentlichungstag der Anmeldung: 22.04.2020
(73) Patentinhaber: Bayer Aktiengesellschaft, 51373 Leverkusen (DE); Bayer CropScience Aktiengesellschaft, 40789 Monheim am Rhein (DE)
(72) Erfinder: PETERS, Olaf, 99891 Tabarz (DE); HAAF, Klaus, Bernhard, 65779 Kelkheim (DE); BOJACK, Guido, 65207 Wiesbaden-Naurod (DE); LAW, Katherine, Rose, 65812 Bad Soden (DE); MACHETTIRA, Anu, Bheemaiah, 60326 Frankfurt am Main (DE); DIETRICH, Hansjörg, 65835 Liederbach am Taunus (DE); GATZWEILER, Elmar, 61231 Bad Nauheim (DE); ROSINGER, Christopher, Hugh, 65719 Hofheim (DE); ASMUS, Elisabeth, 63768 Hösbach (DE)
(74) Vertreter: BIP Patents
(86) Internationale Anmeldenummer: PCT/EP2018/065334
(87) Internationale Veröffentlichungsnummer: WO 2018/228986

(56) Entgegenhaltungen:
- WO-A1-2012/130798

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Speziell betrifft sie substituierte 3-Phenylisoxazolin-5-carboxamide und -5-thioamide von Tetrahydro-und Dihydrofurancarbonsäureamiden, Verfahren zu ihrer Herstellung und ihre Verwendung als Herbizide.

WO1995/014681 A1, WO1995/014680 A1, WO 2008/035315 A1, WO2005/051931 A1 und WO2005/021515 A1 beschreiben unter anderen jeweils 3-Phenylisoxazolin-5-carbox-amide, die am Phenylring in 3- und 4-Position durch Alkoxy-Reste substiuiert sind. WO1998/057937 A1 beschreibt unter anderem Verbindungen, die am Phenylring in 4-Position durch einen Alkoxy-Rest substiuiert sind. WO2006/016237 A1 beschreibt unter anderen jeweils solche Verbindungen, die am Phenylring durch einen Amido-Rest substiuiert sind. Die in vorstehend genannten Dokumenten beschriebenen Verbindungen werden darin als pharmakologisch wirksam offenbart.

In WO2005/021516 A1 werden 3-(([3-(3-tert-Butylphenyl)-5-ethyl-4,5-dihydro-1,2-oxazol-5-yl]carbonyl)amino)-5-fluor-4-oxopentansäure und 3-(([3-(3-tert-Butylphenyl)-5-isopropyl-4,5-dihydro-1,2-oxazol-5-yl]carbonyl)amino)-5-fluor-4-oxopentansäure als pharmakologisch wirksame Verbindungen offenbart.

Aus DE 4026018 A1, EP 0 520 371 A2 und DE 4017665 sind 3-Phenylisoxazolin-5-carboxamide bekannt, die in 5-Position des Isoxazolin-Rings ein Wasserstoffatom tragen. Diese Verbindungen werden dort als agrochemisch wirksame Safener beschrieben, d.h. als Verbindungen, die die unerwünschte herbizide Wirkung von Herbiziden gegenüber Kulturpflanzen aufheben. Eine herbizide Wirkung dieser Verbindungen ist nicht offenbart. Die prioritätsältere nicht vorveröffentlichte europäische Patentanmeldung Nr. 10170238 offenbart herbizid und fungizid wirksame 3-Phenylisoxazolin-5-carboxamide und 3-Phenylisoxazolin-5-thioamide, die in 5-Position des Isoxazolin-Rings ein Wasserstoffatom tragen. Aus Monatshefte Chemie (2010) 141, 461 und Letters in Organic Chemistry (2010), 7, 502 sind ebenfalls 3-Phenylisoxazolin-5-carboxamide bekannt, die in 5-Position des Isoxazolin-Rings ein Wasserstoffatom tragen. Für einige der genannten Verbindungen wird eine fungizide, jedoch keine herbizide Wirkung offenbart.

WO 2014/048827 beschreibt die herbizide Wirkung von 3-Phenylisoxazolin-5-carbonsäuren, -5-carbonsäureestern, 5-carbaldehyden und 5-nitrilen.

WO 2014/048853 offenbart Isoxazolin-5-carboxamide und -5-thioamide mit Heterocyclen in 3-Position (herbizid und fungizid),

WO 2014/048940 mit Chinolin als speziellem Heterocyclus in der 3-Position (fungizid), WO 2014/048882 mit Alkoxy als speziellen Rest in der 5-Position.

WO 2014/048882 offenbart Isoxazolin-carboxamide mit Alkoxy als speziellen Rest in der 5-Position.

In WO 2012/130798 werden herbizid und fungizid aktive 3-Phenylisoxazolin-5-carboxamide und -5-thioamide von substituierten Heterocyclen beschrieben.

Die herbizide Wirkung dieser bekannten Verbindungen, insbesondere bei niedrigen Aufwandmengen, bzw. deren Verträglichkeit gegenüber Kulturpflanzen bleiben verbesserungswürdig.

Aus den genannten Gründen besteht weiterhin ein Bedarf nach wirkungsstarken Herbiziden und/oder Pflanzenwachstumsregulatoren für die selektive Anwendung in Pflanzenkulturen oder die Anwendung auf Nichtkulturland, wobei diese Wirkstoffe vorzugsweise weitere vorteilhafte Eigenschaften in der Anwendung haben sollten, wie zum Beispiel eine verbesserte Verträglichkeit gegenüber Kulturpflanzen.

Ein Gegenstand der vorliegenden Erfindung ist daher die Bereitstellung von Verbindungen mit herbizider Wirkung (Herbizide), die bereits bei relativ niedrigen Aufwandmengen gegen wirtschaftlich wichtige Schadpflanzen hochwirksam sind und vorzugsweise bei guter Wirksamkeit gegen Schadpflanzen selektiv in Kulturpflanzen eingesetzt werden können, und dabei vorzugsweise eine gute Verträglichkeit gegenüber Kulturpflanzen zeigen. Bevorzugt sollten diese herbiziden Verbindungen insbesondere effektiv und effizient gegen ein breites Spektrum an Ungräsern sein, und vorzugsweise zusätzlich eine gute Wirksamkeit gegen viele Unkräuter aufweisen.

Neben einer herbiziden Wirkung weisen zahlreiche Verbindung der Formel (I) auch fungizide Wirkung auf, die jedoch nur gering ausgeprägt ist.

Überraschenderweise wurde nun gefunden, dass die nachfolgend definierten 3-Phenylisoxazolin-5-carboxamide von Tetrahydro- und Dihydrofurancarbonsäureamide der Formel (I) und deren Salze eine ausgezeichnete herbizide Wirksamkeit gegen ein breites Spektrum wirtschaftlich wichtiger mono- und dikotyler annueller Schadpflanzen aufweisen.

Gegenstand der vorliegenden Erfindung sind daher Verbindungen der allgemeinen Formel (I) 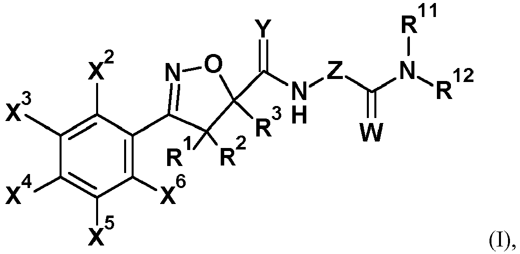 und deren agrochemisch verträglichen Salze, in welchen
- R¹ und R²: unabhängig voneinander jeweils Wasserstoff, Halogen oder Cyano, oder durch jeweils m Reste aus der Gruppe bestehend aus Halogen und Cyano substituiertes (C₁-C₄)-Alkyl und (C₁-C₄)-Alkoxy bedeuten;
- R³: durch jeweils m Reste aus der Gruppe bestehend aus Halogen, Cyano, (C₁-C₅)-Alkoxy und Hydroxy substituiertes (C₁-C₅)-Alkyl, (C₃-C₆)-Cycloalkyl, (C₂-C₅)-Alkenyl, (C₂-C₅)-Alkinyl oder (C₁-C₅)-Alkoxy bedeutet;
- Y: Sauerstoff oder Schwefel bedeutet;
- W: Sauerstoff oder Schwefel bedeutet;
- Z: für einen vollständig gesättigten oder teilweise gesättigten Furanring steht, der mit k Resten der Gruppe R¹⁰ substituiert ist 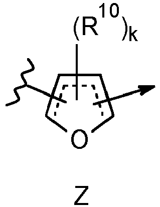 worin der Pfeil jeweils für eine Bindung zur Gruppe C=W der Formel (I) steht;
- R¹⁰: Halogen, Cyano oder CO₂R₇ bedeutet, oder durch jeweils m Reste aus der Gruppe bestehend aus Fluor und Chlor substituiertes (C₁-C₂)-Alkyl oder (C₁-C₂)-Alkoxy bedeutet;
- R¹¹, R¹²: unabhängig voneinander Wasserstoff, Cyano, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, gegebenenfalls substituiertes Aryl, gegebenenfalls substituiertes Heteroaryl und gegebenenfalls substituiertes Heterocyclyl bedeuten, oder durch jeweils m Reste aus der Gruppe bestehend aus Halogen, Cyano, Nitro, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, gegebenenfalls substituiertes Aryl, gegebenenfalls substituiertes Heteroaryl und gegebenenfalls substituiertes Heterocyclyl, substituiertes (C₁-C₂)-Alkyl, (C₃-C₈)-Cycloalkyl, (C₃-C₇)-Cycloalkyl-(C₁-C₇)-Alkyl, (C₂-C₁₂)-Alkenyl, (C₅-C₇)-Cycloalkenyl oder (C₂-C₁₂)-Alkinyl bdeuten, oder
- R¹¹ und R¹²: mit dem Stickstoffatom, an das sie gebunden sind, einen gegebenenfalls ein- bis sechsfach durch Reste aus der Gruppe bestehend aus Halogen, Cyano, Nitro, (C₁-C₆)-Alkyl, Halogen-(C₁-C₆)-Alkyl, Oxo, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸ substituierten, gesättigten, teilweise oder vollständig ungesättigten fünf-, sechs- oder siebengliedrigen Ring bilden, der neben diesem Stickstoffatom r Kohlenstoffatome, n Sauerstoffatome, p Schwefelatome und p Elemente aus der Gruppe bestehend aus NR⁷ und NCOR⁷ als Ringatome enthält;
- X², X⁴ und X⁶: unabhängig voneinander jeweils Wasserstoff, Halogen oder Cyano, oder jeweils durch m Reste aus der Gruppe bestehend aus Fluor, Chlor, Brom und (C₁-C₂)-Alkoxy substituiertes (C₁-C₂)-Alkyl bedeuten;
- X³ und X⁵: unabhängig voneinander Wasserstoff, Fluor, Chlor, Brom, Iod, Hydroxy, Cyano, Nitro, S(O)ₙR⁶ oder CO₂R⁷, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl bedeuten;
- R⁵: durch jeweils m Reste aus der Gruppe bestehend aus Halogen, Cyano und Hydroxy substituiertes (C₁-C₈)-Alkyl, (C₃-C₆)-Cycloalkyl oder Aryl bedeutet;
- R⁶: Wasserstoff oder R⁵ bedeutet;
- R⁷: Wasserstoff, oder durch jeweils m Reste aus der Gruppe bestehend aus Halogen, Cyano und (C₁-C₂)-Alkoxy substituiertes (C₁-C₆)-Alkyl, (C₃-C₆)-Cycloalkyl, (C₃-C₄)-Alkenyl oder (C₃-C₄)-Alkinyl bedeutet;
- R⁸: Wasserstoff, oder durch jeweils m Reste aus der Gruppe bestehend aus Halogen, Cyano und (C₁-C₂)-Alkoxy substituiertes (C₁-C₈)-Alkyl, (C₃-C₆)-Cycloalkyl, (C₃-C₈)-Alkenyl oder (C₃-C₈)-Alkinyl bedeutet;
- k: die Laufzahl 0, 1 oder 2 beträgt;
- m: die Laufzahl 0, 1, 2, 3, 4 oder 5 beträgt;
- n: die Laufzahl 0, 1 oder 2 beträgt;
- p: die Laufzahl 0 oder 1 beträgt; und
- r: die Laufzahl 3, 4, 5 oder 6 beträgt.

Alkyl bedeutet gesättigte, geradkettige oder verzweigte Kohlenwasserstoffreste mit der jeweils angegebenen Anzahl von Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Di-methylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1 -Trifluorprop-2-yl.

Alkenyl bedeutet ungesättigte, geradkettige oder verzweigte Kohlenwasserstoffreste mit der jeweils angegebenen Anzahl von Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit der jeweils angegebenen Anzahl von Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Cycloalkyl bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind.

Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1 -yl und Adamantan-2-yl.

Im Falle von substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

Cycloalkenyl bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Alkoxy bedeutet gesättigte, geradkettige oder verzweigte Alkoxyreste mit der jeweils angegebenen Anzahl von Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Durch Halogen substitiertes Alkoxy bedeutet geradkettige oder verzweigte Alkoxyreste mit der jeweils angegebenen Anzahl von Kohlenstoffatomen, wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlor-fluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-1,2-difluorethoxy, 2,2-Dichlor-2-fluorethoxy, 2,2,2-Trichlorethoxy, Pentafluor-ethoxy und 1,1,1-Trifluorprop-2-oxy.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst.

Die oben aufgeführten Aryle sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfaßt, wie beispielsweise 8-Aza-bicyclo[3.2.1]octanyl, 8-Aza-bicyclo[2.2.2]octanyl oder 1-Aza-bicyclo[2.2.1]heptyl. Im Falle von gegebenenfalls substituiertem Heterocyclyl werden auch spirocyclische Systeme umfaßt, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2-oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2-oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4-oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6-oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2-oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3-oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3-oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4-oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2-oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3-oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5-oder 6-yl; 1,2,3,4-Tetrahydropyrazin-1- oder 2- oder 3- oder 4- oder 5- oder 6-yl; 1,2-Dihydropyrazin-1-oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2-oder 4-yl; 1,3-Dioxan-2- oder 4- oder 5-yl; 4H-1,3-Dioxin-2- oder 4- oder 5- oder 6-yl; 1,4-Dioxan-2-oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2-oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2-oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3-oder 4- oder 5- oder 6-yl; 6H-1,2-Oxazin-3- oder 4- oder 5- oder 6-yl; 4H-1,2-Oxazin-3- oder 4- oder 5-oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2-oder 4- oder 5- oder 6-yl; Morpholin-2- oder 3- oder 4-yl; 3,4-Dihydro-2H-1,4-oxazin-2- oder 3- oder 4-oder 5- oder 6-yl; 3,6-Dihydro-2H-1,4-oxazin-2- oder 3- oder 5- oder 6-yl; 2H-1,4-oxazin-2- oder 3-oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2-oder 3- oder 4- oder 5- oder 6- oder 7-yl; 2,5-Dihydro-1,2-oxazepin-2- oder 3- oder 4- oder 5- oder 6-oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5-oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5-oder 6- oder 7-yl; 1,3-Oxazepin-2- oder 4- oder 5- oder 6- oder 7-yl; 1,4-Oxazepan-2- oder 3- oder 5-oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2-oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5-oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5-oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5-oder 6- oder 7-yl; 5H-1,4,2-Dioxazepin-3- oder 5- oder 6- oder 7-yl; 7H-1,4,2-Dioxazepin-3- oder 5-oder 6- oder 7-yl. Strukturbeispiele für gegebenenfalls weiter substituierte Heterocyclen sind auch im

### Folgenden aufgeführt:

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten Stickstoff-Heterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O), S(O) (auch kurz SO) und S(O)₂ (auch kurz SO₂) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet Fluor, Chlor, Brom oder Iod. Wird die Bezeichnung für einen Rest verwendet, dann bedeutet "Halogen" ein Fluor-, Chlor-, Brom- oder Iodatom.

Die Verbindungen der Formel (I) können je nach Art und Verknüpfung der Substituenten als Stereoisomere vorliegen. Sind beispielsweise ein oder mehrere asymmetrisch substiuierte Kohlenstoffatome und/oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden.

Die Erfindung betrifft auch alle Stereoisomeren und deren Gemische, die von der Formel (I) umfaßt, jedoch nicht spezifisch definiert sind. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Je nach Art der oben definierten Substituenten weisen die Verbindungen der Formel (I) saure Eigenschaften auf und können mit anorganischen oder organischen Basen oder mit Metallionen Salze, gegebenenfalls auch innere Salze oder Addukte bilden. Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin, sowie organische Amine, wie Trialkylamine, Morpholin, Piperidin oder Pyridin. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R'"]⁺, worin R bis R'" jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Ist eine Gruppe mehrfach durch Reste substituiert, so bedeutet dies, dass diese Gruppe durch einen oder mehrere gleiche oder verschiedene der genannten Reste substituiert ist.

In allen nachfolgend genannten Formeln haben die Substituenten und Symbole, sofern nicht anders definiert, dieselbe Bedeutung wie unter Formel (I) beschrieben. Pfeile in einer chemischen Formel bedeuten die Verknüpfungsorte zum restlichen Molekül.

Im Folgenden werden, jeweils für die einzelnen Substituenten, bevorzugte, besonders bevorzugte und ganz besonders bevorzugte Bedeutungen beschrieben. Die übrigen Substituenten der allgemeinen Formel (I), welche nachfolgend nicht genannt werden, weisen die oben genannte Bedeutung auf.

In einer ersten Ausführungsform der vorliegenden Erfindung bedeuten
R¹ und R² bevorzugt unabhängig voneinander jeweils Wasserstoff, Fluor, Chlor oder Cyano, oder durch jeweils m Reste aus der Gruppe bestehend aus Fluor, Chlor, Brom und Cyano substituiertes (C₁-C₃)-Alkyl oder (C₁-C₃)-Alkoxy.

Besonders bevorzugt bedeuten R¹ und R² jeweils unabhängig voneinander Wasserstoff, Fluor, Chlor oder Cyano, oder durch jeweils m Reste aus der Gruppe bestehend aus Fluor und Chlor substituiertes Methyl oder Methoxy.

Am meisten bevorzugt bedeuten R¹ und R² jeweils Wasserstoff.

In einer zweiten Ausführungsform der vorliegenden Erfindung bedeutet
R³ bevorzugt durch jeweils m Reste aus der Gruppe bestehend aus Fluor, Chlor, Brom, Cyano, (C₁-C₄)-Alkoxy und Hydroxy substituiertes (C₁-C₄)-Alkyl, (C₃-C₅)-Cycloalkyl, (C₂-C₄)-Alkenyl, (C₂-C₄)-Alkinyl oder (C₁-C₄)-Alkoxy.

Besonders bevorzugt bedeutet R³ durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy.

In einer dritten Ausführungsform der vorliegenden Erfindung bedeutet
- Y: Sauerstoff.

In einer vierten Ausführungsform der vorliegenden Erfindung bedeutet
- W: Sauerstoff.

In einer sechsten Ausführungsform der vorliegenden Erfindung steht
- Z: bevorzugt für eine Gruppe Z-1 bis Z-33, wobei Z-1 bis Z-33 folgende Bedeutung haben: 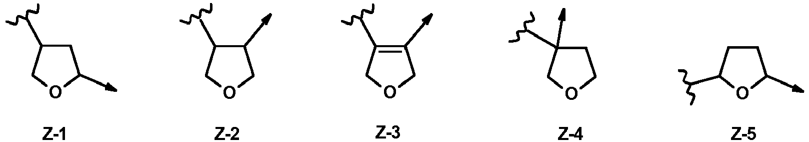 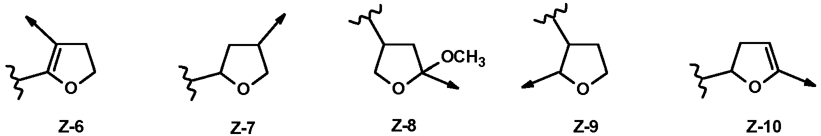 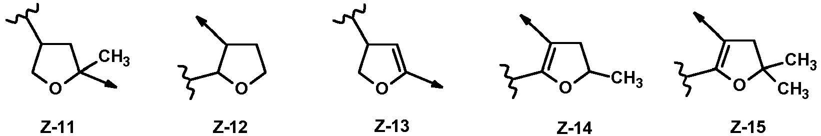 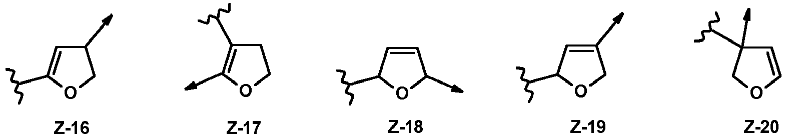 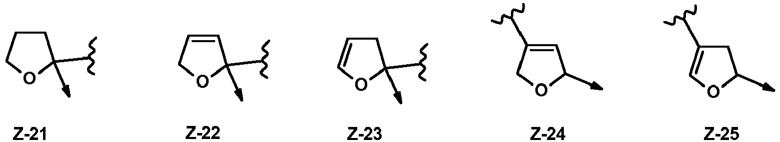 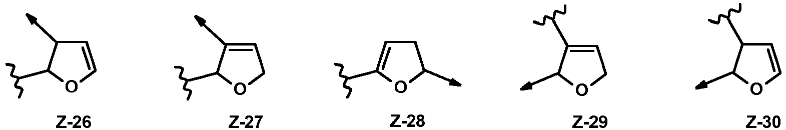 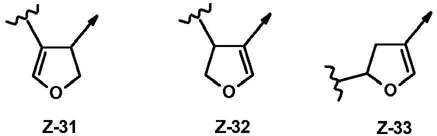
worin der Pfeil jeweils für eine Bindung zur Gruppe C=W der Formel (I) steht.

Besonders bevorzugt steht Z für eine Gruppe Z-1 bis Z-15, wobei Z-1 bis Z-15 folgende Bedeutung haben: 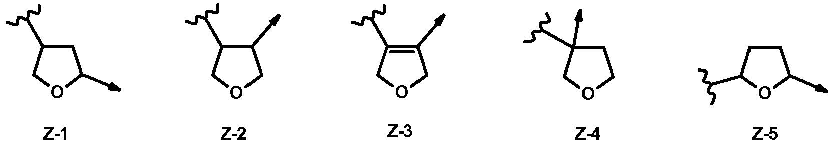 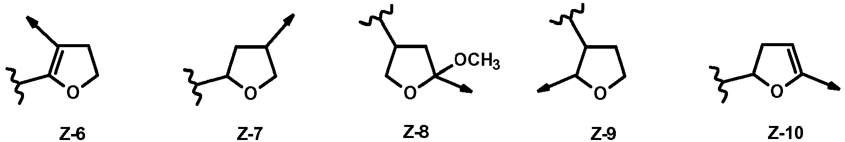 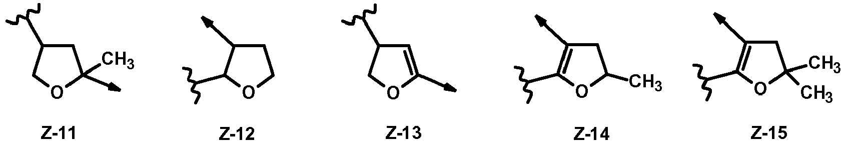 worin der Pfeil jeweils für eine Bindung zur Gruppe C=W der Formel (I) steht.
Am meisten bevorzugt steht Z für Z-1 bis Z-4 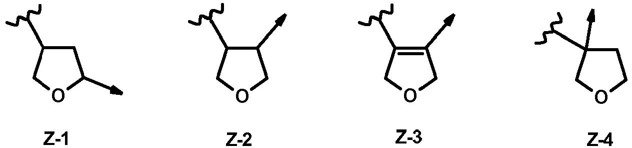 worin der Pfeil jeweils für eine Bindung zur Gruppe C=W der Formel (I) steht.

In einer siebten Ausführungsform der vorliegenden Erfindung bedeutet
R¹⁰ bevorzugt für Fluor, Chlor, Cyano, CO₂H, CO₂CH₃ oder CO₂CH₂CH₃, oder durch jeweils m Reste aus der Gruppe bestehend aus Fluor und Chlor substituiertes (C₁-C₂)-Alkyl oder (C₁-C₂)-Alkoxy.

In einer achten Ausführungsform der vorliegenden Erfindung bedeutet
R¹¹ bevorzugt Wasserstoff, oder durch jeweils m Reste aus der Gruppe bestehend aus Fluor und Chlor substituiertes (C₁-C₃)-Alkyl oder (C₃-C₆)-Cycloalkyl.

In einer neunten Ausführungsform der vorliegenden Erfindung bedeutet
R¹² bevorzugt Wasserstoff, Cyano oder Hydroxy, oder durch jeweils m Reste aus der Gruppe bestehend aus Fluor, Chlor, Brom, Cyano oder Hydroxy substituiertes OR⁷, S(O)ₙR⁵, NR⁶R⁸, NR⁶CO₂R⁸, (C₁-C₆)-Alkyl, (C₃-C₆)-Cycloalkyl, (C₂-C₃)-Alkenyl oder (C₂-C₃)-Alkinyl bedeutet.

In einer zehnten Ausführungsform der vorliegenden Erfindung bilden
R¹¹ und R¹² mit dem Stickstoffatom, an das sie gebunden sind, einen gesättigten, teilweise oder vollständig ungesättigten fünf- oder sechsgliedrigen Ring, der neben diesem Stickstoffatom r Kohlenstoffatome, n Sauerstoffatome und p Elemente aus der Gruppe bestehend aus NR⁷ und NCOR⁷ als Ringatome enthält, wobei ein Kohlenstoffatom p Oxogruppen trägt.

In einer elften Ausführungsform der vorliegenden Erfindung bedeuten
X², X⁴ und X⁶ bevorzugt unabhängig voneinander jeweils Wasserstoff, Fluor, Chlor, Brom oder Cyano, oder jeweils durch m Reste aus der Gruppe bestehend aus Fluor und Chlor substituiertes Methyl oder Methoxy.

Besonders bevorzugt bedeuten X², X⁴ und X⁶ unabhängig voneinander Wasserstoff oder Fluor.

In einer zwölften Ausführungsform der vorliegenden Erfindung bedeuten
X³ und X⁵ bevorzugt unabhängig voneinander Wasserstoff, Fluor, Chlor, Brom, Hydroxy oder Cyano, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl.

In einer dreizehnten Ausführungsform der vorliegenden Erfindung bedeutet
R⁵ bevorzugt durch jeweils m Reste aus der Gruppe bestehend aus Fluor, Chlor und Hydroxy substituiertes (C₁-C₆)-Alkyl, (C₃-C₆)-Cycloalkyl oder Aryl.

In einer vierzehnten Ausführungsform der vorliegenden Erfindung bedeutet
R⁷ bevorzugt Wasserstoff, oder durch jeweils m Reste aus der Gruppe bestehend aus Fluor, Chlor und (C₁-C₂)-Alkoxy substituiertes (C₁-C₆)-Alkyl oder (C₃-C₆)-Cycloalkyl.

In einer fünfzehnten Ausführungsform der vorliegenden Erfindung bedeutet
R⁸ bevorzugt Wasserstoff, oder durch jeweils m Reste aus der Gruppe bestehend aus Fluor, Chlor und (C₁-C₂)-Alkoxy substituiertes (C₁-C₆)-Alkyl, (C₃-C₆)-Cycloalkyl oder Aryl.

In einer sechzehnten Ausführungsform der vorliegenden Erfindung beträgt
m die Laufzahl 0, 1, 2 oder 3.

Im Rahmen der vorliegenden Erfindung ist es möglich, die einzelnen bevorzugten, besonders bevorzugten und am meisten bevorzugten Bedeutungen für die Substituenten R¹ bis R³, R⁵ bis R⁸, R¹⁰ bis R¹², X² bis X⁶, W, Y und Z, sowie die Laufzahlen k, m, n, p und r beliebig miteinander zu kombinieren.

Das heißt, dass Verbindungen der allgemeinen Formel (I) von der vorliegenden Erfindung umfasst sind, in welchen beispielsweise der Substituent R¹ eine bevorzugte Bedeutung aufweist und die Substituenten R⁵ bis R⁷ die allgemeine Bedeutung aufweisen oder aber der Substituent R² eine bevorzugte Bedeutung aufweist, der Substituent R³ eine besonders bevorzugte, bzw. eine ganz besonders bevorzugte, Bedeutung aufweist und die übrigen Substituenten eine allgemeine Bedeutung aufweisen.

Zwei dieser Kombinationen der oben für die Substituenten R¹ bis R³, R⁵ bis R⁸, R¹⁰ bis R¹², X² bis X⁶, W, Y und Z, sowie für die Laufzahlen k, m, n, p und r angegebenen Definitionen werden nachfolgend beispielhaft erläutert und jeweils als weitere Ausführungsformen offenbart:

In einer siebzehnten Ausführungsform der vorliegenden Erfindung bedeuten
- R¹ und R²: bevorzugt unabhängig voneinander jeweils Wasserstoff, Fluor, Chlor oder Cyano, oder durch jeweils m Reste aus der Gruppe bestehend aus Fluor, Chlor, Brom und Cyano substituiertes (C₁-C₃)-Alkyl oder (C₁-C₃)-Alkoxy;
- R³: bedeutet durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy;
- Y: bedeutet Sauerstoff;
- W: bedeutet Sauerstoff;
- Z: steht für eine Gruppe Z-1 bis Z-15, wobei Z-1 bis Z-15 folgende Bedeutung haben: 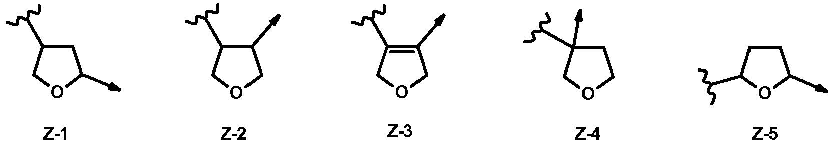 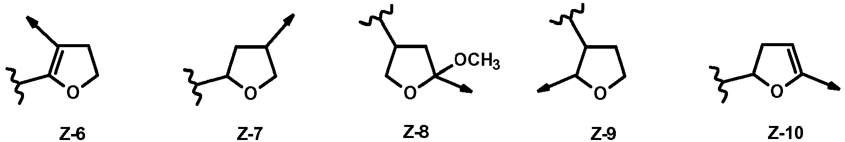 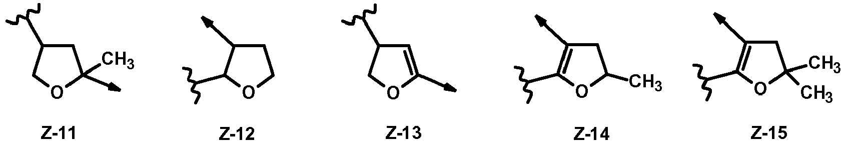
worin der Pfeil jeweils für eine Bindung zur Gruppe C=W der Formel (I) steht;
- X², X⁴ und X⁶: bedeuten unabhängig voneinander jeweils Wasserstoff, Fluor, Chlor, Brom oder Cyano, oder jeweils durch m Reste aus der Gruppe bestehend aus Fluor und Chlor substituiertes Methyl oder Methoxy;
- X³ und X⁵: bedeuten unabhängig voneinander Wasserstoff, Fluor, Chlor, Brom, Hydroxy oder Cyano, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl; und
- R⁵: bedeutet durch jeweils m Reste aus der Gruppe bestehend aus Fluor, Chlor und Hydroxy substituiertes (C₁-C₆)-Alkyl, (C₃-C₆)-Cycloalkyl oder Aryl;
- R⁶: bedeutet Wasserstoff oder R⁵;
- R⁷: bedeutet Wasserstoff, oder durch jeweils m Reste aus der Gruppe bestehend aus Fluor, Chlor und (C₁-C₂)-Alkoxy substituiertes (C₁-C₆)-Alkyl oder (C₃-C₆)-Cycloalkyl;
- R⁸: bedeutet Wasserstoff, oder durch jeweils m Reste aus der Gruppe bestehend aus Fluor, Chlor und (C₁-C₂)-Alkoxy substituiertes (C₁-C₆)-Alkyl, (C₃-C₆)-Cycloalkyl oder Aryl;
- R¹¹: bedeutet Wasserstoff, oder durch jeweils m Reste aus der Gruppe bestehend aus Fluor und Chlor substituiertes (C₁-C₃)-Alkyl oder (C₃-C₆)-Cycloalkyl;
- R¹²: bedeutet Wasserstoff, Cyano oder Hydroxy, oder durch jeweils m Reste aus der Gruppe bestehend aus Fluor, Chlor, Brom, Cyano und Hydroxy substituiertes OR⁷, S(O)ₙR⁵, NR⁶R⁸, NR⁶CO₂R⁸, (C₁-C₆)-Alkyl, (C₃-C₆)-Cycloalkyl, (C₂-C₃)-Alkenyl oder (C₂-C₃)-Alkinyl; oder
- R¹¹ und R¹²: bilden mit dem Stickstoffatom, an das sie gebunden sind, einen gesättigten, teilweise oder vollständig ungesättigten fünf- oder sechsgliedrigen Ring, der neben diesem Stickstoffatom r Kohlenstoffatome, n Sauerstoffatome und p Elemente aus der Gruppe bestehend aus NR⁷ und NCOR⁷ als Ringatome enthält, wobei ein Kohlenstoffatom p Oxogruppen trägt;
- m: die Laufzahl beträgt 0, 1, 2 oder 3;
- n: die Laufzahl beträgt 0, 1 oder 2;
- p: die Laufzahl beträgt 0 oder 1; und
- r: die Laufzahl beträgt 3, 4 oder 5.

In einer achtzehnten Ausführungsform der vorliegenden Erfindung bedeuten
- R¹ und R²: jeweils Wasserstoff;
- R³: bedeutet durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy;
- Y: bedeutet Sauerstoff;
- W: bedeutet Sauerstoff;
- Z: steht für eine Gruppe Z-1 bis Z-4, wobei Z-1 bis Z-4 folgende Bedeutung haben: 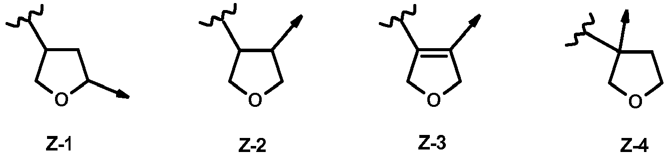
worin der Pfeil jeweils für eine Bindung zur Gruppe C=W der Formel (I) steht;
- X², X⁴ und X⁶: bedeuten unabhängig voneinander jeweils Wasserstoff oder Fluor;
- X³ und X⁵: bedeuten unabhängig voneinander Wasserstoff, Fluor, Chlor, Brom, Hydroxy oder Cyano, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl; und
- R⁵: bedeutet durch jeweils m Reste aus der Gruppe bestehend aus Fluor, Chlor und Hydroxy substituiertes (C₁-C₆)-Alkyl, (C₃-C₆)-Cycloalkyl oder Aryl;
- R⁶: bedeutet Wasserstoff oder R⁵;
- R⁷: bedeutet Wasserstoff, oder durch jeweils m Reste aus der Gruppe bestehend aus Fluor, Chlor und (C₁-C₂)-Alkoxy substituiertes (C₁-C₆)-Alkyl oder (C₃-C₆)-Cycloalkyl;
- R⁸: bedeutet Wasserstoff, oder durch jeweils m Reste aus der Gruppe bestehend aus Fluor, Chlor und (C₁-C₂)-Alkoxy substituiertes (C₁-C₆)-Alkyl, (C₃-C₆)-Cycloalkyl oder Aryl;
- R¹¹: bedeutet Wasserstoff, oder durch jeweils m Reste aus der Gruppe bestehend aus Fluor und Chlor substituiertes (C₁-C₃)-Alkyl oder (C₃-C₆)-Cycloalkyl;
- R¹²: bedeutet Wasserstoff, Cyano oder Hydroxy, oder durch jeweils m Reste aus der Gruppe bestehend aus Fluor, Chlor, Brom, Cyano und Hydroxy substituiertes OR⁷, S(O)ₙR⁵, NR⁶R⁸, NR⁶CO₂R⁸, (C₁-C₆)-Alkyl, (C₃-C₆)-Cycloalkyl, (C₂-C₃)-Alkenyl oder (C₂-C₃)-Alkinyl; oder
- R¹¹ und R¹²: bilden mit dem Stickstoffatom, an das sie gebunden sind, einen gesättigten, teilweise oder vollständig ungesättigten fünf- oder sechsgliedrigen Ring, der neben diesem Stickstoffatom r Kohlenstoffatome, n Sauerstoffatome und p Elemente aus der Gruppe bestehend aus NR⁷ und NCOR⁷ als Ringatome enthält, wobei ein Kohlenstoffatom p Oxogruppen trägt;
- m: die Laufzahl beträgt 0, 1, 2 oder 3;
- n: die Laufzahl beträgt 0, 1 oder 2;
- p: die Laufzahl beträgt 0 oder 1; und
- r: die Laufzahl beträgt 3, 4, oder 5.

Im Folgenden werden in tabellarischer Form Beispiele der Verbindungen der allgemeinen Formel (I) wiedergegeben. In der nachfolgenden Tabelle 1 werden die in Formel (I) allgemein definerten Substituenten spezifiziert.

**Tabelle 1: Verbindungen der allgemeinen Formel (I), worin X² = X⁴ = X⁶ = R¹ = R² = H und für Y = W = O steht**

| **Beispiel - Nr.** | **X**³ | **X**⁵ | **R**³ | **Z** | **R**¹¹ | **R**¹² |
|---|---|---|---|---|---|---|
| I-1 | F | F | CH₃ | Z-4 | H | cyc-Propyl |
| I-2 | F | F | CH=CH₂ | Z-1 | H | CH₃ |
| I-3 | F | F | CH₃ | Z-1 | -(CH₂)₄- | |
| I-4 | F | F | CH₃ | Z-1 | H | cyc-Propyl |
| I-5 | F | F | CH₃ | Z-1 | H | CH₃ |
| I-6 | F | F | CH=CH₂ | Z-1 | -(CH₂)₄- | |
| I-7 | F | F | CH₃ | Z-2 | H | CH₃ |
| I-8 | F | F | CH₃ | Z-2 | H | cyc-Propyl |
| I-9 | F | F | CH₃ | Z-2 | -(CH₂)₄- | |
| I-10 | F | H | CH₃ | Z-1 | H | O-CH₃ |
| I-11 | F | F | (S) - CH=CH₂ | Z-1 | H | O-CH₃ |
| I-12 | F | F | (S) - CH=CH₂ | Z-1 | H | O-C₂H₅ |
| I-13 | F | F | (S) - CH=CH₂ | Z-1 | H | O-CH(CH₃)₂ |
| I-14 | F | F | (S) - CH=CH₂ | Z-1 | H | SO₂CF₃ |
| I-15 | F | F | (S) - CH=CH₂ | Z-1 | H | N(CH₃)₂ |
| I-16 | F | F | (S) - CH=CH₂ | Z-1 | H | SO₂CH₃ |
| I-17 | F | F | (S) - CH=CH₂ | Z-1 | CH₃ | CH₃ |
| I-18 | F | F | (S) - CH=CH₂ | Z-1 | H | cyc-Propyl |
| I-19 | F | F | (S) - CH=CH₂ | Z-1 | H | NH-(C=O)-OC(CH₃)₃ |
| I-20 | F | F | (S) - CH=CH₂ | Z-1 | H | CH₂-CH₂-CO₂Me |
| I-21 | F | F | (S) - CH=CH₂ | Z-1 | H | CH₂-CO₂Me |
| I-22 | F | F | (S) - CH=CH₂ | Z-1 | H | (3S)-CH(CH₃)-CH_{z}-CO₂Et |
| I-23 | F | F | (R) - CH₃ | Z-1 | H | SO₂Me |
| I-24 | F | F | (R) - CH₃ | Z-1 | H | OMe |
| I-25 | F | F | (R) - CH₃ | Z-1 | Me | Me |
| I-26 | F | F | (R) - CH₃ | Z-1 | H | NMe₂ |
| I-27 | F | F | (R) - CH₃ | Z-1 | H | [3-chloro-5-(trifluoromethyl)pyridin-2-yl]methyl |
| I-28 | F | F | (R) - CH₃ | Z-1 | H | SO₂CH₂-CH₂-CH₃ |
| I-29 | F | F | (S) - CH=CH₂ | Z-1 | H | SO₂CH₂-CH₂-CH₃ |
| I-30 | F | F | (R) - CH₃ | Z-1 | H | SO₂CF₃ |

Die erfindungsgemäßen Verbindungen können nach verschiedenen Verfahren hergestellt werden, die beispielhaft im Folgenden aufgeführt werden:

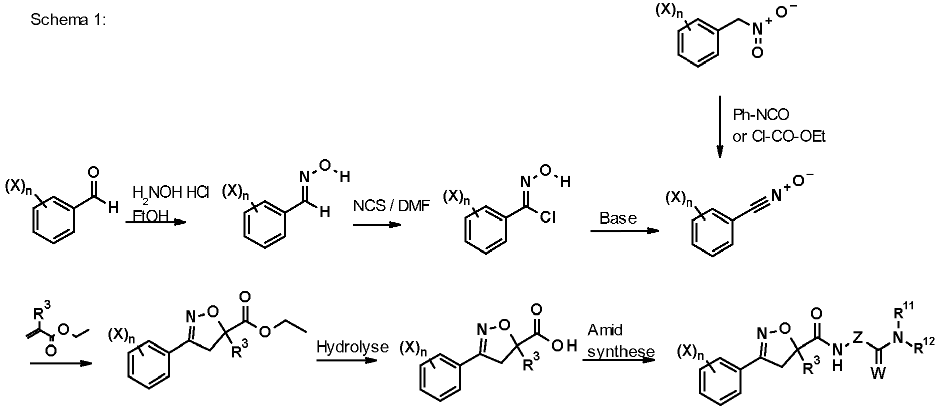

In Schema 1 und den nachfolgenden Schemata steht (X)ₙ für die Substituenten X², X³, X⁴, X⁵ und X⁶. Solche 1,3-dipolaren Cycloadditionen von Nitriloxiden mit geeigneten Dipolarophilen sind beispielsweise beschrieben in Reviews: 1,3 dipolar Cycloaddition Chemistry, Padwa, ed. Wiley, New York, 1984; Kanemasa and Tsuge, Heterocycles 1990, 30, 719. Zur Darstellung von Chloroximen siehe Kim, Jae N., Ryu, Eung K. J. Org. Chem. 1992, 57, 6649).

Erfindungsgemäße Verbindungen, die in der 4- und 5- Position des Isoxazolin-Ringsystems substituiert sind, lassen sich ebenfalls durch 1,3-dipolare Cycloaddition herstellen, indem geeignet 1,2-disubstituierte Olefine als Dipolarophile eingesetzt werden. Zumeist entstehen bei dieser Reaktion Diastereomerengemische, die durch Säulenchromatographie getrennt werden können. Optisch aktive Isoxazoline können durch chirale HPLC geeigneter Vorstufen oder Endstufen erhalten werden, ebenso auch durch enantioselektive Reakionen wie z.B. enzymatische Ester- oder Amidspaltungen oder durch den Einsatz von chiralen Hilfsreagenzien am Dipolarophil wie von Olssen beschrieben, (J. Org.Chem. 1988, 53, 2468).

Zur Herstellung der erfindungsgemäßen Verbindungen können auch geeignet substituierte 2-Alkoxyacrylsäureamide eingesetzt werden (Schema 3). Diese sind aus den in Schema 2 beschriebenen Acrylsäureestern nach Hydrolyse und Amidbildung zugänglich.

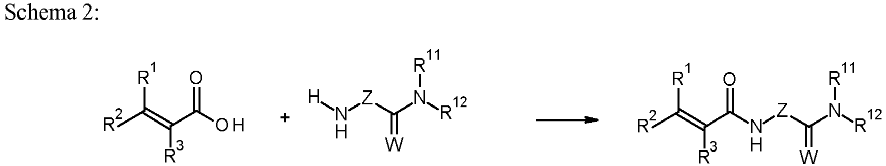

Zur Aktivierung der Acrylsäure bieten sich dabei Carbodiimide wie zum Beispiel EDCI an (Chen, F. M. F.; Benoiton, N. L. Synthesis 1979, 709). Zur Herstellung von Acrylsäureamiden siehe US 2,521,902, JP60112746, J. of Polymer Science 1979, 17 (6), 1655. Geeignet substituierte Acrylsäureamide können in einer 1,3-Cycloadditionsreaktion mit Nitriloxiden zu den erfindungsgemäßen Verbindungen umgesetzt werden (Schema 3).

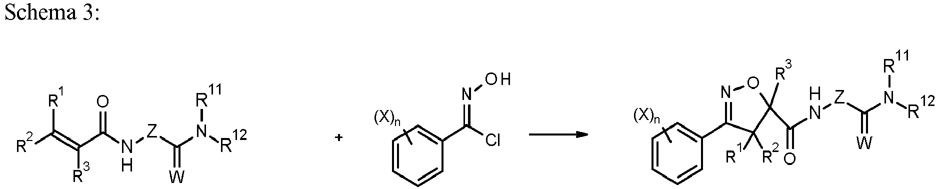

Transformationen der funktionellen Gruppen R³ sind sowohl auf der Stufe der Alkene als auch auf der Stufe der Isoxazoline möglich.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Die erfindungsgemäßen Verbindungen können in Nutzkulturen Selektivitäten aufweisen und können auch als nichtselektive Herbizide eingesetzt werden.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten in der Agrarindustrie verwendeten Wirkstoff, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften liegen in einer Toleranz oder Resistenz gegen abiotische Stressoren z.B. Hitze, Kälte, Trockenheit, Salz und ultraviolette Strahlung.

Bevorzugt ist die Anwendung der erfindungsgemäßen Verbindungen der Formel (I) oder deren Salze in wirtschaftlich bedeutenden transgenen Kulturen von Nutz-und Zierpflanzen,
Die Verbindungen der Formel (I) können als Herbizide in Nutzpflanzenkulturen eingesetzt werden, welche gegenüber den phytotoxischen Wirkungen der Herbizide resistent sind bzw. gentechnisch resistent gemacht wurden.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen gentechnische Veränderungen von Kulturpflanzen zwecks Modifikation der in den Pflanzen synthetisierten Stärke (z.B. WO 92/011376 A, WO 92/014827 A, WO 91/019806 A), transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg, oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4-D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung OptimumTM GATTM (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxid-addukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen Wirkstoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2006 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfaßt, auch wenn diese nicht explizit erwähnt sind.

### Beispiele für solche herbiziden Mischungspartner sind:

Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, - potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

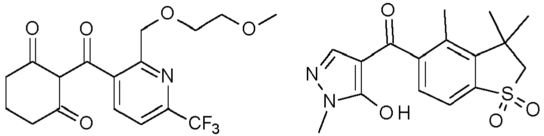

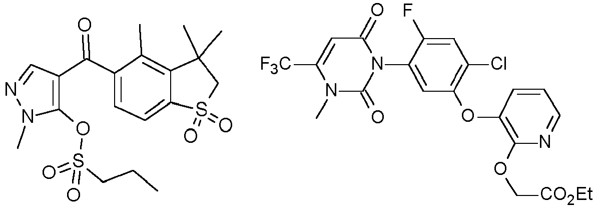

### Beispiele für Pflanzenwachstumsregulatoren als mögliche Mischungspartner sind:

Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2- naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Safener, die in Kombination mit den erfindungsgemäßen Verbindungen der Formel (I) und ggf. in Kombinationen mit weiteren Wirkstoffen wie z.B. Insektiziden, Akariziden, Herbiziden, Fungiziden wie oben aufgelistet, eingesetzt werden können, sind vorzugsweise ausgewählt aus der Gruppe bestehend aus:
S1) Verbindungen der Formel (S1), 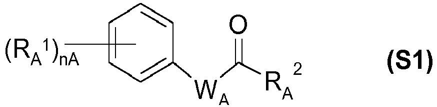 wobei die Symbole und Indizes folgende Bedeutungen haben:
   n_{A} ist eine natürliche Zahl von 0 bis 5, vorzugsweise 0 bis 3;
   R_{A}¹ ist Halogen, (C₁-C₄)Alkyl, (C₁-C₄)Alkoxy, Nitro oder (C₁-C₄)Haloalkyl;
   W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 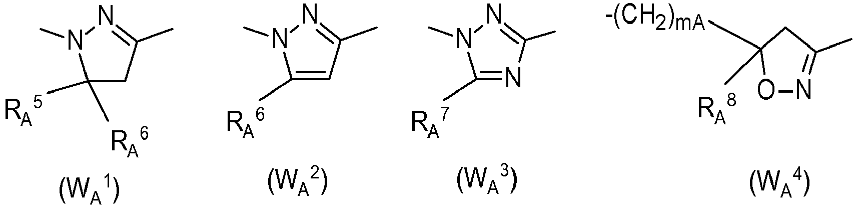
m_{A} ist 0 oder 1;
R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
R_{A}³ ist Wasserstoff oder ein unsubstituierter oder substituierter aliphatischer Kohlenwasserstoffrest, vorzugsweise mit insgesamt 1 bis 18 C-Atomen;
R_{A}⁴ ist Wasserstoff, (C₁-C₆)Alkyl, (C₁-C₆)Alkoxy oder substituiertes oder unsubstituiertes Phenyl;
R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
R_{A}⁶, R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 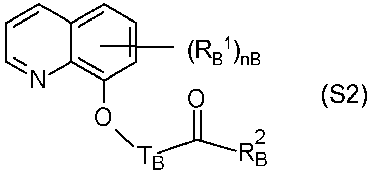 wobei die Symbole und Indizes folgende Bedeutungen haben:
   R_{B}¹ ist Halogen, (C₁-C₄)Alkyl, (C₁-C₄)Alkoxy, Nitro oder (C₁-C₄)Haloalkyl;
   n_{B} ist eine natürliche Zahl von 0 bis 5, vorzugsweise 0 bis 3;
   R_{B}² ist OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   R_{B}³ ist Wasserstoff oder ein unsubstituierter oder substituierter aliphatischer Kohlenwasserstoffrest, vorzugsweise mit insgesamt 1 bis 18 C-Atomen;
   R_{B}⁴ ist Wasserstoff, (C₁-C₆)Alkyl, (C₁-C₆)Alkoxy oder substituiertes oder unsubstituiertes Phenyl;
   T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
      a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
      b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 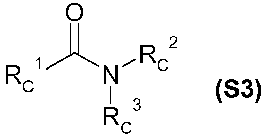 wobei die Symbole und Indizes folgende Bedeutungen haben:
   R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
      Wirkstoffe vom Typ der Dichloracetamide, die häufig als Vorauflaufsafener (bodenwirksame Safener) angewendet werden, wie z. B.
      "Dichlormid" (N,N-Diallyl-2,2-dichloracetamid) (S3-1),
      "R-29148" (3-Dichloracetyl-2,2,5-trimethyl-1,3-oxazolidin) der Firma Stauffer (S3-2),
      "R-28725" (3-Dichloracetyl-2,2,-dimethyl-1,3-oxazolidin) der Firma Stauffer (S3-3),
      "Benoxacor" (4-Dichloracetyl-3,4-dihydro-3-methyl-2H-1,4-benzoxazin) (S3-4),
      "PPG-1292" (N-Allyl-N-[(1,3-dioxolan-2-yl)-methyl]-dichloracetamid) der Firma PPG Industries (S3-5),
      "DKA-24" (N-Allyl-N-[(allylaminocarbonyl)methyl]-dichloracetamid) der Firma Sagro-Chem (S3-6),
      "AD-67" oder "MON 4660" (3-Dichloracetyl-1-oxa-3-aza-spiro[4,5]decan) der Firma Nitrokemia bzw. Monsanto (S3-7),
      "TI-35" (1-Dichloracetyl-azepan) der Firma TRI-Chemical RT (S3-8),
      "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF,
      "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 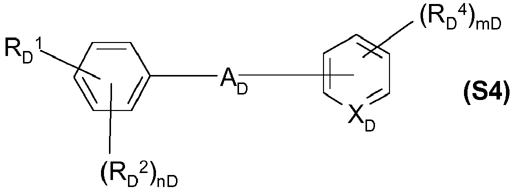 worin die Symbole und Indizes folgende Bedeutungen haben:
   A_{D} ist SO₂-NR_{D}³-CO oder CO-NR_{D}³-SO₂
   X_{D} ist CH oder N;
   R_{D}¹ ist CO-NR_{D}⁵R_{D}⁶ oder NHCO-R_{D}⁷;
   R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   R_{D}³ ist Wasserstoff, (C₁-C₄)Alkyl, (C₂-C₄)Alkenyl oder (C₂-C₄)Alkinyl;
   R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   R_{D}⁶ ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   R_{D}⁵ und R_{D}⁶ gemeinsam mit dem dem sie tragenden Stickstoffatom einen Pyrrolidinyl- oder Piperidinyl-Rest bilden;
   R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   n_{D} ist 0, 1 oder 2;
   m_{D} ist 1 oder 2;
   v_{D} ist 0, 1, 2 oder 3;
   davon bevorzugt sind Verbindungen vom Typ der N-Acylsulfonamide, z.B. der nachfolgenden Formel (S4^{a}), die z. B. bekannt sind aus WO-A-97/45016 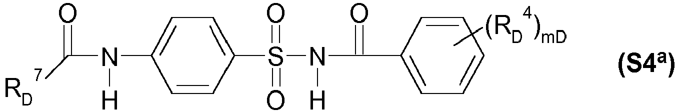 worin
   R_{D}⁷ (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   R_{D}⁴ Halogen, (C₁-C₄)Alkyl, (C₁-C₄)Alkoxy, CF₃;
   m_{D} 1 oder 2;
   v_{D} ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 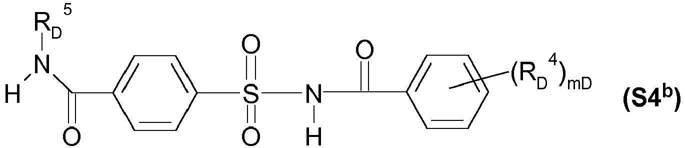
   z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 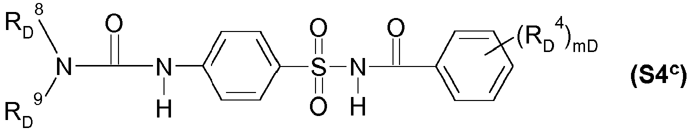
   worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   R_{D}⁴ Halogen, (C₁-C₄)Alkyl, (C₁-C₄)Alkoxy, CF₃
   m_{D} 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   sowie
   N-Phenylsulfonylterephthalamide der Formel (S4^{d}), die z.B. bekannt sind aus CN 101838227, 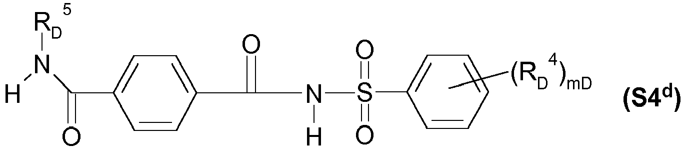
   z.B. solche worin
   R_{D}⁴ Halogen, (C₁-C₄)Alkyl, (C₁-C₄)Alkoxy, CF₃;
   m_{D} 1 oder 2;
   R_{D}⁵ Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 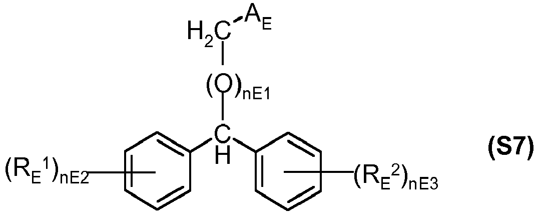 worin die Symbole und Indizes folgende Bedeutungen haben:
   R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   A_{E} ist COOR_{E}³ oder COSR_{E}⁴
   R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   n_{E}¹ ist 0 oder 1
   n_{E}², n_{E}³ sind unabhängig voneinander 0, 1 oder 2,
   vorzugsweise:
      Diphenylmethoxyessigsäure,
      Diphenylmethoxyessigsäureethylester,
      Diphenylmethoxyessigsäuremethylester (CAS-Reg.Nr. 41858-19-9) (S7-1).
S8) Verbindungen der Formel (S8),wie sie in der WO-A-98/27049 beschrieben sind 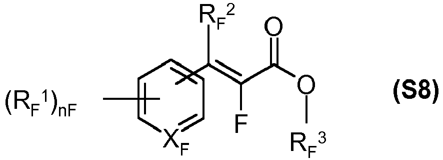 worin
   X_{F} CH oder N, n_{F} für den Fall, dass X_{F}=N ist, eine ganze Zahl von 0 bis 4 und
   für den Fall, dass X_{F}=CH ist, eine ganze Zahl von 0 bis 5 ,
   R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   R_{F}² Wasserstoff oder (C₁-C₄)Alkyl
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   X_{F} CH,
   n_{F} eine ganze Zahl von 0 bis 2 ,
   R_{F}¹ Halogen, (C₁-C₄)Alkyl, (C₁-C₄)Haloalkyl, (C₁-C₄)Alkoxy, (C₁-C₄)Haloalkoxy,
   R_{F}² Wasserstoff oder (C₁-C₄)Alkyl,
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten,
   oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 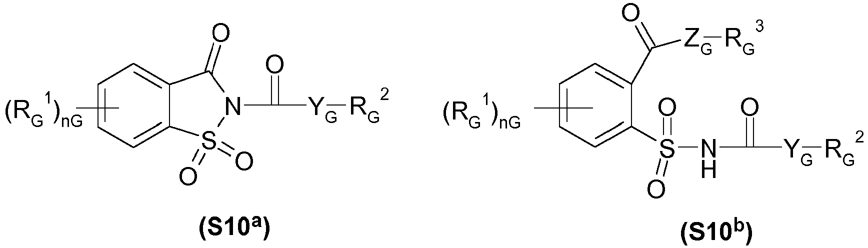 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5) (2-propenyl l-oxa-4-azaspiro[4.5]decan-4-carbodithioat) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY 93" (*S*-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC 940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 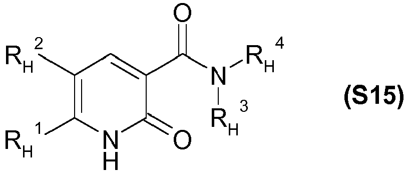 wie sie in der WO-A-2008/131861 und WO-A-2008/131860 beschrieben sind
   worin
   R_{H}¹ einen (C₁-C₆)Haloalkylrest bedeutet und
   R_{H}² Wasserstoff oder Halogen bedeutet und
   R_{H}³, R_{H}⁴ unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   R_{H}³ (C₁-C₄)-Alkoxy, (C₂-C₄)Alkenyloxy, (C₂-C₆)Alkinyloxy oder (C₂-C₄)Haloalkoxy bedeutet und
   R_{H}⁴ Wasserstoff oder (C₁-C₄)-Alkyl bedeutet oder
   R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Besonders bevorzugte Safener sind Mefenpyr-diethyl, Cyprosulfamid, Isoxadifen-ethyl, Cloquintocetmexyl, Benoxacor und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolether-sulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepoly-glykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfett-säureester oder Polyoxethylensorbitanester wie z.B. Polyoxyethylensorbitan-fettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoff-konzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasser-dispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Trägerstoff bedeutet eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels. Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden. Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent. Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgut-behandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430, US 5,876,739, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende Hauptanbaupflanzen erwähnt: Mais, Sojabohne, Baumwolle, Brassica Ölsaaten wie Brassica napus (z.B. Canola), Brassica rapa, B. juncea (z.B. (Acker-)Senf) und Brassica carinata, Reis, Weizen Zuckerrübe, Zurckerrohr, Hafer, Roggen, Gerste, Hirse, Triticale, Flachs, Wein und verschiedene Früchte und Gemüse von verschiedenen botanischen Taxa wie z.B. Rosaceae sp. (beispielsweise Kernfrüchte wie Apfel und Birne, aber auch Steinfrüchte wie Aprikosen, Kirschen, Mandeln und Pfirsiche und Beerenfrüchte wie Erdbeeren), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (beispielsweise Bananenbäume und -plantagen), Rubiaceae sp. (beispielsweise Kaffee), Theaceae sp., Sterculiceae sp., Rutaceae sp. (beispielsweise Zitronen, Organen und Grapefruit); Solanaceae sp. (beispielsweise Tomaten, Kartoffeln, Pfeffer, Auberginen), Liliaceae sp., Compositae sp. (beispielsweise Salat, Artischocke and Chicoree - einschließlich Wurzelchicoree, Endivie oder gemeinen Chicoree), Umbelliferae sp. (beispielsweise Karrotte, Petersilie, Stangensellerie und Knollensellerie), Cucurbitaceae sp. (beispielsweise Gurke - einschließlich Gewürzgurke, Kürbis, Wassermelone, Flaschenkürbis und Melonen), Alliaceae sp. (beispielsweise Lauch und Zwiebel), Cruciferae sp. (beispielsweise Weißkohl, Rotkohl, Brokkoli, Blumenkohl, Rosenkohl, Pak Choi, Kohlrabi, Radieschen, Meerrettich, Kresse und Chinakohl), Leguminosae sp. (beispielsweise Erdnüsse, Erbsen, und Bohnen - wie z.B. Stangenbohne und Ackerbohne), Chenopodiaceae sp. (beispielsweise Mangold, Futterrübe, Spinat, Rote Rübe), Malvaceae (beispielsweise Okra), Asparagaceae (beispielsweise Spargel); Nutzpflanzen und Zierpflanzen in Garten und Wald; sowie jeweils genetisch modifizierte Arten dieser Pflanzen.

Wie oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Beispiele für Nematoden-resistente Pflanzen sind z.B. folgenden US Patentanmeldungen beschrieben: 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396 und 12/497,221.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und - abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im Allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d.h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. Pflanzen können mit verschiedenen Methoden tolerant gegenüber Glyphosate gemacht werden. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakteriums Salmonella typhimurium (Comai et al., 1983, Science 221, 370-371), das CP4-Gen des Bakteriums Agrobacterium sp. (Barry et al., 1992, Curr. Topics Plant Physiol. 7, 139-145), die Gene, die für eine EPSPS aus der Petunie (Shah et al., 1986, Science 233, 478-481), für eine EPSPS aus der Tomate (Gasser et al., 1988, J. Biol. Chem. 263, 4280-4289) oder für eine EPSPS aus Eleusine (WO 01/66704) kodieren. Es kann sich auch um eine mutierte EPSPS handeln. Glyphosate-tolerante Pflanzen können auch dadurch erhalten werden, dass man ein Gen exprimiert, das für ein Glyphosate-Oxidoreduktase-Enzym kodiert. Glyphosate-tolerante Pflanzen können auch dadurch erhalten werden, dass man ein Gen exprimiert, das für ein Glyphosateacetyltransferase-Enzym kodiert. Glyphosatetolerante Pflanzen können auch dadurch erhalten werden, dass man Pflanzen, die natürlich vorkommende Mutationen der oben erwähnten Gene enthalten, selektiert. Pflanzen, die EPSPS Gene, welche Glyphosate-Toleranz verleihen, exprimieren, sind beschrieben. Pflanzen, welche andere Gene, die Glyphosate-Toleranz verleihen, z.B. Decarboxylase-Gene, sind beschrieben.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes oder chimäres HPPD-Enzym kodiert, transformiert werden, wie in WO 96/38567, WO 99/24585, WO 99/24586, WO 2009/144079, WO 2002/046387 oder US 6,768,044 beschrieben. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen sind in WO 99/34008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie in WO 2004/024928 beschrieben ist. Außerdem können Pflanzen noch toleranter gegen HPPD-Hemmern gemacht werden, indem man ein Gen in ihr Genom einfügt, welches für ein Enzym kodiert, das HPPD-Hemmer metabolisiert oder abbaut, wie z.B. CYP450 Enzyme (siehe WO 2007/103567 und WO 2008/150473).

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen wie z.B. in Tranel und Wright (Weed Science 2002, 50, 700-712) beschrieben ist. Die Herstellung von sulfonylharnstofftoleranten Pflanzen und imidazolinontoleranten Pflanzen ist beschrieben. Weitere sulfonylharnstoff- und imidazolinontolerante Pflanzen sind auch beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden (vgl. z.B. für Sojabohne US 5,084,082, für Reis WO 97/41218, für Zuckerrübe US 5,773,702 und WO 99/057965, für Salat US 5,198,599 oder für Sonnenblume WO 01/065922).

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus Bacillus thuringiensis oder einen insektiziden Teil davon, wie die insektiziden Kristallproteine, aufgelistet von Crickmore et al. (Microbiology and Molecular Biology Reviews 1998, 62, 807-813), aktualisiert von Crickmore et al. (2005) bei der Bacillus thuringiensis Toxin Nomenclatur, online bei: http://www.lifesci.sussex.ac.uk/Home/Neil_Crickmore/Bt/),
   oder insektizide Teile davon, z.B. Proteine der Cry-Proteinklassen Cry1Ab, Cry1Ac, Cry1B, Cry1C, Cry1D, Cry1F, Cry2Ab, Cry3Aa, or Cry3Bb oder insektizide Teile davon (z.B. EP-A 1999141 und WO 2007/107302), oder solche Proteine, kodiert durch synthetische Gene wie in US Patentanmeldung 12/249,016 beschrieben ist; oder
2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Nat. Biotechnol. 2001, 19, 668-72; Applied Environm. Microbiol. 2006, 71, 1765-1774) oder das binäre Toxin, das aus den Cry1A oder Cry1F Proteinen besteht und die Cry2Aa oder Cry2Ab oder Cry2Ae Proteine (US Patentanmeldung 12/214,022 und EP08010791.5); oder
3) ein insektizides Hybridprotein, das Teile von zwei unterschiedlichen insektiziden Kristallproteinen aus Bacillus thuringiensis umfasst, wie zum Beispiel ein Hybrid aus den Proteinen von 1) oben oder ein Hybrid aus den Proteinen von 2) oben, z. B. das Protein CrylA.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus Bacillus thuringiensis oder Bacillus cereus oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 94/21795); oder
7) ein insektizides Hybridprotein, das Teile von verschiedenen sezernierten Proteinen von Bacillus thuringiensis oder Bacillus cereus umfasst, wie ein Hybrid der Proteine von 1) oder ein Hybrid der Proteine von 2) oben; oder
8) ein Protein gemäß einem der Punkte 5) bis 7) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102; oder
9) ein sezerniertes Protein aus Bacillus thuringiensis oder Bacillus cereus, das in Gegenwart eines Kristallproteins von Bacillus thuringiensis insektizid wirkt, wie das binäre Toxin, das aus den Proteinen VIP3 und Cry1A oder Cry1F besteht (US Patentanmeldungen 61/126083 und 61/195019), oder das binäre Toxin, das aus dem VIP3 Protein und den Cry2Aa oder Cry2Ab oder Cry2Ae Proteinen besteht (US Patentanmeldung 12/214,022 und EP 08010791.5); oder
10) ein Protein gemäß Punkt 9) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt). Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 10 kodieren. In einer Ausführungsform enthält eine insektenresistente Pflanze mehr als ein Transgen, das für ein Protein nach einer der oben genannten 1 bis 10 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Eine "insekten-resistente transgene Pflanze" umfasst im vorliegenden Zusammenhang weiterhin jede Pflanze, die wenigstens ein Transgen enthält, welches eine Sequenz zur Herstellung einer Doppelstrang-RNA umfasst, die nach Nahrungsaufnahme durch einen Insektenschädling das Wachstum dieses Schädlings hindert.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.
4) Transgene Pflanzen oder Hybridpflanzen wie Zwiebeln mit bestimmten Eigenschaften wie "hohem Anteil an löslichen Feststoffen" (,high soluble solids content'), geringe Schärfe (,low pungency', LP) und/oder lange Lagerfähigkeit (,long storage', LS).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten, wie Baumwollpflanzen mit einer erhöhten Expression der Saccharosephosphatsynthase;
c) Pflanzen wie Baumwollpflanzen mit einer erhöhten Expression der Saccharosesynthase;
d) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
e) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Kartoffeln, welche Virus-resistent sind z.B. gegen den Kartoffelvirus Y (Event SY230 und SY233 von Tecnoplant, Argentinien), oder welche resistent gegen Krankheiten wie die Kraut- und Knollenfäule (potato late blight) (z.B. RB Gen), oder welche eine verminderte kälteinduzierte Süße zeigen (welche die Gene Nt-Inh, II-INV tragen) oder welche den Zwerg-Phänotyp zeigen (Gen A-20 Oxidase).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften im Samenausfall (seed shattering). Solche Pflanzen können durch genetische Transformation oder durch Selektion von Pflanzen, die eine Mutation enthalten, die solche veränderten Eigenschaften verleihen, und umfassen Pflanzen wie Raps mit verzögertem oder vermindertem Samenausfall.

Besonders nützliche transgene Pflanzen, die erfindungsgemäß behandelt werden können, sind Pflanzen mit Transformationsevents oder Kombinationen von Transformationsevent, welche in den USA beim Animal and Plant Health Inspection Service (APHIS) of the United States Department of Agriculture (USDA) Gegenstand von erteilten oder anhängigen Petitionen für den nicht-regulierten Status sind. Die Information hierzu ist jederzeit beim APHIS (4700 River Road Riverdale, MD 20737, USA) erhältlich, z.B. über die Internetseite http://www.aphis.usda.gov/brs/not_reg.html. Am Anmeldetag dieser Anmeldung waren beim APHIS die Petitionen mit folgenden Informationen entweder erteilt oder anhängig:
- Petition: Identifikationsnummer der Petition. Die Technische Beschreibung des Transformationsevents kann im einzelnen Petitionsdokument erhältlich von APHIS auf der Website über die Petitionsnummer gefunden werden. Diese Beschreibungen sind hiermit per Referenz offenbart.
- Erweiterung einer Petition: Referenz zu einer frühere Petition, für die eine Erweiterung oder Verlängerung beantragt wird.
- Institution: Name der die Petition einreichenden Person.
- Regulierter Artikel: die betroffen Pflanzenspecies.
- Transgener Phänotyp: die Eigenschaft ("Trait"), die der Pflanze durch das Transformationsevent verliehen wird.
- Transformationevent oder -linie: der Name des oder der Events (manchmal auch als Linie(n) bezeichnet), für die der nicht-regulierte Status beantragt ist.
- APHIS Documente: verschiedene Dokumente, die von APHIS bzgl. der Petition veröffentlicht warden oder von APHIS auf Anfrage erhalten werden können.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://cera-gmc.org/index.php?evidcode=&hstIDXCode=&gType=&AbbrCode=&atCode=&stCode=&coID Code=&action=gm_crop_database&mode=Submit).

Die erfindungsgemäßen Wirkstoffe bzw. Mittel können außerdem im Materialschutz zum Schutz von technischen Materialien gegen Befall und Zerstörung durch unerwünschte Mikroorganismen, wie z.B. Pilzen und Insekten, eingesetzt werden.

Weiter können die erfindungsgemäßen Verbindungen allein oder in Kombinationen mit anderen Wirkstoffen als Antifouling-Mittel eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt: Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator; Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus; Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum; Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis; Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola; Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum; Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici; Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum; Verticilium-Arten, wie beispielsweise Verticilium alboatrum; Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii; Krebserkrankungen, Gallen und Hexenbesen, hervorgerufen durch z.B. Nectria-Arten, wie beispielsweise Nectria galligena; Welkeerkrankungen hervorgerufen durch z.B. Monilinia-Arten, wie beispielsweise Monilinia laxa; Deformationen von Blättern, Blüten und Früchten, hervorgerufen durch z.B. Taphrina-Arten, wie beispielsweise Taphrina deformans; Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea; Blüten- und Samenerkrankungen, hervorgerufen durch z.B. Botrytis-Arten, wie beispielsweise Botrytis cinerea; Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani; Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Darüber hinaus weisen die erfindungsgemäßen Wirkstoffeauch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe können daher sowohl in medizinischen als auch in nicht-medizinischen Anwendungen eingesetzt werden.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

### A. Chemische Beispiele

Die NMR-Daten offenbarter Beispiele werden entweder in klassischer Form (δ-Werte, Multiplettaufspaltung, Anzahl der H-Atome) oder als sogenannte NMR-Peak-Listen aufgefuhrt. Beim NMR-Peak-Listenverfahren werden die NMR-Daten ausgewahlter Beispiele in Form von NMR-Peaklisten notiert, wobei zu jedem Signalpeak erst der δ-Wert in ppm und dann die Signalintensität durch ein Leerzeichen getrennt aufgeführt wird. Die δ-Wert-Signalintensitäts - Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ(Intensitätᵢ);......; δₙ(Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu ¹H-NMR-Peaklisten können der Research Disclosure Database Number 564025 entnommen werden.

Die nachstehenden Beispiele erläutern die Erfindung näher.

### Intermediat 1

### Herstellung von 3,5-Difluor-N-hydroxybenzolcarboximidoylchlorid

Analog der Vorschrift in WO2012/130798 für 3,5-Dichlor-N-hydroxybenzolcarboximidoylchlorid wurde 3,5-Difluor-N-hydroxybenzolcarboximidoylchlorid ausgehend von 3,5-Difluorbenzaldehyd in zwei Stufen hergestellt.

### Intermediat 2

Herstellung von 3-(3,5-Difluorphenyl)-5-methyl-4H-isoxazol-5-carbonsäuremethylester Analog der Vorschrift in WO2012/130798 für 3-(3,5-Difluorphenyl)-5-methyl-4H-isoxazol-5-carbonsäuremethylester wurde 3-(3,5-Difluorphenyl)-5-methyl-4H-isoxazol-5-carbonsäuremethylester ausgehend von 3,5-Difluorbenzaldehyd in drei Stufen hergestellt.

### Intermediat 3

### Herstellung von 3-(3,5-Difluorphenyl)-5-methyl-4H-isoxazol-5-carbonsäure

Analog der Vorschrift in WO2012/130798 für 3-(3,5-Dichlorphenyl)-5-methyl-4H-isoxazol-5-carbonsäure wurde 3-(3,5-Difluorphenyl)-5-methyl-4H-isoxazol-5-carbonsäure durch Verseifung von 3-(3,5-Difluorphenyl)-5-methyl-4H-isoxazol-5-carbonsäuremethylester hergestellt.

### Intermediat 4

### Herstellung von 3-(3,5-Difluorphenyl)-5-methyl-4H-isoxazol-5-carbonsäurechlorid

Analog der Vorschrift in WO2012/130798 für N-tert-Butyl-3-(3,5-dichlorphenyl)-5-methyl-4, 5-dihydro-1,2-oxazol-5-carboxamid wurde aus 3-(3,5-Difluorphenyl)-5-methyl-4H-isoxazol-5-carbonsäure durch Umsetzung mit Oxalylchlorid 3-(3,5-Difluorphenyl)-5-methyl-4H-isoxazol-5-carbonsäurechlorid hergestellt und ohne weitere Reinigung als Rohprodukt eingesetzt.

### Intermediat 5

### Herstellung von 3-(3,5-Difluorphenyl)-5-(1-hydroxyethyl)-4H-isoxazol-5-carbonsäuremethylester

19.9 g (104 mmol) 3,5-Difluor-N-hydroxybenzimidoylchlorid (s. Intermediat 1) wurden in 330 ml 2-Propanol gelöst und mit 15.0 g (104 mmol) 3-Hydroxy-2-methylenbutansäuremethylester versetzt. Nach Zugabe von 43.8 g (522 mmol) Natriumhydrogencarbonat wurde die Suspension auf 50°C geheizt und die Temperatur für 2 h, bis zum vollständigen Umsatz des Ausgangsmaterials, gehalten. Die Suspension wurde filtriert und das Filtrat im Vakuum eingeengt. Der erhaltene Rückstand wurde in Dichlormethan aufgenommen, danach mit gesättigter Natriumchloridlösung gewaschen, die organische Phase mit Natriumsulfat getrocknet und nach Filtrieren im Vakuum eingedampft. Das so erhaltene rohe Produkt wurde in Toluol aufgenommen und durch Zugabe von n-Heptan zum Kristallisieren gebracht. Auf diese Weise erhielt man 25.5 g (86 %) 3-(3,5-Difluorphenyl)-5-(1-hydroxyethyl)-4H-isoxazol-5-carbonsäuremethylester in Form farbloser Kristalle.
Diastereomer 1: ¹H NMR (CDC13): δ = 1.20 (d, 3H), 2.36 (d, 1H), 3.52 (d, 1H), 3.72 (d, 1H), 3.83 (s, 3H), 4.34 (m, 1H), 6.88 (m, 1H), 7.20 (m, 2H).
Diastereomer 2: ¹H NMR (CDC13): δ = 1.29 (d, 3H), 2.12 (d, 1H), 3.58 (d, 1H), 3.68 (d, 1H), 3.83 (s, 3H), 4.23 (m, 1H), 6.88 (m, 1H), 7.20 (m, 2H).

### Intermediat 6

### Herstellung von 3-(3,5-Difluorphenyl)-5-[1-(trifluormethylsulfonyloxy)ethyl]-4H-isoxazol-5-carbonsäuremethylester

29.9 (105 mmol) 3-(3,5-Difluorphenyl)-5-(1-hydroxyethyl)-4H-isoxazol-5-carbonsäuremethylester in 660 ml Dichlormethan wurden auf 0°C gekühlt und mit 16.3 g (210 mmol) Pyridin versetzt. Dann wurde langsam eine Lösung aus 38.6 g (137 mmol) Trifluormethansulfonsäureanhydrid in 80 ml Dichloromethan hinzugefügt. Nach 30 Minuten bei 0°C wurde mit 300 ml Dichlormethan versetzt und die organische Phase dreimal mit je 200 ml einer Lösung aus gesättigter Natriumchloridlösung und 1 N Salzsäure (3:1) gewaschen. Danach wurde die organische Phase zweimal mit gesättigter Natriumchloridlösung gewaschen, mit Natriumsulfat getrocknet und das Lösungsmittel im Vakuum entfernt. Das so erhaltene Rohprodukt wurde ohne weitere Reinigung in die nächste Stufe eingesetzt. Diastereomer 1: ¹H NMR (CDC13): δ = 1.54 (d, 3H), 3.44 (d, 1H), 3.89 (s, 3H), 3.94 (d, 1H), 5.49 (q, 1H), 6.91 (m, 1H), 7.20 (m, 2H).
Diastereomer 2: ¹H NMR (CDC13): δ = 1.59 (d, 3H), 3.53 (d, 1H), 3.89 (s, 3H), 3.90 (d, 1H), 5.57 (q, 1H), 6.91 (m, 1H), 7.20 (m, 2H).

### Intermediat 7

### Herstellung von 3-(3,5-Difluorphenyl)-5-vinyl-4H-isoxazol-5-carbonsäuremethylester

43.0 g (103 mmol) Rohes Produkt der letzten Stufe (3-(3,5-Difluorphenyl)-5-[1-(trifluormethylsulfonyloxy)ethyl]-4H-isoxazol-5-carbonsäuremethylester) wurden in 500 ml Dimethylacetamid gelöst und innerhalb von 20 Minuten tropfenweise mit einer Lösung aus 18.8 g (124 mmol) DBU in 50 ml Dimethylacetamid versetzt. Die Reaktionsmischung wurde bei Raumtemperatur 2 h gerührt, dann auf 1 l eisgekühlte 2 N Salzsäure gegeben und zweimal mit je 500 ml Diethylether extrahiert. Die vereinigten organischen Phasen wurden mit Natriumsulfat getrocknet, filtriert und im Vakuum eingedampft. Nach chromatographischer Reinigung an Kieselgel mit Dichlormethan als Eluent wurde das Rohprodukt aus Cyclohexan kristalllisiert. Auf diese Weise erhielt man 23.4 g (85 %) farblose Kristalle.
¹H NMR (CDC13): δ = 3.34 (d, 1H), 3.84 (s, 3H),3.93 (d, 1H), 5.38 (d, 1H), 5.55 (d, 1H), 6.14 (dd, 1H), 6.88 (m, 1H), 7.19 (m, 2H).

### Intermediat 8

### Herstellung von 3-(3,5-Difluorphenyl)-5-vinyl-4H-isoxazol-5-carbonsäure

7.5 g (28.0 mmol) 3-(3,5-Difluorphenyl)-5-vinyl-4H-isoxazol-5-carbonsäuremethylester wurden mit 21 ml 2 N Natronlauge versetzt und 8 h zum Rückfluss erhitzt. Nach dem Abkühlen wurde die Reaktionsmischung mit Ethylacetat gewaschen, die wässrige Phase mit 2 N Salzsäure auf pH 1 angesäuert, der farblose Niederschlag abfiltriert und an der Luft getrocknet. Die Ausbeute betrug 6.8 g, (96 %).
1H NMR (CDC13): δ = 3.40 (d, 1H), 3.92 (d, 1H), 5,00 (dd, 1H), 5.45 (d, 1H), 5.63 (d, 1H), 6.16 (dd, 1H), 6.87-6.93 (m, 1H), 7.16-7.21 (m, 2H).

### Intermediat 9

### Herstellung von 3-(3,5-Difluorphenyl)-5-vinyl-4H-isoxazol-5-carbonsäurechlorid

2.70 g (10.6 mmol) 3-(3,5-Difluorphenyl)-5-vinyl-4H-isoxazol-5-carbonsäure wurden in 45 ml Dichlormethan gegeben, dann drei Tropfen Dimethylformamid (DMF) und anschließend 2.03 g (15.9 mmol) Oxalylchlorid hinzugefügt. Eine lebhafte Gasentwicklung war sichtbar. Die Mischung wurde 6 h bei Raumtemperatur gerührt und dann das Lösungsmittel und überschüssiges Oxallylchlorid im Vakuum abgedampft. Das so erhaltene Rohprodukt wurde ohne weitere Reinigung in die nächste Stufe eingesetzt.

### Intermediat 10

### Herstellung von 4-Aminotetrahydrofuran-3-carbonsäuremethylester

4-Aminotetrahydrofuran-3-carbonsäuremethylester kann nach der von G.R. Ott et al.; Bioorg. Med. Chem. Lett. 2008, 694-699 beschriebenen Methode hergestellt werden.

### Intermediat 11

Herstellung von Cis-4-Aminotetrahydrofuran-2-carbonsäuremethylester Hydrochlorid Cis-4-Aminotetrahydrofuran-2-carbonsäuremethylester Hydrochlorid kann nach der von D.P. Walker et al., Synthesis 2011, 1113-1119 beschriebenen Methode hergestellt werden.

### Intermediat 12

### Herstellung von [Cis-4-aminotetrahydrofuran-2-yl](pyrrolidin-1-yl)methanon Hydrochlorid

Zu 100 mg (0.55 mmol) Cis-4-Aminotetrahydrofuran-2-carbonsäuremethylester Hydrochlorid wurden 1.15 ml (13.7 mmol) Pyrrolidin gegeben und 3 Tage bei Raumtemperatur gerührt. Danach wurde die Reaktionsmischung im Vakuum eingedampft. Auf diese Weise wurde die Titelverbindung als Rohprodukt erhalten, welches ohne weitere Reinigung in die nächste Stufe eingesetzt wurde.

### Intermediat 13

### Herstellung von Cis-4-amino-N-cyclopropyltetrahydrofuran-2-carboxamid Hydrochlorid

Zu 100 mg (0.55 mmol) Cis-4-Aminotetrahydrofuran-2-carbonsäuremethylester Hydrochlorid wurden 0.95 ml (13.7 mmol) Cyclopropylamin gegeben und 3 Tage bei Raumtemperatur gerührt. Danach wurde die Reaktionsmischung im Vakuum eingedampft. Auf diese Weise wurde die Titelverbindung als Rohprodukt erhalten, welches ohne weitere Reinigung in die nächste Stufe eingesetzt wurde.

### Intermediat 14

### Herstellung von Cis-4-amino-N-methyltetrahydrofuran-2-carboxamid Hydrochlorid

Zu 100 mg (0.55 mmol) Cis-4-Aminotetrahydrofuran-2-carbonsäuremethylester Hydrochlorid in 2.5 ml Methanol wurden 0.20 ml (2.75 mmol) einer 40%igen Lösung von Methylamin in Methanol gegeben und 3 Tage bei Raumtemperatur gerührt. Danach wurde die Reaktionsmischung im Vakuum eingedampft. Auf diese Weise wurde die Titelverbindung als Rohprodukt erhalten, welches ohne weitere Reinigung in die nächste Stufe eingesetzt wurde.

### Intermediat 15

### Herstellung von Cis-4-[[3-(3,5-Difluorphenyl)-5-vinyl-4H-isoxazol-5-carbonyl]amino]tetrahydrofuran-2-carbonsäuremethylester

Zu 601 mg (4.14 mmol) cis-4-Aminotetrahydrofuran-2-carbonsäuremethylester Hydrochlorid in 12.5 ml Dichlormethan wurden 1.15 ml Triethylamin gegeben, dann bei 0°C 750 mg (2.76 mmol) Carbonsäurechlorid Intermediat 9 in 12.5 ml Dichlormethan hinzugefügt, 6 h unter Erwärmung auf Raumtemperatur gerührt und schließlich Wasser hinzugegeben. Die organische Phase wurde abgetrennt, mit Natriumsulfat getrocknet und im Vakuum eingedampft. Nach Chromatographie des Eindampfrückstandes an Kieselgel wurden 1.00 g (91 %) cis-4-[[3-(3,5-Difluorphenyl)-5-vinyl-4H-isoxazol-5-carbonyl]amino]tetrahydrofuran-2-carbonsäuremeethylester erhalten.

### Intermediat 16

### Herstellung von Cis-4-[[3-(3,5-Difluorphenyl)-5-methyl-4H-isoxazol-5-carbonyl]amino]tetrahydrofuran-2-carbonsäuremethylester

Zu 262 mg (1.44 mmol) Intermediat 14 in 5 ml Dichlormethan wurden 292 mg (2.88 mmol) Triethylamin gegeben, dann bei 0°C 250 mg (0.96 mmol) Carbonsäurechlorid (Intermediat 4) in 6 ml Dichlormethan hinzugefügt, 6 h unter Erwärmung auf Raumtemperatur gerührt und schließlich Wasser hinzugegeben. Die organische Phase wurde abgetrennt, mit Natriumsulfat getrocknet und im Vakuum eingedampft. Nach Chromatographie des Eindampfrückstandes an Kieselgel wurden 75 mg (21 %) cis-4-[[3-(3,5-Difluorphenyl)-5-methyl-4H-isoxazol-5-carbonyl]amino]tetrahydrofuran-2-carbonsäure-meethylester erhalten.

### Intermediat 17

### Herstellung von Cis-4-[[(5S)-3-(3,5-Difluorphenyl)-5-vinyl-4H-isoxazol-5-carbonyl] amino]tetrahydrofuran-2- carbonsäuremethylester

In Analogie zur Herstellung von Intermediat 9 wurde zunächst das entsprechende Carbonsäurechlorid hergestellt, welches analog Intermediat 15 zur Titelverbindung umgesetzt wurde.

Auf diese Weise wurden aus 246 mg (1.35 mmol) cis-4-Aminotetrahydrofuran-2-carbonsäuremethylester Hydrochlorid und 228 mg (0.90 mmol) Carbonsäurechlorid 110 mg (31 %) cis-4-[[(5S)-3-(3,5-Difluorphenyl)-5-vinyl-4H-isoxazol-5-carbonyl]amino]tetrahydrofuran-2- carbonsäuremethylester erhalten.

### Intermediat 18

### Herstellung von Cis-4-[[3-(3-Fluorphenyl)-5-methyl-4H-isoxazol-5-carbonyl]amino]tetrahydrofuran-2-carbonsäuremethylester

In Analogie zur Herstellung von Intermediat 4 wurde zunächst das entsprechende Carbonsäurechlorid hergestellt, welches analog Intermediat 16 zur Zielverbindung umgesetzt wurde.

Auf diese Weise wurden aus 169 mg (0.93 mmol) cis-4-Aminotetrahydrofuran-2-carbonsäuremethylester Hydrochlorid und 150 mg (0.62 mmol) Carbonsäurechlorid 171 mg (75 %) cis-4-[[3-(3-Fluorphenyl)-5-methyl-4H-isoxazol-5-carbonyl]amino]tetrahydrofuran-2-carbonsäuremethylester erhalten.

### Intermediat 19

### Herstellung von Cis-4-[[3-(3-Fluorphenyl)-5-methyl-4H-isoxazol-5-carbonyl]amino]tetrahydrofuran-2-carbonsäure

85 mg (0.24 mmol) Cis-4-[[3-(3-Fluorphenyl)-5-methyl-4H-isoxazol-5-carbonyl]amino]tetrahydrofuran-2-carbonsäuremethylester wurden mit 1 ml 2 N Salzsäure versetzt und 3 Tage bei Raumtemperatur belassen. Danach wurde die Reaktionsmischung am Rotationsverdampfer eingedampft. Auf diese Weise wurden 33 mg (40 %) cis-4-[[3-(3-Fluorphenyl)-5-methyl-4H-isoxazol-5-carbonyl] amino]tetrahydrofuran-2-carbonsäure erhalten.

### Intermediat 20

### Herstellung von 3-[[3-(3,5-Difluorphenyl)-5-methyl-4H-isoxazol-5-carbonyl]amino]tetrahydrofuran-3-carbonsäuremethylester und 3-[[3-(3,5-Difluorphenyl)-5-methyl-4H-isoxazol-5-carbonyl]amino]tetrahydrofuran-3-carbonsäure

Zunächst wurde das Carbonsäurechlorid Intermediat 4 hergestellt, welches analog Intermediat 16 zur Titelverbindung umgesetzt wurde.

Auf diese Weise wurden aus 787 mg (4.33 mmol) 3-Aminotetrahydrofuran-3-carbonsäuremethylester - Hydrochlorid und 750 mg (2.88 mmol) Carbonsäurechlorid nach Aufarbeitung und Chromatographie an Kieselgel 223 mg (21 %) 3-[[3-(3,5-Difluorphenyl)-5-methyl-4H-isoxazol-5-carbonyl]amino]tetrahydrofuran-3-carbonsäuremethylester und 535 mg (47 %) 3-[[3-(3,5-Difluorphenyl)-5-methyl-4H-isoxazol-5-carbonyl]amino]tetrahydrofuran-3-carbonsäure erhalten.

### Intermediat 21

### Herstellung von 4-[[3-(3,5-Difluorphenyl)-5-methyl-4H-isoxazol-5-carbonyl]amino]tetrahydrofuran-3-carbonsäuremethylester

Zunächst wurde das entsprechende Carbonsäurechlorid hergestellt, welches in Analogie zur Herstellung von Intermediat 16 mit 4-Aminotetrahydrofuran-3-carbonsäuremethylester zur Titelverbindung umgesetzt wurde.

Auf diese Weise wurden aus 419 mg (8.66 mmol) 4-Aminotetrahydrofuran-3-carbonsäuremethylester und 500 mg (1.92 mmol) Carbonsäurechlorid 657 mg (91 %) 4-[[3-(3,5-Difluorphenyl)-5-methyl-4H-isoxazol-5-carbonyl]amino]tetrahydrofuran-3-carbonsäuremethylester erhalten.

### Intermediat 22

### Herstellung von Cis-4-[[(5S)-3-(3,5-Difluorphenyl)-5-vinyl-4H-isoxazol-5-carbonyl] amino]tetrahydrofuran-2- carbonsäure

0.52 g des Methylesters wurden in 8 ml Tetrahydrofuran gelöst und auf 0°C gekühlt. Zu dieser Lösung wurde eine Lösung von 98 mg Lithiumhydroxid in 4 ml Wasser tropfenweise unter Rühren zugegeben. Die Reaktionsmischung wurde unter Rühren innerhalb 1 h auf Raumtemperatur gebracht. Das Reaktionsgemisch wurde mit Wasser verdünnt, mit 0.5 M wässriger Salzsäure angesäuert und mit Ethylacetat extrahiert. Die so erhaltene Ethylacetat-Phase wurde dann getrocknet und im Vakuum eingeengt.

Das Rohprodukt wurde zur weiteren Reinigung in 2 M wässriger Natronlauge aufgenommen und mit Ethylacetat gewaschen. Die wässrige Phase wurde daraufhin mit 2 M wässriger Salzsäure angesäuert und mit Dichlormethan extrahiert. Die Dichlormethan-Phase wurde getrocknet und eingeengt. Man erhielt 0.53 g Carbonsäure (84 % Ausbeute).

### Analytische Daten der Intermediate

| Intermediat Nr. | NMR |
|---|---|
| Intermediat - 16 | ¹H-NMR(400.0 MHz, CDCl3): |
| | δ= 7.518(1.0);7.260(181.8);7.171(1.8);7.166(2.2);7.162(1.3);7.155(1.2);7.151(2.2); 7.146(1.8);6.996(1.0);6.898(0.6);6.883(0.7);6.882(0.7);6.878(1.2);6.876(1.2);6.870( 0.6);6.854(0.6);4.578(1.1);4.573(0.7);4.569(1.1);4.560(0.5);4.554(1.6);4.545(1.3);4. 530(1.0);4.521(0.8);4.065(0.8);4.052(0.8);4.042(1.3);4.029(1. 7);4.017(0.8);4.006(1. 1);3.993(1.0);3.967(0.7);3.962(0.7);3.900(0.6);3.896(0.6);3.834(14.5);3.785(16.0);3 .773(2.0);3.756(1.8);3.730(2.2);3.712(2.0);3.190(1.9);3.182(2.0);3.146(1.6);3.138(1 .8);2.558(0.7);2.541(0.9);2.524(0.8);2.506(0.5);2.019(0.5);1.710(11.3);1.687(10.3); 1.541(35.4);0.008(2.1);0.000(68.8);-0.008(1.9) |
| Intermediat - 17 | ¹H-NMR(400.0 MHz, CDCl3): |
| | δ= 7.262(28.6);7.180(2.2);7.174(2.6);7.171(1.5);7.163(1.5);7.160(2.6);7.154(2.1);6. 903(0.8);6.887(0.9);6.882(1.6);6.876(0.8);6.860(0.8);6.176(2.0);6.150(2.3);6.133(2. 4);6.106(2.5);5.560(1.8);5.558(1.8);5.544(1.7);5.543(1.7);5.517(1.5);5.515(1.5);5.5 01(1.5);5.500(1.4);5.357(1.6);5.356(1.6);5.343(1.5);5.342(1.5);5.330(1.5);5.329(1.5 );5.316(1.4);5.315(1.4);5.299(9.2);4.603(0.5);4.596(0.6);4.591(0.6);4.585(0.6);4.57 9(0.6);4.574(1.1);4.565(1.2);4.558(1.1);4.550(1.4);4.542(0.9);4.534(0.9);4.525(0.8); 4.056(0.8);4.043(0.9);4.041(0.9);4.033(1.3);4.028(0.8);4.020(1.3);4.017(1.4);4.004( 1.1);3.950(0.7);3.946(0.7);3.944(0.7);3.929(1.1);3.922(2.7);3.905(0.5);3.899(2.3);3. 878(2.2);3.856(2.2);3.814(15.9);3.800(16.0);3.322(1.9);3.313(1.9);3.279(1.6);3.270 (1.7);2.566(0.5);2.564(0.5);2.555(0.6);2.549(0.5);2.546(0.5);2.540(0.6);2.538(0.6);2 .531(0.6);2.529(0.6);2.514(0.5);2.511(0.6);2.094(0.6);2.060(0.5);2.052(0.5);2.044(0 .6);1.577(5.9);0.000(11.4) |
| Intermediat - 18 | ¹H-NMR(400.0 MHz, CDCl3): |
| | δ= 7.518(1.2);7.394(1.4);7.389(2.4);7.374(5.0);7.365(4.2);7.358(0.6);7.310(0.7);7.2 68(1.5);7.265(3.0);7.259(207.2);7.253(1.0);7.252(0.8);7.251(0.6);7.145(0.8);7.140( 0.6);7.130(1.0);7.121(0.9);7.113(0.6);7.110(0.6);6.995(1.2);4.577(1.2);4.567(1.1);4. 557(0.6);4.553(1.2);4.550(1.2);4.544(1.0);4.540(1.1);4.526(0.9);4.517(0.8);4.072(0. 8);4.059(0.8);4.048(1.3);4.035(1.6);4.021(0.8);4.010(1.1);3.997(0.9);3.966(0. 7);3.9 61(0.8);3.896(0.6);3.890(0.6);3.835(14.3);3.800(2.0);3.785(2.1);3.778(16.0);3.762( 0.6);3.757(2.3);3.742(2.1);3.223(1.9);3.215(2.1);3.180(1.7);3.172(1.9);2.561(0.8);2. 549(0.5);2.543(0.9);2.526(0.9);2.520(0.5);2.509(0.5);2.503(0.5);2.018(0.6);1.983(0. 5);1.725(0.6);1.714(1.4);1.708(11.3);1.697(0.7);1.686(10.3);1.666(0.6);1.606(2.7);0 .008(2.2);0.000(86.6);-0.008(2.6) |
| Intermediat - 19 | ¹H-NMR(400.0 MHz, d₆-DMSO): |
| | δ= 8.147(0.6);8.130(0.6);8.087(0.6);8.069(0.6);7.526(2.2);7.524(2.3);7.517(3.2);7.5 12(2.2);7.498(2.4);7.469(1.1);7.465(0.9);7.349(0.6);7.341(0.6);7.334(0.7);7.326(1.0 );7.322(0.9);7.315(0.6);7.308(0.6);7.303(0.7);5.756(5.0);4.380(0.6);4.365(1.4);4.35 9(0.8);4.349(1.0);4.343(1.5);4.327(0.9);4.318(0.8);4.303(0.8);3.911(0.7);3.895(0.7); 3.889(0.9);3.873(0.8);3.868(0.7);3.853(0.7);3.847(0.8);3.831(0.7);3.761(1.3);3.740( 1.5);3.717(1.6);3.696(1.9);3.678(0.8);3.671(0.7);3.660(0.8);3.647(0.7);3.638(0.6);3. 625(0.6);3.390(1.8);3.385(1.7);3.346(1.9);3.341(2.0);3.322(43.8);2.670(0.6);2.544( 0.8);2.524(1.3);2.519(2.0);2.510(32.8);2.506(72.0);2.501(100.7);2.497(70.3);2.492( 31.4);2.468(0.8);2.456(1.2);2.451(1.0);2.447(1.1);2.436(0.5);2.425(0.7);2.328(0.6); 2.048(0.5);1.979(0.6);1.963(0.6);1.947(0.6);1.567(1.4);1.534(16.0);0.000(1.8) |
| Intermediat - 20 | ¹H-NMR(400.0 MHz, CDCl3): |
| | δ= 7.260(62.3);7.217(0.8);7.186(1.3);7.181(2.3);7.178(1.4);7.175(1.5);7.171(1.1);7. 167(1.6);7.164(1.3);7.161(2.3);7.155(1.1);6.919(0.5);6.913(0.9);6.897(1.1);6.891(1. 8);6.886(0.9);6.876(0.5);6.870(0.9);4.239(1.7);4.215(2.1);4.203(1.6);4.179(1.9);4.0 12(1.6);4.002(2.5);3.996(1.5);3.993(2.0);3.988(1.1);3.982(2.1);3.977(2.8);3.968(0.9 );3.964(0.9);3.946(1.3);3.922(1.1);3.769(1.9);3.760(1.7);3.724(14.8);3.716(2.1);3.7 06(16.0);3.208(3.1);3.165(2.8);2.624(0.6);2.603(0.5);2.590(0.9);2.584(0.6);2.570(0. 6);2.551(0.8);2.252(0.6);2.236(0.5);2.219(0.5);2.190(0.6);2.174(0.6);1.723(14.0);1. 547(5.9);0.008(0.7);0.000(23.6);-0.008(0.7) |
| Intermediat - 21 | ¹H-NMR(400.0 MHz, CDCl3): |
| | δ= 7.263(33.4);7.258(0.6);7.179(0.5);7.174(1.4);7.168(1.8);7.165(2.3);7.159(2.1);7. 157(1.5);7.154(2.0);7.148(2.2);7.145(1.7);7.140(1.3);7.040(0.7);7.021(0.7);6.916(0. 8);6.910(0.8);6.900(0.8);6.894(1.7);6.888(1.5);6.882(0.6);6.879(0.7);6.872(1.0);6.8 66(0.8);4.715(0.8);4.710(0.6);4.706(0.8);4.700(0.6);4.696(0.8);4.690(0.5);4.225(0.7 );4.203(1.7);4.181(1.8);4.159(0.9);4.149(0.6);4.131(1.6);4.113(1.6);4.095(0.6);4.03 8(0.9);4.023(0.9);4.014(1.8);4.000(1.8);3.991(1.0);3.976(1.1);3.950(1.0);3.934(1.0); 3.927(0.9);3.922(1.0);3.911(0.9);3.907(1.1);3.899(0.9);3.884(0.9);3.792(2.0);3.780( 2.0);3.774(0.6);3.772(0.5);3.757(0.9);3.746(16.0);3.741(1.2);3.737(6.0);3.731(1.2); 3.725(1.0);3.710(0.6);3.704(0.6);3.691(16.0);3.680(1.1);3.665(0.9);3.656(0.8);3.54 1(3.0);3.211(2.8);3.188(0.6);3.168(2.4);3.145(0.5);3.007(1.0);2.045(7.1);1.722(10.8 );1.715(13.2);1.688(2.5);1.277(2.6);1.264(1.8);1.260(5.3);1.242(2.3);0.899(0.9);0.8 82(3.1);0.864(1.2);0.000(13.7) |
| Intermediat - 22 | ¹H-NMR(400.0 MHz, CDCl3): |
| | δ= 7.520(1.5);7.343(1.5);7.322(1.6);7.305(1.7);7.284(1.9);7.261(268.6);7.179(1.2); 7.177(1.2);7.166(5.7);7.164(8.0);7.161(9.1);7.158(8.3);7.147(8.5);7.144(8.8);7.141( 7.4);7.139(5.1);7.129(1.0);6.997(1.5);6.906(1.3);6.900(2.2);6.896(1.6);6.890(2.3);6. 884(3.3);6.878(4.5);6.874(3.2);6.869(4.3);6.863(3.1);6.857(2.3);6.853(1.6);6.847(2. 1);6.841(0.9);6.168(4.3);6.156(4.4);6.141(4.9);6.130(5.1);6.124(5.4);6.113(5.2);6.0 98(5.5);6.086(5.4);5.544(7.8);5.543(6.8);5.532(7.5);5.531(6.8);5.501(6.8);5.500(6.0 );5.489(6.7);5.355(7.2);5.346(7.0);5.328(6.8);5.319(6.4);5.299(16.0);4.609(3.9);4.5 98(7.8);4.588(5.5);4.585(6.1);4.575(8.8);4.565(5.7);4.553(2.3);4.546(2.1);4.540(1.5 );4.527(1.2);4.380(0.5);4.150(1.0);4.132(2.6);4.115(2.7);4.103(3.0);4.097(1.2);4.09 0(3.0);4.079(7.4);4.066(7.2);4.056(4.9);4.042(4.5);4.012(3.1);4.007(3.1);3.988(2.1); 3.983(4.6);3.976(3.1);3.958(1.9);3.952(1.8);3.926(7.3);3.915(7.6);3.884(8.4);3.872( 8.6);3.698(0.6);3.332(7.6);3.325(7.6);3.289(6.6);3.282(6.7);2.681(1.5);2.664(1.6);2. 657(1.6);2.646(2.1);2.640(3.3);2.629(2.0);2.623(3.4);2.616(1.7);2.605(3.7);2.599(1. 8);2.588(1.8);2.582(1.8);2.564(1.6);2.226(1.3);2.219(2.1);2.210(1.3);2.192(1.2);2.1 84(3.0);2.176(3.1);2.167(1.4);2.150(1.1);2.141(1.8);2.133(1.1);2.116(4.1);2.046(10. 8);1.432(3.6);1.304(1.2);1.277(5.0);1.265(5.9);1.259(10.0);1.242(3.6);0.899(3.2);0. 882(11.0);0.864(4.2);0.008(2.5);0.000(95.7);-0.008(2.8) |

### Beispiel I-01

### Herstellung von N- [3 -(Cyclopropylcarbamoyl)tetrahydrofuran-3 -yl] -3 -(3,5-difluorphenyl)-5 -methyl-4H-isoxazol-5-carboxamid

100 mg (0.28 mmol) 3-[[3-(3,5-Difluorphenyl)-5-methyl-4H-isoxazol-5-carbonyl]amino]-tetrahydrofuran-3-carbonsäure, 20 mg (0.35 mmol) Cyclopropylamin, 11.4 mg (0.08 mmol) 1-Hydroxy-1H-benzotriazol und 65 mg (0.33 mmol) 1-(3-Dimethylaminopropyl)-3-ethylcarbodiimid Hydrochlorid wurden bei Raumtemperatur in 5 ml Dichlormethan gegeben. Dann wurden 41 mg (0.35 mmol) N,N-Diethylisopropylamin hinzugefügt und die Reaktionsmischung 16 h bei Raumtemperatur gerührt. Anschließend wurden erneut 2 ml Dichlormethan, 20 mg (0.35 mmol) Cyclopropylamin, 11.4 mg (0.08 mmol) 1-Hydroxy-1H-benzotriazol und 65 mg (0.33 mmol) 1-(3-Dimethylaminopropyl)-3-ethylcarbodiimid hydrochlorid hinzugefügt und 16 h bei Raumtemperatur gerührt. Danach wurde die Reaktionsmischung mit Wasser versetzt, die organische Phase abgetrennt, mit Natriumsulfat getrocknet und im Vakuum eingedampft. Der Eindampfrückstand wurde an Kieselgel chromatographiert (Eluent: Ethylacetat / n-Heptan). Auf diese Weise wurden 91 mg (82 %) der Titelverbindung erhalten.

### Beispiel I-02

### Herstellung von 3-(3,5-Difluorphenyl)-N-[cis-5-(methylcarbamoyl)tetrahydrofuran-3-yl]-5-vinyl-4H-isoxazol-5-carboxamid

100 mg (0.26 mmol) Carbonsäuremethylester Intermediat 15 in 2 ml Methanol wurden mit 0.47 ml (6.57 mmol) einer 40%igen Lösung von Methylamin in Methanol versetzt und 16 h bei Raumtemperatur gerührt. Danach wurde das Reaktionsgemisch im Vakuum eingedampft. Auf diese Weise wurden 73 mg (72 %) der Titelverbindung erhalten.

### Beispiel I-03

### Herstellung von 3-(3,5-Difluorphenyl)-5-methyl-N-[cis-5-(pyrrolidin-1-carbonyl)tetrahydrofuran-3-yl]-4H-isoxazol-5-carboxamid

Zu 100 mg (0.38 mmol) Carbonsäurechlorid Intermediat 4 in 8 ml Dichlormethan wurden 106 mg (0.57 mmol) Carbonsäureamid Intermediat 12 gegeben, dann bei 0°C 117 mg (1.15 mmol) Triethylamin hinzugefügt und 6 h unter Erwärmung auf Raumtemperatur gerührt. Die Reaktionsmischung wurde im Vakum eingedampft und der erhaltene Rückstand durch Chromatographie an Kieselgel (Eluent n-Heptan / Ethylacetat) gereinigt. Auf diese Weise wurden 96 mg (61 %) 3-(3,5-Difluorphenyl)-5-methyl-N-[cis-5-(pyrrolidine-1-carbonyl)tetrahydrofuran-3-yl]-4H-isoxazol-5-carboxamid erhalten.

### Beispiel I-04

### Herstellung von N-[Cis-5-(cyclopropylcarbamoyl)tetrahydrofuran-3-yl]-3-(3,5-difluorphenyl)-5-methyl-4H-isoxazol-5-carboxamid

Zu 100 mg (0.38 mmol) Carbonsäurechlorid Intermediat 4 in 8 ml Dichlormethan wurden 98 mg (0.57 mmol) Carbonsäureamid Intermediat 13 gegeben, dann bei 0°C 117 mg (1.15 mmol) Triethylamin hinzugefügt und 6 h unter Erwärmung auf Raumtemperatur gerührt. Die Reaktionsmischung wurde im Vakum eingedampft und der erhaltene Rückstand durch Chromatographie an Kieselgel (Eluent n-Heptan / Ethylacetat) gereinigt. Auf diese Weise wurden 101 mg (65 %) N-[Cis-5-(cyclopropylcarbamoyl)tetrahydrofuran-3-yl]-3-(3,5-difluorphenyl)-5-methyl-4H-isoxazol-5-carboxamid erhalten.

### Beispiel I-05

### Herstellung von 3-(3,5-Difluorphenyl)-5-methyl-N-[cis--5-(methylcarbamoyl)tetrahydrofuran-3-yl]-4H-isoxazol-5-carboxamid

Zu 100 mg (0.38 mmol) Carbonsäurechlorid Intermediat 4 in 8 ml Dichlormethan wurden 83 mg (0.57 mmol) Carbonsäureamid Intermediat 14 gegeben, dann bei 0°C 117 mg (1.15 mmol) Triethylamin hinzugefügt und 6 h unter Erwärmung auf Raumtemperatur gerührt. Die Reaktionsmischung wurde im Vakum eingedampft und der erhaltene Rückstand durch Chromatographie an Kieselgel (Eluent n-Heptan / Ethylacetat) gereinigt. Auf diese Weise wurden 50 mg (34 %) 3-(3,5-Difluorphenyl)-5-methyl-N-[cis-5-(methylcarbamoyl)tetrahydrofuran-3-yl]-4H-isoxazol-5-carboxamid erhalten.

### Beispiel I-06

### Herstellung von 3-(3,5-Difluorphenyl)-N-[cis-5-(pyrrolidin-1-carbonyl)tetrahydrofuran-3-yl]-5-vinyl-4H-isoxazol-5-carboxamid

100 mg (0.26 mmol) Carbonsäuremethylester Intermediat 15 wurden mit 0.52 ml (6.57 mmol) Pyrrolidin versetzt und 16 h bei Raumtemperatur gerührt. Danach wurde das Reaktionsgemisch im Vakuum eingedampft und der erhaltene Rückstand durch Chromatographie an Kieselgel (Eluent n-Heptan / Ethylacetat) gereinigt. Auf diese Weise wurden 57 mg (48 %) der Titelverbindung erhalten.

### Beispiel I-07

### Herstellung von 3-(3,5-Difluorphenyl)-5-methyl-N-[4-(methylcarbamoyl)tetrahydrofuran-3-yl]-4H-isoxazol-5-carboxamid

Zu 150 mg (0.40 mmol) Carbonsäuremethylester Intermediat 21 in 5 ml Methanol wurden 0.15 ml (2.00 mmol) einer 40%igen Lösung von Methylamin in Methanol gegeben und 3 Tage bei Raumtemperatur gerührt. Danach wurde die Reaktionsmischung im Vakuum eingedampft. Auf diese Weise wurden 125 mg (84 %) der rohen Titelverbindung erhalten.

### Beispiel I-08

### Herstellung von N-[4-(Cyclopropylcarbamoyl)tetrahydrofuran-3-yl]-3-(3,5-difluorphenyl)-5-methyl-4H-isoxazol-5-carboxamid

150 mg (0.40 mmol) Carbonsäuremethylester Intermediat 21 wurden mit 0.51 ml (6.10 mmol) Cyclopropylamin versetzt und 3 Tage bei Raumtemperatur gerührt. Danach wurde das Reaktionsgemisch im Vakuum eingedampft. Auf diese Weise wurden 193 mg der rohen Titelverbindung erhalten.

### Beispiel I-09

### Herstellung von 3-(3,5-Difluorphenyl)-5-methyl-N-[4-(pyrrolidine-1-carbonyl)tetrahydrofuran-3-yl]-4H-isoxazol-5-carboxamid

150 mg (0.40 mmol) Carbonsäuremethylester Intermediat 21 wurden mit 0.51 ml (6.10 mmol) Pyrrolidin versetzt und 3 Tage bei Raumtemperatur gerührt. Danach wurde das Reaktionsgemisch im Vakuum eingedampft und der erhaltene Rückstand durch Chromatographie an Kieselgel (Eluent n-Heptan / Ethylacetat) gereinigt. Auf diese Weise wurden 58 mg (34 %) der Titelverbindung erhalten.

### Beispiel I-10

### Herstellung von 3 -(3 -Fluorphenyl)-N- [cis-5 -(methoxycarbamoyl)tetrahydrofuran-3 -yl] -5-methyl-4H-isoxazol-5-carboxamid

0.11 g Carbonsäure Intermediat 19 und 30 mg (Aminooxy)methanhydrochlorid wurden in Dichlormethan gelöst und nacheinander mit 0.08 g Triethylamin und 0.28 g einer Lösung von Propylphosphonsäureanhydrid (50%ig) in Tetrahydrofuran versetzt. Die Reaktionsmischung wurde daraufhin 2.5 h bei Raumtemperatur gerührt. Dann wurde das Reaktionsgemisch mit gesättigter wässriger Natriumhydrogencarbonat-Lösung gewaschen, getrocknet und im Vakuum eingeengt. Das Rohprodukt wurde mittels Chromatographie an Kieselgel gereinigt. So wurde 0.10 g (86 %) Hydroxamsäureester erhalten.

### Beispiel I-11

### Herstellung von (5S)-3-(3,5-Difluorphenyl)-N-[cis-5-(methoxycarbamoyl)tetrahydrofuran-3-yl]-5-vinyl-4H-isoxazol-5-carboxamid

100 mg Carbonsäure Intermediat 22 und 27 mg (Aminooxy)methanhydrochlorid wurden in Dichlormethan gelöst und nacheinander mit 0.08 g Triethylamin und 0.26 g einer Lösung von Propylphosphonsäureanhydrid (50%ig) in Tetrahydrofuran versetzt. Die Reaktionsmischung wurde daraufhin 3 h bei Raumtemperatur gerührt. Dann wurde das Reaktionsgemisch mit gesättigter wässriger Natriumhydrogencarbonat-Lösung gewaschen, getrocknet und im Vakuum eingeengt. Das Rohprodukt wurde mittels Chromatographie an Kieselgel gereinigt. Man erhielt 96 mg Hydroxamsäureester (89 % Ausbeute).

### Beispiel I-12

### Herstellung von (5S)-3-(3,5-Difluorphenyl)-N-[cis-5-(ethoxycarbamoyl)tetrahydrofuran-3-yl]-5-vinyl-4H-isoxazol-5-carboxamid

80 mg Carbonsäure Intermediat 22 und 26 mg (Aminooxy)ethanhydrochlorid wurden in Dichlormethan gelöst und nacheinander mit 0.07 g Triethylamin und 0.21 g einer Lösung von Propylphosphonsäureanhydrid (50%ig) in Tetrahydrofuran versetzt. Die Reaktionsmischung wurde daraufhin 3 h bei Raumtemperatur gerührt. Dann wurde das Reaktionsgemisch mit gesättigter wässriger Natriumhydrogencarbonat-Lösung gewaschen, getrocknet und im Vakuum eingeengt. Das Rohprodukt wurde mittels Chromatographie an Kieselgel gereinigt. Man erhielt 87 mg Hydroxamsäureester (98 % Ausbeute).

### Beispiel I-13

### Herstellung von (5S)-3-(3,5-Difluorphenyl)-N-[cis-5-(isopropoxycarbamoyl)tetrahydrofuran-3-yl]-5-vinyl-4H-isoxazol-5-carboxamid

96 mg Carbonsäure Intermediat 22 und 29 mg 2-(Aminooxy)propanhydrochlorid wurden in Dichlormethan gelöst und nacheinander mit 0.07 g Triethylamin und 0.21 g einer Lösung von Propylphosphonsäureanhydrid (50%ig) in Tetrahydrofuran versetzt. Die Reaktionsmischung wurde daraufhin 3 h bei Raumtemperatur gerührt. Dann wurde das Reaktionsgemisch mit gesättigter wässriger Natriumhydrogencarbonat-Lösung gewaschen, getrocknet und im Vakuum eingeengt. Das Rohprodukt wurde mittels Chromatographie an Kieselgel gereinigt. Man erhielt 95 mg Hydroxamsäureester (86 % Ausbeute).

### Beispiel I-14

### Herstellung von (5S)-3-(3,5-Difluorphenyl)-N-[cis-5-(trifluormethylsulfonylcarbamoyl)tetrahydrofuran-3-yl]-5-vinyl-4H-isoxazol-5-carboxamid

0.11g Carbonsäure Intermediat 22 und 6 mg 4-Dimethylaminopyridin wurden in 5 ml Dichlormethan gelöst. Daraufhin wurden unter Rühren bei Raumtemperatur 65 mg 1-(3-Dimethylaminopropyl)-3-ethylcarbodiimid hydrochlorid gefolgt von 0.17 g 1,1,1-Trifluormethansulfonamid zugegeben, und das Reaktionsgemisch wurde 2 h bei Raumtemperatur und dann 3 Tage bei dieser Temperatur rühren gelassen. Das Reaktionsgemisch wurde mit 0.5 M wässriger Salzsäure gewaschen, getrocknet und im Vakuum eingeengt. Das Rohprodukt wurde mittels Chromatographie an Kieselgel gereinigt. Man erhielt 94 mg Acylsulfonamid (81 % Ausbeute).

### Beispiel I-15

### Herstellung von (5S)-3-(3,5-Difluorphenyl)-N-[cis-5-(dimethylaminocarbamoyl)tetrahydrofuran-3-yl]-5-vinyl-4H-isoxazol-5-carboxamid und

### Beispiel I-16

### Herstellung von (5S)-3-(3,5-Difluorphenyl)-N-[cis-5-(methylsulfonylcarbamoyl)tetrahydrofuran-3-yl]-5-vinyl-4H-isoxazol-5-carboxamid

0.12 g Carbonsäure Intermediat 22 und 94 mg 1-Hydroxybenzotriazol 86%ig wurden in 3ml Dichlormethan gelöst und bei Raumtemperatur 15min lang gerührt. Dann wurden 0,16 g Methansulfonamid zugegeben, gefolgt von einer Lösung von 94 mg 1-(3-Dimethylaminopropyl)-3-ethylcarbodiimid hydrochlorid und 0,09 g N-Ethyl-N-isopropylpropan-2-amin in 3 ml Dichlormethan. Das Reaktionsgemisch wurde 0,5 h bei Raumtemperatur gerührt und 2 Tage stehen gelassen. Dann wurde das Reaktionsgemisch mit 0.5 M wässriger Salzsäure gewaschen, getrocknet und im Vakuum eingeengt. Das Rohprodukt wurde mittels Chromatographie an Kieselgel gereinigt. Man erhielt 43 mg Acylsulfonamid (29 % Ausbeute).

### Beispiel I-17

### Herstellung von (5S)-3-(3,5-Difluorphenyl)-N-[cis-5-(dimethylcarbamoyl)tetrahydrofuran-3-yl]-5-vinyl-4H-isoxazol-5-carboxamid

97 mg Carbonsäure Intermediat 22 und 24 mg 1,1-Dimethylhydrazin wurden in 5 ml Dichlormethan gelöst. Daraufhin wurden unter Rühren bei Raumtemperatur 80 mg Triethylamin gefolgt von 0.51 g einer Lösung von Propylphosphonsäureanhydrid (50%ig) in Tetrahydrofuran zugegeben, und das Reaktionsgemisch wurde anschließend 4 h bei Raumtemperatur gerührt. Dann wurde das Reaktionsgemisch mit gesättigter wässriger Natriumhydrogencarbonat-Lösung gewaschen, getrocknet und im Vakuum eingeengt. Das Rohprodukt wurde mittels Chromatographie an Kieselgel gereinigt. Man erhielt 0.08 g des Dimethylhydrazids I-15 (73 % Ausbeute) sowie 0.01 g des Dimethylamids I-17 (9 % Ausbeute).

### Beispiel I-18

### Herstellung von (5S)-N-[Cis-5-(cyclopropylcarbamoyl)tetrahydrofuran-3-yl]-3-(3,5-difluorphenyl)-5-vinyl-4H-isoxazol-5-carboxamid

93 mg Carbonsäure Intermediat 22 und 17 mg Cyclopropylamin wurden in 5 ml Dichlormethan gelöst. Daraufhin wurden unter Rühren bei Raumtemperatur 77 mg Triethylamin gefolgt von 0.48 g einer Lösung von Propylphosphonsäureanhydrid (50%ig) in Tetrahydrofuran zugegeben, und das Reaktionsgemisch wurde anschließend 2.5 h bei Raumtemperatur gerührt. Dann wurde das Reaktionsgemisch mit gesättigter wässriger Natriumhydrogencarbonat-Lösung gewaschen, getrocknet und im Vakuum eingeengt. Das Rohprodukt wurde mittels Chromatographie an Kieselgel gereinigt. Man erhielt 95 mg des Cyclopropylamids (91 % Ausbeute).

### Beispiel I-19

### Herstellung von tert.-Butyl N-[[cis-4-[[(5S)-3-(3,5-difluorphenyl)-5-vinyl-4H-isoxazol-5-carbonyl]amino]tetrahydrofuran-2-carbonyl]amino]carbamat

0.18 g Carbonsäure Intermediat 22 und 84 mg 1-Hydroxybenzotriazol (86%ig) wurden in 8 ml Dichlormethan gelöst und bei Raumtemperatur 30 min lang gerührt. Dann wurden 77 mg tert-Butylhydrazincarboxylat zugegeben, gefolgt von einer Lösung von 141 mg 1-(3-Dimethylaminopropyl)-3-ethylcarbodiimid hydrochlorid und 95 mg N-Ethyl-N-isopropylpropan-2-amin in 4ml Dichlormethan. Das Reaktionsgemisch wurde 0.5 h bei Raumtemperatur gerührt, über Nacht stehen gelassen und weitere 6 h bei Raumtemperatur nachgerührt. Dann wurde das Reaktionsgemisch mit 0.5 M wässriger Salzsäure gewaschen, getrocknet und im Vakuum eingeengt. Das Rohprodukt wurde mittels Chromatographie an Kieselgel gereinigt. Man erhielt 112 mg Produkt (47 % Ausbeute).

### Beispiele I-20 bis I-30 aus Tabelle 1

Die Herstellung erfolgte in Analogie zu den oben angeführten Herstellungsbeispielen.

### Analytische Daten der Beispiele I-01 - I-30.

| | |
|---|---|
| I-01 | ¹H-NMR(400.0 MHz, CDCl3): |
| | δ= 7.717(1.5);7.519(1.0);7.260(176.6);7.193(0.5);7.181(2.9);7.178(1.9);7.175(3.2);7.172(1.8); 7.164(1.7);7.161(3.3);7.155(2.8);7.143(0.5);6.996(1.0);6.908(0.5);6.904(0.6);6.902(0.9);6.889( 0.7);6.886(1.0);6.883(1.2);6.880(1.7);6.877(0.7);6.875(0.8);6.864(0.5);6.861(0.6);6.859(0.9);6. 483(0.5);6.466(0.7);5.299(5.5);4.073(2.4);4.060(2.0);4.049(5.0);4.039(2.9);4.032(2.4);4.026(1. 2);4.022(1.3);4.016(0.9);4.014(1.0);3.991(2.7);3.988(3.6);3.967(1.6);3.964(2.1);3.768(2.8);3.7 58(1.9);3.725(3.3);3.715(2.2);3.201(2.9);3.192(2.0);3.158(2.6);3.149(1.8);2.719(0.6);2.709(1.1 );2.699(1.2);2.691(1.3);2.682(0.9);2.414(1.0);2.399(2.0);2.388(1.0);2.383(2.1);2.379(1.6);2.36 8(2.0);2.360(0.8);2.347(0.8);2.335(0.6);1.720(10.9);1.709(16.0);1.545(3.5);1.259(0.6);0.790(0. 6);0.785(0.8);0.781(1.2);0.778(1.9);0.774(1.7);0.772(2.7);0.768(1.6);0.764(1.6);0.760(2.0);0.7 57(2.0);0.754(2.2);0.750(1.0);0.743(0.6);0.739(0.6);0.464(0.6);0.461(1.0);0.454(1.2);0.451(2.4 );0.448(2.6);0.444(2.4);0.441(1.9);0.438(2.5);0.435(1.8);0.430(1.0);0.421(0.6);0.008(2.4);0.00 0(80.2);-0.008(2.1) |
| I-02 | ¹H-NMR(400.0 MHz, CDCl3): |
| | δ= 7.264(29.0);7.174(1.0);7.170(1.6);7.168(1.6);7.165(1.6);7.162(0.8);7.157(0.8);7.154(1.6);7. 151(1.6);7.148(1.4);7.145(1.0);6.924(0.6);6.918(0.7);6.913(0.9);6.907(0.9);6.902(0.8);6.897(0. 9);6.891(0.9);6.886(1.0);6.880(0.6);6.186(0.9);6.159(1.0);6.142(1.1);6.128(0.8);6.116(1.1);6.1 01(0.9);6.085(0.9);6.058(1.0);5.532(1.4);5.531(1.5);5.521(1.3);5.519(1.3);5.489(1.3);5.488(1.2 );5.478(1.2);5.476(1.2);5.367(1.2);5.340(2.2);5.315(1.1);5.313(1.2);5.300(16.0);4.460(0.6);4.4 49(0.8);4.437(1.3);4.426(1.2);4.414(0.7);4.403(0.5);4.027(0.7);4.015(0.7);4.004(1.0);3.991(1.0 );3.987(0.6);3.975(0.6);3.963(0.9);3.951(0.8);3.934(1.4);3.908(0.5);3.904(0.6);3.891(1.6);3.87 0(1.6);3.826(2.0);3.322(1.5);3.303(1.3);3.278(1.3);3.260(1.1);2.900(3.4);2.899(3.4);2.888(3.4); 2.887(3.3);2.854(3.9);2.853(4.0);2.848(1.0);2.847(1.0);2.841(4.0);2.840(3.8);2.836(1.0);2.835( 0.9);2.812(1.5);2.811(1.6);2.799(1.5);2.575(0.5);2.198(0.6);1.978(3.0);0.000(12.6) |
| I-03 | Zwei Diastereomere liegen in Mischung vor, die Angabe der Signalintegrale ist gerundet angegeben. |
| | ¹H-NMR(400.0 MHz, CDCl3): |
| | δ= 8.51 - 8.39 (br, 1 H), 7.21 - 7.14 (m, 2 H), 6.88 - 6.82 (m, 1 H), 4.72 - 4.69 (m, 1 H), 4.63 - 4.54 (m, 1 H), 4.02 - 3.93 (m, 1 H), 3.89 - 3.67 (m, 3 H), 3.58 - 3.44 (m, 3 H), 3,16 - 2.88 (m, 2 H), 2.34 - 2.20 (m, 2 H), 2.01 - 1.80 (m, 3 H), 1.72 - 1.70 (m, 3 H). |
| I-04 | Zwei Diastereomere liegen in Mischung vor, die Angabe der Signalintegrale ist gerundet angegeben. |
| | ¹H-NMR(400.0 MHz, CDCl3): |
| | δ= 7.20 - 7.13 (m, 2 H), 6.92 - 6.85 (m, 1 H), 6.77 - 6.69 (br, 1 H), 4.49 - 4.34 (m, 2 H), 4.02 - 3.93 (m, 1 H), 3.88 - 3.69 (m, 2 H), 3.20 - 3.05 (m, 1 H), 2.99 - 2.65 (m, 2 H), 2.62 - 2.48 (m, 1 H), 2.26 - 2.08 (m, 1 H), 1.71 - 1.69 (m, 3 H), 0.88 - 0.73 (m, 2 H), 0.65 - 0.47 (m, 2 H). |
| I-05 | ¹H-NMR(499.9 MHz, d₆-DMSO): |
| | δ= 7.483(3.5);7.469(4.2);7.294(4.8);7.269(11.7);7.254(8.5);7.240(6.2);7.219(14.9);7.205(6.8); 4.962(2.7);4.946(2.8);4.830(2.2);4.800(3.5);4.776(2.4);4.551(4.7);4.542(9.5);4.536(6.4);4.526( 7.7);4.519(10.5);4.499(2.9);4.457(3.7);4.423(5.8);4.392(3.1);4.111(2.9);4.077(2.4);3.750(4.0); 3.714(3.6);3.368(2.0);3.344(3.1);3.320(2.0);3.298(50.8);3.276(0.4);2.923(1.6);2.898(4.4);2.87 5(7.2);2.844(8.7);2.814(7.7);2.806(7.5);2.784(16.0);2.776(7.7);2.760(2.3);2.753(3.0);2.744(1.3 );2.616(2.0);2.502(12.6);2.499(17.3);2.496(13.3);2.084(0.5);1.706(9.0);1.676(9.6);1.647(12.4); 1.403(6.2);1.384(5.6);1.352(7.1);1.335(8.5);1.315(8.2);1.298(5.5);1.179(3.5);1.174(3.5);0.000( 8.4) |
| I-06 | ¹H-NMR(400.0 MHz, CDCl3): |
| | δ= 7.265(29.8);7.200(1.0);7.197(0.7);7.194(1.2);7.191(0.7);7.183(0.7);7.180(1.8);7.178(1.2);7. 174(1.7);7.164(0.6);7.160(1.0);7.155(0.8);6.877(0.8);6.861(0.9);6.855(1.6);6.849(0.8);6.833(0. 8);6.215(0.8);6.188(1.0);6.186(1.0);6.172(1.0);6.159(1.0);6.145(1.1);6.142(1.1);6.116(1.1);5.5 93(1.2);5.591(1.2);5.562(1.4);5.560(1.5);5.550(1.1);5.548(1.1);5.519(1.3);5.517(1.3);5.335(1.0 );5.333(1.1);5.324(1.2);5.323(1.2);5.308(1.1);5.306(1.2);5.300(16.0);5.298(2.0);5.296(1.4);4.7 15(1.0);4.707(0.8);4.694(0.7);4.687(1.0);4.601(0.5);4.014(0.7);4.001(0.7);3.991(1.0);3.982(0.8 );3.978(1.0);3.969(0.7);3.959(1.0);3.951(0.5);3.946(0.9);3.942(1.7);3.900(1.9);3.888(1.3);3.87 2(0.6);3.867(0.6);3.845(1.8);3.810(0.5);3.805(0.6);3.712(0.6);3.687(0.8);3.539(1.1);3.534(0.7); 3.529(0.6);3.520(1.4);3.514(1.1);3.503(0.8);3.494(0.9);3.480(0.8);3.476(0.7);3.467(0.6);3.462( 0.6);3.455(0.6);3.298(1.4);3.294(1.6);3.267(0.6);3.256(1.3);3.251(1.5);2.956(1.1);2.321(0.6);2. 313(0.7);2.303(1.5);2.291(1.0);2.284(0.6);2.280(0.6);2.250(0.6);2.050(1.5);2.014(0.7);1.991(0. 6);1.987(0.6);1.979(0.8);1.971(0.8);1.964(1.1);1.957(0.8);1.954(0.8);1.950(1.1);1.933(0.7);1.8 98(0.8);1.882(1.2);1.866(1.2);1.847(0.8);1.778(0.6);1.769(0.7);1.761(1.4);1.753(0.7);1.744(0.5 );0.000(12.3) |
| I-07 | ¹H-NMR(400.0 MHz, d₆-DMSO): |
| | δ= 8.328(1.3);8.314(1.6);8.310(1.6);8.296(1.2);7.910(0.6);7.899(0.8);7.888(0.9);7.864(0.6);7.8 55(0.8);7.844(0.8);7.419(1.9);7.414(3.9);7.410(2.2);7.405(4.3);7.400(5.6);7.396(6.0);7.388(4.2 );7.384(2.0);7.380(4.0);7.377(3.5);7.370(0.9);5.756(16.0);4.467(0.6);4.453(1.4);4.439(1.8);4.4 35(1.6);4.426(0.9);4.422(1.3);4.418(0.9);4.023(1.1);4.016(1.2);4.002(2.5);3.996(2.6);3.981(1.3 );3.975(1.4);3.910(0.7);3.902(0.7);3.875(0.5);3.871(0.5);3.856(1.7);3.850(0.8);3.843(0.7);3.84 0(1.5);3.835(2.0);3.824(1.4);3.818(1.5);3.806(1.6);3.802(1.6);3.793(0.6);3.784(3.6);3.775(2.7); 3.747(0.8);3.740(3.1);3.731(3.2);3.703(0.8);3.669(1.6);3.661(0.7);3.653(1.6);3.648(1.5);3.644( 1.7);3.632(1.6);3.628(1.8);3.623(1.6);3.617(0.9);3.607(1.8);3.588(1.5);3.575(1.5);3.566(1.3);3. 558(0.6);3.553(1.3);3.507(1.4);3.494(1.4);3.486(1.3);3.472(1.2);3.420(0.7);3.404(0.8);3.389(3. 1);3.380(2.9);3.359(0.8);3.345(2.8);3.336(3.0);3.320(19.5);3.006(0.6);2.989(0.8);2.985(0.8);2. 976(0.8);2.972(0.9);2.954(0.9);2.941(0.7);2.938(0.8);2. 933(0.8);2. 925(0.8);2.920(0.8);2.917(0. 7);2.904(0.6);2.602(0.6);2.594(2.7);2.586(10.1);2.583(3.8);2.574(9.8);2.538(10.5);2.526(11.0); 2.520(2.0);2.512(23.8);2.507(53.0);2.502(74.2);2.498(51.2);2.493(22.4);2.452(0.6);2.394(2.3); 2.383(2.4);2.366(4.0);2.150(1.1);1.553(15.0);1.541(15.0);1.514(3.7);1.235(1.0);0.000(6.6) |
| I-08 | ¹H-NMR(400.0 MHz, d₆-DMSO): |
| | δ= 8.293(1.4);8.025(1.0);7.958(1.2);7.839(1.0);7.412(3.2);7.403(4.8);7.397(5.0);7.387(3.7);7.3 78(3.0);4.534(1.3);4.521(3.1);4.508(1.5);4.440(1.3);4.426(1.9);4.408(1.1);3.999(1.0);3.993(1.1 );3.979(2.1);3.972(2.3);3.958(1.1);3.951(1.2);3.894(0.9);3.857(1.6);3.840(1.6);3.835(1.9);3.81 9(2.3);3.798(1.4);3.782(2.5);3.756(2.4);3.738(2.5);3.712(3.0);3.700(0.9);3.670(1.4);3.654(1.5); 3.643(1.6);3.632(1.8);3.626(1.6);3.622(1.7);3.605(1.5);3.599(2.0);3.583(3.3);3.570(3.3);3.566( 2.0);3.560(1.6);3.553(2.3);3.538(1.2);3.509(1.1);3.494(1.3);3.487(1.2);3.473(1.2);3.436(1.1);3. 430(1.3);3.394(3.2);3.378(2.7);3.367(1.5);3.349(2.4);3.333(4.5);3.316(477.3);3.266(2.6);2.909 (0.8);2.892(0.9);2.847(0.9);2.675(3.0);2.670(4.3);2.665(3.0);2.615(2.0);2.606(1.5);2.592(1.6);2 .583(1.1);2.574(1.8);2.564(1.7);2.556(2.2);2.546(2.4);2.536(1.5);2.523(10.9);2.519(15.3);2.51 0(219.7);2.505(486.3);2.501(679.4);2.496(468.6);2.492(204.7);2.455(3.4);2.451(4.1);2.446(3.1 );2.401(1.2);2.332(2.7);2.328(4.1);2.323(2.8);2.177(2.9);2.161(5.6);2.144(2.6);2.074(1.2);2.00 9(0.8);1.990(0.9);1.741(11.8);1.560(3.5);1.549(11.6);1.538(16.0);1.236(1.0);0.980(1.2);0.961( 2.4);0.942(1.2);0.614(1.2);0.604(2.0);0.600(2.8);0.588(3.2);0.584(4.7);0.579(3.4);0.571(3.1);0. 566(4.0);0.554(2.7);0.549(2.1);0.543(1.7);0.536(1.8);0.530(1.6);0.526(1.5);0.380(1.4);0.368(3. 4);0.363(3.5);0.358(4.0);0.346(2.3);0.341(2.3);0.314(0.9);0.301(2.3);0.296(2.5);0.292(2.6);0.2 86(2.1);0.242(0.8);0.008(1.1);0.000(51.9);-0.008(1.6) |
| I-09 | ¹H-NMR(400.0 MHz, CDCl3): |
| | δ= 7.520(1.8);7.280(0.5);7.278(0.6);7.277(0.6);7.276(0.8);7.275(0.8);7.274(0.9);7.274(1.1);7.2 73(1.2);7.272(1.2);7.271(1.4);7.271(1.4);7.270(1.5);7.269(2.0);7.268(2.4);7.267(2.8);7.266(3.3 );7.266(4.0);7.265(5.4);7.264(7.3);7.261(310.2);7.258(4.3);7.257(2.6);7.256(2.1);7.255(1.5);7. 254(1.2);7.254(0.9);7.253(0.8);7.252(0.9);7.251(0.7);7.250(0.5);7.227(0.5);7.211(1.2);7.184(0. 8);7.176(2.3);7.170(2.3);7.164(2.9);7.158(3.4);7.150(2.2);7.148(2.5);7.144(3.1);7.138(2.6);7.1 25(1.0);7.094(0.5);6.997(1.8);6.925(0.8);6.919(0.7);6.913(0.8);6.909(0.9);6.904(1.5);6.897(1.6 );6.892(1.7);6.886(0.9);6.882(0.9);6.875(1.2);6.870(1.1);4.652(0.5);4.645(0.6);4.639(0.6);4.57 2(0.8);4.567(0.7);4.246(0.6);4.239(0.8);4.227(0.7);4.218(1.6);4.205(1.0);4.197(1.0);4.184(1.5); 4.163(0.9);4.083(0.6);4.077(0.5);4.062(1.5);4.054(0.7);4.040(1.1);4.027(1.0);4.021(0.5);4.014( 1.5);4.005(1.7);3.991(1.8);3.981(1.3);3.968(1.2);3.947(1.1);3.932(1.2);3.926(1.0);3.915(1.2);3. 910(1.2);3.899(1.2);3.893(1.0);3.877(1.0);3.833(0.9);3.828(1.0);3.809(0.8);3.804(0.8);3.791(1. 1);3.782(3.1);3.767(3.4);3.742(1.8);3.739(3.1);3.733(0.9);3.729(1.5);3.724(3.5);3.710(1.0);3.6 99(2.4);3.686(1.9);3.669(0.8);3.652(0.6);3.551(0.9);3.538(1.2);3.520(2.0);3.509(1.1);3.503(1.5 );3.483(1.4);3.468(1.8);3.450(2.0);3.434(1.0);3.422(1.4);3.404(1.4);3.389(0.9);3.382(1.0);3.37 2(1.0);3.353(0.9);3.332(0.6);3.313(0.8);3.306(0.7);3.288(0.6);3.220(2.7);3.212(2.6);3.177(2.5); 3.169(2.5);3.166(1.7);3.152(1.3);3.123(1.6);3.109(1.8);3.036(0.5);3.031(0.7);2.022(0.6);2.005( 1.4);1.990(1.7);1.973(1.4);1.957(0.6);1.927(0.8);1.910(1.3);1.891(1.9);1.874(1.9);1.868(1.8);1. 860(2.1);1.842(1.2);1.825(0.6);1.718(15.5);1.715(16.0);1.698(6.4);1.652(6.8);1.255(0.6);0.008 (4.0);0.006(1.3);0.005(1.5);0.004(2.1);0.000(138.3);-0.005(1.2);-0.006(1.0);-0.007(0.8);-0.008(3.7);-0.050(0.6) |
| I-10 | ¹H-NMR(400.6 MHz, CDCl3): |
| | δ= 9.764(1.2);9.707(1.3);7.404(1.4);7.397(1.6);7.393(1.6);7.390(1.7);7.384(2.1);7.379(2.0);7.3 69(3.1);7.365(4.8);7.360(6.5);7.356(6.0);7.352(4.6);7.346(2.0);7.340(3.2);7.310(1.3);7.148(0.6 );7.142(1.1);7.136(1.1);7.129(1.1);7.127(1.1);7.121(1.7);7.115(1.2);7.112(1.2);7.106(1.0);7.09 8(1.0);4.532(0.7);4.521(0.9);4.508(1.2);4.498(1.8);4.488(1.7);4.477(1.8);4.465(1.5);4.026(0.7); 4.013(0.8);4.002(1.2);3.989(1.1);3.973(0.7);3.960(0.7);3.949(1.3);3.943(1.4);3.936(2.0);3.920( 0.8);3.912(0.7);3.890(1.2);3.885(1.1);3.866(0.8);3.861(0.8);3.840(3.0);3.834(12.0);3.808(1.2); 3.803(1.5);3.797(2.3);3.790(2.3);3.781(1.2);3.778(1.5);3.774(0.9);3.758(0.7);3.743(15.7);3.33 5(0.8);3.316(0.8);3.274(0.9);3.271(0.9);3.240(2.5);3.227(2.5);3.197(2.2);3.184(2.2);2.627(0.5); 2.622(0.7);2.610(0.7);2.605(0.8);2.594(0.8);2.588(0.9);2.570(0.9);2.327(0.6);2.317(0.7);2.284( 0.5);2.239(0.6);1.713(1.1);1.707(2.2);1.702(1.9);1.693(15.1);1.689(16.0);1.287(0.6);1.259(2.8) ;0.000(0.9) |
| I-11 | ¹H-NMR(400.0 MHz, CDCl₃): |
| | δ= 8.80 (br d, J=4.13 Hz, 1 H), 7.20 - 7.13 (m, 2 H), 7.00 - 6.93 (m, 1 H), 6.92 - 6.85 (m, 1 H), 6.19 - 6.06 (m, 1 H), 5.51 (dd, J=17.17, 1.91 Hz, 1 H), 5.34 (dd, J=10.65, 8.42 Hz, 1 H), 4.54 - 4.41 (m, 2 H), 4.18 (sept, J=6.04 Hz, 1 H), 4.05 - 3.96 (m, 1 H), 3.93 - 3.77 (m, 2 H), 3.34 - 3.26 (m, 1 H), 2.71 - 2.58 (m, 1 H), 2.22 - 2.13 (m, 1 H), 1.32 - 1.22 (m, 6 H) |
| I-12 | ¹H-NMR(400.0 MHz, CDCl₃): |
| | δ= 8.93 (br d, J=7.31 Hz, 1 H), 7.20 - 7.13 (m, 2 H), 7.00 - 6.93 (m, 1 H), 6.93 - 6.85 (m, 1 H), 6.20 - 6.06 (m, 1 H), 5.51 (dd, J=17.32, 3.66 Hz, 1 H), 5.34 (dd, J=10.33, 8.42 Hz, 1 H), 4.54 - 4.41 (m, 2 H), 4.08 - 3.95 (m, 3 H), 3.93 - 3.79 (m, 2 H), 3.34 - 3.27 (m, 1 H), 2.69 - 2.56 (m, 1 H), 2.25 - 2.17 (m, 1 H), 1.34 - 1.24 (m, 3 H) |
| I-13 | ¹H-NMR(400.0 MHz, CDCl₃): |
| | δ= 9.01 (br d, J=10.49 Hz, 1 H), 7.20 - 7.12 (m, 2 H), 7.02 - 6.94 (m, 1 H), 6.93 - 6.85 (m, 1 H), 6.19 - 6.07 (m, 1 H), 5.51 (dd, J=17.33, 4.29 Hz, 1 H), 5.35 (dd, J=10.65, 8.11 Hz, 1 H), 4.55 - 4.41 (m, 2 H), 4.03 - 3.95 (m, 1 H), 3.94 - 3.79 (m, 5 H), 3.34 - 3.26 (m, 1 H), 2.68 - 2.55 (m, 1 H), 2.27 - 2.19 (m, 1 H). |
| I-14 | ¹H-NMR(400.0 MHz, d₆-DMSO): |
| | δ= 8.339(1.3);8.319(1.5);8.313(1.7);8.293(1.5);7.445(0.8);7.428(5.5);7.422(3.9);7.416(3.3);7.4 10(5.0);7.406(5.3);7.392(2.0);7.386(3.2);7.381(2.1);7.369(0.7);7.364(1.3);7.358(0.9);6.158(2.0 );6.152(2.6);6.132(2.3);6.126(2.9);6.115(2.3);6.109(3.1);6.088(2.3);6.082(3.0);5.753(3.3);5.41 1(3.4);5.408(3.6);5.404(2.9);5.402(3.0);5.368(3.0);5.365(3.2);5.361(2.5);5.358(2.7);5.316(0.7); 5.304(2.7);5.301(2.8);5.298(3.4);5.296(3.2);5.289(0.7);5.277(2.6);5.275(2.6);5.271(3.2);5.269( 3.0);4.346(1.2);4.326(1.1);4.313(1.0);4.306(1.0);4.292(0.8);4.171(1.7);4.158(2.5);4.149(2.3);4. 146(2.0);4.136(2.1);4.125(1.4);3.893(15.9);3.881(16.0);3.864(9.4);3.836(7.4);3.820(5.8);3.814 (4.7);3.800(3.8);3.793(2.9);3.778(2.3);3.666(0.6);3.646(0.5);3.633(2.0);3.627(1.8);3.622(1.8);3 .614(1.9);3.606(1.4);3.601(1.5);3.594(1.2);3.574(1.0);3.558(5.2);3.529(0.7);3.514(4.0);2.674(1 .9);2.670(2.6);2.665(1.8);2.661 (0.9);2.523(11.0);2.519(15.3);2.510(144.2);2.505(295.0);2.501( 402.4);2.496(275.2);2.492(123.6);2.387(1.0);2.366(0.9);2.354(0.9);2.347(0.9);2.337(1.8);2.332 (2.7);2.328(3.2);2.323(2.0);2.314(0.8);2.308(0.9);2.288(0.5);1.827(0.6);1.813(1.5);1.800(1.8);1 .780(1.4);1.767(1.5);1.755(0.6);1.236(1.0);0.008(3.1);0.000(95.5);-0.008(2.9) |
| I-15 | ¹H-NMR(400.0 MHz, CDCl3): |
| | δ= 7.263(36.9);7.174(0.5);7.170(1.1);7.162(1.5);7.156(1.4);7.153(1.3);7.150(1.3);7.144(1.3);7. 142(1.3);7.137(1.0);6.921(0.7);6.915(0.8);6.912(0.7);6.906(0.8);6.900(0.8);6.894(0.8);6.890(0. 7);6.884(0.9);6.878(0.6);6.182(0.8);6.155(0.9);6.138(1.0);6.128(0.7);6.112(1.0);6.102(0.7);6.0 85(0.8);6.058(0.8);5.529(1.2);5.528(1.3);5.517(1.5);5.486(1.1);5.485(1.1);5.474(1.2);5.370(1.3 );5.344(1.8);5.319(1.1);4.456(0.7);4.443(1.1);4.432(1.0);4.429(1.1);4.419(1.0);4.405(0.7);4.14 8(0.6);4.131(1.9);4.113(1.9);4.095(0.6);4.020(0.7);4.007(0.7);3.996(0.9);3.983(1.1);3.972(0.6); 3.961(0.8);3.948(0.8);3.937(1.3);3.894(1.6);3.882(1.9);3.839(1.6);3.834(0.6);3.327(1.4);3.314( 1.3);3.284(1.2);3.271(1.1);2.668(0.5);2.654(15.4);2.645(1.2);2.634(16.0);2.621(0.5);2.610(0.7) ;2.548(1.0);2.532(0.5);2.044(8.4);1.603(1.0);1.277(2.4);1.259(5.6);1.241(2.3);0.008(0.5);0.000 (13.7) |
| I-16 | ¹H-NMR(400.0 MHz, CDCl3): |
| | δ= 8.922(0.9);7.518(2.7);7.295(0.5);7.260(481.5);7.208(1.0);7.181(1.5);7.176(1.8);7.162(2.2); 7.144(1.7);7.138(1.4);6.996(2.6);6.919(0.9);6.914(0.7);6.903(0.8);6.897(1.6);6.892(1.5);6.876( 0.8);6.870(0.7);6.800(1.0);6.170(1.2);6.143(2.4);6.127(1.3);6.116(1.3);6.100(2.9);6.073(1.6);5. 540(2.0);5.527(2.2);5.497(1.7);5.483(2.0);5.379(3.7);5.352(3.5);4.541(1.0);4.529(1.2);4.518(1. 6);4.508(1.2);4.496(0.9);4.434(0.7);4.092(0.9);4.079(0.9);4.067(1.9);4.054(1.7);4.042(1.4);4.0 29(1.2);3.978(0.9);3.969(0.8);3.954(0.6);3.931(0.8);3.925(0.9);3.916(2.2);3.899(2.5);3.873(2.4 );3.856(2.3);3.492(1.7);3.376(16.0);3.350(14.7);3.331(2.2);3.328(2.2);3.288(2.0);3.284(1.9);2. 699(0.6);2.682(0.7);2.661(0.7);2.641(0.8);2.621(0.6);2.314(0.7);2.261(0.6);2.225(0.5);1.548(1 3.3);1.256(1.3);1.242(0.6);0.008(5.3);0.000(176.6);-0.008(5.2);-0.150(0.6) |
| I-17 | ¹H-NMR(400.6 MHz, CDCl3): |
| | δ= 8.303(0.5);8.282(0.9);8.261(0.5);7.271(4.1);7.205(1.4);7.199(1.7);7.188(1.2);7.185(1.9);7.1 79(2.8);7.174(2.0);7.162(1.2);7.160(1.8);7.154(1.4);6.888(0.7);6.883(1.2);6.877(0.6);6.867(1.4 );6.861(2.3);6.855(1.2);6.845(0.7);6.839(1.2);6.833(0.6);6.213(1.2);6.186(1.4);6.180(1.2);6.17 0(1.4);6.153(1.3);6.143(1.5);6.137(1.4);6.110(1.4);5.594(1.9);5.593(2.0);5.551(3.6);5.510(1.6); 5.508(1.6);5.342(2.0);5.341(2.0);5.323(1.8);5.322(1.8);5.316(1.8);5.314(1.8);5.297(1.6);5.296( 1.6);4.850(1.7);4.843(1.6);4.829(2.0);4.822(1.7);4.586(0.7);4.581(0.7);4.573(0.8);4.568(1.1);4. 562(0.9);4.555(0.8);4.549(0.7);4.01 0(1.0);3.998(1.1);3.988(2.2);3.975(2.2);3.965(1.4);3.952(1. 4);3.941(2.1);3.898(2.4);3.883(2.2);3.845(1.1);3.840(3.4);3.822(0.7);3.818(0.7);3.793(1.0);3.7 88(0.9);3.770(0.8);3.765(0.7);3.304(4.4);3.261(3.8);3.137(14.7);3.133(16.0);3.027(0.5);3.002( 14.1);2.992(13.2);2.357(0.8);2.352(1.3);2.331(0.8);2.322(0.8);2.314(1.8);2.311(1.7);2.308(1.5) ;2.293(1.1);2.291(1.2);2.288(1.0);2.270(0.8);2.045(2.1);1.853(1.0);1.334(0.9);1.286(1.5);1.277 (1.0);1.256(4.5);1.242(0.9);0.880(0.8);0.000(3.1) |
| I-18 | ¹H-NMR(400.0 MHz, CDCl3): |
| | δ= 7.519(2.5);7.315(0.8);7.260(401.6);7.258(381.2);7.170(16.6);7.155(17.2);6.996(2.3);6.883( 6.9);6.863(9.3);6.845(7.2);6.719(4.5);6.693(5.1);6.180(3.8);6.153(4.1);6.135(7.3);6.108(7.9);6. 089(4.4);6.064(4.4);5.532(12.0);5.489(10.4);5.372(8.6);5.346(13.4);5.322(8.8);4.448(3.4);4.42 2(7.0);4.405(8.8);4.399(9.3);4.371(4.7);4.002(3.8);3.990(3.8);3.978(5.9);3.966(9.0);3.954(4.3); 3.942(5.4);3.927(9.7);3.879(16.0);3.859(4.3);3.835(9.4);3.812(5.4);3.790(4.0);3.322(9.3);3.30 7(8.8);3.278(7.9);3.264(7.7);2.786(2.8);2.776(4.0);2.768(4.6);2.760(4.5);2.752(4.6);2.744(4.8); 2.735(4.7);2.727(3.1);2.632(1.9);2.615(4.1);2.597(5.1);2.591(5.0);2.581(5.3);2.574(5.4);2.557( 5.0);2.538(2.4);2.196(3.5);2.161(6.4);2.125(3.3);1.548(100.8);1.333(1.1);1.283(1.5);1.256(4.6) ;0.879(1.6);0.843(3.0);0.827(9.9);0.808(13.3);0.787(8.2);0.617(11.2);0.601(4.7);0.591(6.6);0.5 81(5.7);0.563(5.8);0.000(138.7);-0.002(131.1);-0.152(0.8) |
| I-19 | ¹H-NMR(400.0 MHz, CDC13): |
| | δ= 8.214(1.2);8.106(1.4);7.518(2.9);7.293(1.2);7.260(529.3);7.227(1.7);7.221(2.0);7.208(2.6); 7.188(2.3);7.182(2.6);7.168(2.6);7.163(2.2);6.996(3.2);6.916(1.7);6.900(1.8);6.895(3.0);6.888( 1.6);6.873(1.5);6.171(1.7);6.158(1.5);6.145(2.0);6.128(2.2);6.115(1.8);6.101(1.9);6.088(1.8);5. 538(3.0);5.529(3.3);5.494(2.6);5.487(2.8);5.359(4.2);5.332(3.8);4.580(1.4);4.437(1.5);4.049(0. 9);4.037(0.9);4.025(1.9);4.013(2.0);3.997(2.1);3.984(2.1);3.968(2.5);3.928(3.1);3.924(3.3);3.8 85(3.0);3.324(2.9);3.295(2.8);3.281(2.6);3.252(2.6);2.553(0.7);2.447(1.1);2.424(0.9);2.410(0.8 );2.364(1.1);2.044(1.5);1.541(30.8);1.507(52.0);1.500(32.6);1.464(1.0);1.266(9.4);0.899(4.9);0 .882(16.0);0.864(6.2);0.146(1.0);0.008(6.7);0.000(193.0);-0.008(6.3) |
| I-20 | ¹H-NMR(400.0 MHz, CDCl3): |
| | δ= 7.5193 (0.6); 7.2604 (100.9); 7.1884 (0.7); 7.1762 (1.8); 7.1698 (2.6); 7.1635 (2.2); 7.1601 (2.0); 7.1565 (2.2); 7.1503 (2.4); 7.1438 (1.6); 7.0179 (1.0); 6.9965 (1.4); 6.9101 (0.9); 6.9045 (0.9); 6.8940 (0.8); 6.8884 (1.9); 6.8829 (1.8); 6.8773 (0.7); 6.8667 (0.9); 6.8611 (0.9); 6.1799 (1.2); 6.1531 (1.3); 6.1367 (1.5); 6.1321 (1.2); 6.1100 (1.5); 6.1053 (1.4); 6.0889 (1.4); 6.0621 (1.5); 5.5268 (2.0); 5.5255 (2.0); 5.5113 (2.3); 5.4837 (1.8); 5.4824 (1.7); 5.4680 (1.9); 5.3594 (2.1); 5.3440 (1.9); 5.3326 (2.0); 5.3173 (1.8); 4.4620 (0.9); 4.4432 (1.6); 4.4327 (1.3); 4.4199 (1.7); 4.4091 (1.8); 4.3969 (1.0); 4.3858 (0.8); 4.0356 (0.9); 4.0228 (0.9); 4.0117 (1.4); 4.0034 (1.1); 3.9990 (1.3); 3.9909 (0.9); 3.9794 (1.4); 3.9669 (1.2); 3.9270 (2.1); 3.9098 (0.9); 3.9031 (0.9); 3.8840 (3.2); 3.8778 (3.5); 3.8522 (0.6); 3.8478 (0.6); 3.8348 (2.5); 3.7243 (16.0); 3.7111 (15.6); 3.6112 (1.2); 3.6027 (0.5); 3.5953 (1.3); 3.5871 (1.4); 3.5716 (1.8); 3.5609 (1.1); 3.5562 (1.3); 3.5463 (1.0); 3.5385 (0.8); 3.3163 (2.2); 3.3043 (2.2); 3.2733 (1.9); 3.2613 (1.9); 2.6165 (2.4); 2.6103 (1.4); 2.6011 (4.7); 2.5938 (2.6); 2.5860 (2.3); 2.5787 (1.4); 2.5639 (0.7); 2.5592 (0.9); 2.5536 (0.7); 2.5418 (0.8); 2.5363 (0.7); 2.2238 (0.6); 2.1888 (0.5); 2.1623 (0.6); 2.1280 (0.5); 1.5572 (6.4); 1.2563 (1.1); 0.0079 (1.2); -0.0002 (33.7); -0.0085 (1.1) |
| I-21 | ¹H-NMR(400.0 MHz, CDCl3): |
| | δ= 7.2608 (68.6); 7.1807 (0.5); 7.1751 (0.8); 7.1685 (2.1); 7.1626 (3.6); 7.1571 (3.1); 7.1529 (2.8); 7.1484 (3.0); 7.1428 (4.0); 7.1371 (2.4); 7.1245 (0.8); 7.0700 (0.6); 7.0533 (0.6); 7.0156 (0.6); 6.9967 (0.9); 6.9102 (0.9); 6.9049 (0.9); 6.8938 (0.8); 6.8885 (1.8); 6.8832 (1.7); 6.8777 (0.7); 6.8668 (0.9); 6.8616 (0.9); 6.1800 (1.2); 6.1532 (1.3); 6.1368 (1.5); 6.1325 (1.3); 6.1100 (1.6); 6.1057 (1.4); 6.0893 (1.4); 6.0625 (1.5); 5.5114 (2.2); 5.5018 (2.4); 5.4690 (1.9); 5.4588 (2.1); 5.3527 (2.3); 5.3259 (4.2); 5.2990 (1.9); 4.5138 (0.9); 4.5037 (1.0); 4.4891 (1.5); 4.4783 (1.9); 4.4710 (0.7); 4.4646 (1.7); 4.4607 (1.2); 4.4541 (2.0); 4.4479 (1.2); 4.4420 (1.0); 4.4367 (0.8); 4.4307 (0.6); 4.1635 (0.8); 4.1487 (0.8); 4.1380 (1.8); 4.1242 (1.8); 4.1176 (1.6); 4.1092 (1.7); 4.1027 (1.6); 4.0953 (1.7); 4.0798 (1.0); 4.0669 (1.0); 4.0557 (1.5); 4.0497 (0.6); 4.0428 (1.4); 4.0174 (1.8); 4.0044 (1.6); 3.9944 (1.6); 3.9823 (1.6); 3.9700 (1.6); 3.9580 (0.8); 3.9502 (0.7); 3.9409 (2.5); 3.9288 (1.0); 3.9250 (1.0); 3.8979 (2.9); 3.8705 (2.2); 3.8275 (2.6); 3.8001 (16.0); 3.7868 (15.7); 3.3070 (2.4); 3.2866 (2.3); 3.2639 (2.0); 3.2436 (2.0); 2.6322 (0.5); 2.6156 (0.5); 2.6087 (0.5); 2.5978 (0.7); 2.5917 (0.6); 2.5808 (0.6); 2.5739 (0.7); 2.5569 (0.7); 2.5539 (0.6); 2.5472 (0.5); 2.5360 (0.7); 2.5305 (0.6); 2.5192 (0.6); 2.5127 (0.6); 2.4959 (0.6); 2.2739 (1.1); 2.2666 (1.2); 2.2392 (0.9); 2.2321 (0.9); 1.5678 (1.1); 1.2583 (0.9); 0.0079 (0.9); -0.0002 (22.3); -0.0085 (0.6) |
| I-22 | ¹H-NMR(400.0 MHz, CDCl3): |
| | δ= 7.5193 (0.6); 7.2605 (104.8); 7.2594 (101.9); 7.2228 (2.2); 7.2086 (1.5); 7.1842 (2.0); 7.1701 (4.3); 7.1659 (4.7); 7.1520 (7.7); 7.1384 (4.6); 6.9958 (0.6); 6.9514 (1.3); 6.9334 (1.4); 6.8996 (1.4); 6.8922 (1.8); 6.8861 (2.4); 6.8822 (2.4); 6.8777 (2.4); 6.8717 (1.8); 6.8653 (1.4); 6.1851 (1.6); 6.1589 (1.7); 6.1424 (1.8); 6.1300 (1.6); 6.1156 (2.0); 6.1034 (1.8); 6.0871 (1.9); 6.0601 (2.0); 5.5101 (4.2); 5.5046 (4.2); 5.4669 (3.6); 5.4615 (3.8); 5.3541 (3.6); 5.3266 (6.0); 5.2986 (3.5); 4.4793 (2.3); 4.4734 (2.1); 4.4671 (2.0); 4.4120 (1.8); 4.4009 (2.5); 4.3947 (2.5); 4.3886 (3.0); 4.3776 (3.1); 4.3711 (3.7); 4.3527 (2.3); 4.3353 (1.9); 4.3187 (0.9); 4.2141 (0.9); 4.2043 (1.2); 4.1967 (2.6); 4.1858 (4.2); 4.1790 (3.2); 4.1680 (7.4); 4.1502 (6.0); 4.1324 (2.0); 4.0309 (1.2); 4.0173 (1.3); 4.0072 (3.8); 3.9945 (3.7); 3.9842 (2.3); 3.9713 (2.1); 3.9575 (2.4); 3.9521 (2.4); 3.9337 (1.4); 3.9209 (3.4); 3.8839 (3.5); 3.8778 (4.1); 3.8415 (5.4); 3.8249 (1.5); 3.8186 (1.5); 3.3108 (5.7); 3.2676 (4.9); 2.6780 (1.2); 2.6649 (1.3); 2.6401 (2.4); 2.6273 (2.3); 2.6194 (1.2); 2.6139 (1.3); 2.6029 (1.3); 2.5955 (1.3); 2.5845 (2.0); 2.5736 (1.5); 2.5553 (7.3); 2.5416 (6.4); 2.5297 (3.3); 2.5150 (2.5); 2.4914 (1.5); 2.4772 (1.4); 2.1670 (2.1); 2.1415 (1.9); 2.1330 (2.0); 1.9583 (0.9); 1.5574 (5.4); 1.3281 (9.5); 1.3104 (14.3); 1.2918 (12.0); 1.2850 (8.0); 1.2729 (7.6); 1.2671 (12.9); 1.2493 (16.0); 1.2326 (10.6); -0.0002 (38.6); -0.0015 (37.8) |
| I-23 | ¹H-NMR(400.0 MHz, CDCl3): |
| | δ= 8.9621 (1.0); 7.5207 (2.6); 7.2955 (0.6); 7.2862 (0.8); 7.2619 (459.9); 7.1725 (1.8); 7.1668 (2.2); 7.1527 (2.6); 7.1471 (2.1); 7.1352 (0.7); 6.9978 (2.5); 6.9156 (0.8); 6.8995 (0.8); 6.8939 (1.5); 6.8721 (1.1); 6.8525 (0.7); 4.5515 (1.0); 4.5405 (1.0); 4.5285 (1.1); 4.5168 (1.1); 4.4210 (0.8); 4.4138 (0.8); 4.0623 (1.0); 4.0489 (0.9); 4.0378 (1.3); 4.0249 (1.3); 3.9220 (0.9); 3.9143 (0.9); 3.8967 (0.7); 3.8904 (0.7); 3.7760 (2.6); 3.7591 (0.5); 3.7327 (3.0); 3.7157 (0.7); 3.3935 (13.3); 3.3551 (3.1); 3.2023 (3.1); 3.1589 (2.7); 2.6827 (0.6); 2.6663 (0.6); 2.6590 (0.6); 2.6481 (0.7); 2.6429 (0.7); 2.6315 (0.6); 2.6247 (0.7); 2.6082 (0.6); 2.3659 (0.8); 2.3307 (0.6); 1.7042 (4.1); 1.6963 (16.0); 1.5537 (39.0); 1.3330 (1.6); 1.2844 (2.4); 1.2541 (6.3); 0.8799 (0.9); 0.1458 (0.6); 0.0080 (5.5); -0.0002 (199.1); -0.0085 (6.8); -0.1498 (0.6) |
| I-24 | ¹H-NMR(400.6 MHz, CDCl3): |
| | δ= 9.5455 (1.6); 9.4925 (1.5); 7.2954 (2.3); 7.2765 (0.9); 7.2510 (0.7); 7.1743 (0.5); 7.1618 (2.9); 7.1564 (3.8); 7.1420 (3.8); 7.1367 (3.0); 7.1302 (0.6); 7.1243 (0.6); 6.9029 (0.9); 6.8969 (0.9); 6.8872 (0.8); 6.8812 (1.8); 6.8753 (1.8); 6.8693 (0.8); 6.8658 (0.6); 6.8595 (1.0); 6.8536 (0.9); 4.5375 (0.6); 4.5267 (0.8); 4.5138 (0.9); 4.5039 (1.4); 4.4931 (1.3); 4.4819 (1.5); 4.4705 (1.5); 4.0333 (0.7); 4.0204 (0.8); 4.0097 (1.1); 3.9968 (1.0); 3.9845 (0.7); 3.9717 (0.7); 3.9606 (1.1); 3.9479 (1.0); 3.9325 (1.0); 3.9258 (1.0); 3.9090 (0.6); 3.9022 (0.6); 3.8828 (1.0); 3.8776 (1.0); 3.8589 (0.8); 3.8523 (0.9); 3.8420 (11.4); 3.8184 (1.8); 3.7751 (2.1); 3.7668 (2.8); 3.7608 (16.0); 3.7237 (2.2); 3.2105 (2.2); 3.1989 (2.6); 3.1671 (1.9); 3.1556 (2.3); 2.6245 (0.5); 2.6141 (0.6); 2.6080 (0.6); 2.5978 (0.8); 2.5900 (0.7); 2.5732 (0.6); 2.3252 (0.6); 2.2563 (0.6); 2.0502 (0.9); 1.6956 (15.4); 1.6893 (15.1); 1.2612 (1.4); 1.2572 (1.9); -0.0002 (0.8) |
| I-25 | ¹H-NMR(400.6 MHz, CDCl3): |
| | δ= 7.2724 (4.6); 7.2001 (1.3); 7.1944 (1.6); 7.1912 (0.9); 7.1838 (1.1); 7.1800 (1.7); 7.1742 (1.6); 7.1720 (1.7); 7.1662 (1.8); 7.1629 (1.2); 7.1550 (1.0); 7.1518 (1.7); 7.1462 (1.4); 6.8826 (0.7); 6.8768 (1.2); 6.8710 (0.6); 6.8609 (1.3); 6.8551 (2.3); 6.8492 (1.2); 6.8391 (0.7); 6.8333 (1.2); 6.8275 (0.6); 4.8488 (1.8); 4.8418 (1.6); 4.8284 (1.8); 4.8211 (1.8); 4.5763 (0.5); 4.5692 (0.5); 4.5628 (0.7); 4.5566 (0.7); 4.5500 (0.6); 4.5439 (0.7); 4.1485 (0.6); 4.1307 (1.8); 4.1128 (1.8); 4.0950 (0.6); 4.0199 (1.0); 4.0074 (1.1); 3.9970 (1.4); 3.9845 (1.3); 3.9751 (1.1); 3.9623 (1.1); 3.9520 (1.4); 3.9393 (1.3); 3.8636 (0.9); 3.8584 (0.8); 3.8407 (0.7); 3.8355 (0.6); 3.8049 (2.2); 3.7621 (3.1); 3.7472 (2.4); 3.7427 (0.8); 3.7368 (0.7); 3.7044 (2.6); 3.1654 (2.6); 3.1615 (2.7); 3.1415 (14.8); 3.1346 (16.0); 3.1225 (2.4); 3.1187 (2.4); 2.9966 (15.0); 2.9905 (14.2); 2.3855 (0.6); 2.3809 (0.9); 2.3765 (0.6); 2.3427 (0.8); 2.3247 (0.8); 2.3220 (0.8); 2.3091 (1.1); 2.3018 (2.0); 2.2964 (0.7); 2.2912 (0.6); 2.2878 (0.6); 2.2849 (1.0); 2.2822 (1.0); 2.2649 (0.8); 2.1269 (0.7); 2.1252 (0.7); 2.0455 (8.6); 1.7186 (13.2); 1.6887 (12.7); 1.4304 (0.6); 1.4163 (0.6); 1.3345 (0.5); 1.2856 (0.8); 1.2776 (2.7); 1.2598 (6.6); 1.2420 (2.5); 0.8802 (0.6); -0.0002 (3.0) |
| I-26 | ¹H-NMR(400.6 MHz, CDCl3): |
| | δ= 7.3193 (0.9); 7.1666 (1.1); 7.1605 (1.9); 7.1572 (1.5); 7.1547 (1.6); 7.1511 (1.3); 7.1466 (1.4); 7.1441 (1.4); 7.1407 (2.0); 7.1349 (1.2); 7.0893 (0.6); 6.9034 (0.6); 6.8957 (0.5); 6.8896 (0.8); 6.8818 (1.0); 6.8757 (0.6); 6.8600 (0.5); 4.4759 (0.9); 4.4644 (1.0); 4.4523 (0.9); 4.4434 (1.0); 4.4413 (1.0); 4.4307 (0.8); 4.4206 (0.7); 4.4075 (0.6); 4.1305 (1.3); 4.1127 (1.3); 4.0416 (0.6); 4.0285 (0.7); 4.0180 (1.0); 4.0049 (0.8); 3.9932 (0.6); 3.9804 (0.6); 3.9693 (0.8); 3.9566 (0.8); 3.9046 (0.5); 3.8976 (0.5); 3.8144 (1.5); 3.7711 (1.4); 3.7617 (1.4); 3.7184 (1.6); 3.2169 (1.5); 3.2000 (1.3); 3.1735 (1.5); 3.1567 (1.1); 2.6648 (14.5); 2.6486 (0.6); 2.6363 (16.0); 2.6143 (0.6); 2.6108 (0.5); 2.6081 (0.6); 2.5741 (1.7); 2.5497 (0.6); 2.1183 (0.5); 2.0718 (0.6); 2.0470 (6.1); 1.6956 (13.2); 1.2782 (1.8); 1.2604 (4.0); 1.2425 (1.7); -0.0002 (0.5) |
| I-27 | ¹H-NMR(400.6 MHz, CDCl3): |
| | δ= 8.8097 (1.6); 8.8075 (1.6); 8.7461 (1.5); 8.7438 (1.5); 8.0732 (0.8); 8.0589 (0.9); 7.9541 (1.8); 7.9503 (1.9); 7.9374 (1.6); 7.9336 (1.6); 7.2999 (1.3); 7.1780 (0.9); 7.1718 (0.9); 7.1604 (1.0); 7.1145 (1.4); 7.1089 (1.8); 7.1059 (1.1); 7.0979 (1.0); 7.0947 (1.8); 7.0891 (1.5); 6.9942 (1.2); 6.9886 (1.6); 6.9855 (1.0); 6.9777 (0.9); 6.9745 (1.6); 6.9689 (1.3); 6.8962 (0.6); 6.8803 (0.7); 6.8745 (1.2); 6.8687 (0.6); 6.8588 (0.5); 6.8531 (0.9); 6.8387 (0.7); 6.8329 (1.1); 6.8271 (0.5); 6.8112 (0.6); 4.8504 (0.7); 4.8364 (0.8); 4.7851 (0.7); 4.7715 (0.7); 4.7592 (0.7); 4.7478 (0.7); 4.6661 (0.7); 4.6553 (0.8); 4.6033 (0.8); 4.5945 (0.9); 4.5794 (0.8); 4.5709 (0.8); 4.5595 (0.8); 4.5498 (0.9); 4.5359 (0.9); 4.5262 (0.9); 4.5050 (0.6); 4.4993 (0.5); 4.4942 (0.6); 4.4877 (0.6); 4.4788 (0.6); 4.4734 (0.6); 4.4684 (0.5); 4.4621 (0.5); 4.1468 (1.2); 4.1290 (3.6); 4.1112 (3.6); 4.1028 (2.0); 4.0917 (3.2); 4.0848 (1.5); 4.0567 (1.7); 4.0454 (1.8); 4.0364 (1.2); 3.7225 (2.0); 3.7118 (2.3); 3.6793 (2.3); 3.6686 (2.6); 3.1364 (2.4); 3.0967 (2.3); 3.0932 (2.4); 3.0535 (1.9); 2.6157 (0.6); 2.5998 (0.9); 2.5950 (0.6); 2.5921 (0.6); 2.5840 (0.7); 2.5812 (0.8); 2.5760 (0.9); 2.5644 (0.7); 2.5605 (0.7); 2.5577 (0.7); 2.5409 (0.5); 2.4130 (0.7); 2.3006 (0.6); 2.2661 (0.5); 2.1363 (0.9); 2.1075 (0.6); 2.0440 (16.0); 1.6123 (11.4); 1.5944 (12.6); 1.2769 (4.5); 1.2591 (9.7); 1.2412 (4.3); -0.0002 (0.7) |
| I-28 | ¹H-NMR(400.0 MHz, CDCl3): |
| | δ= 7.5186 (1.7); 7.3091 (1.1); 7.2917 (0.6); 7.2870 (0.5); 7.2719 (1.4); 7.2597 (290.5); 7.2108 (0.8); 7.1834 (0.6); 7.1774 (0.7); 7.1708 (1.8); 7.1652 (3.4); 7.1598 (3.0); 7.1553 (2.0); 7.1508 (2.6); 7.1454 (3.3); 7.1398 (1.8); 7.1331 (0.7); 7.1273 (0.5); 6.9957 (1.8); 6.9048 (1.1); 6.8859 (2.2); 6.8830 (2.1); 6.8772 (1.4); 6.8701 (1.3); 6.8640 (1.6); 6.8614 (1.5); 4.5156 (1.1); 4.5020 (1.6); 4.4927 (1.3); 4.4862 (1.3); 4.4788 (1.8); 4.4635 (1.2); 4.4239 (1.0); 4.4149 (1.0); 4.1207 (1.0); 4.1064 (1.0); 4.0966 (1.4); 4.0825 (1.2); 4.0733 (1.1); 4.0596 (1.0); 4.0492 (1.4); 4.0354 (1.2); 3.9396 (0.9); 3.9295 (0.9); 3.9153 (0.8); 3.9057 (0.8); 3.8876 (0.9); 3.8807 (0.9); 3.8646 (0.8); 3.8554 (0.8); 3.7763 (2.4); 3.7593 (2.3); 3.7331 (2.8); 3.7161 (2.7); 3.5188 (0.6); 3.5007 (1.3); 3.4897 (1.2); 3.4825 (1.8); 3.4793 (2.0); 3.4720 (1.6); 3.4653 (2.7); 3.4616 (2.4); 3.4506 (1.3); 3.4472 (1.3); 3.4431 (1.8); 3.4260 (1.0); 3.1963 (2.8); 3.1936 (3.0); 3.1531 (2.5); 3.1504 (2.7); 2.7050 (0.5); 2.6998 (0.6); 2.6868 (0.6); 2.6828 (1.0); 2.6767 (0.6); 2.6708 (0.7); 2.6649 (1.2); 2.6597 (0.7); 2.6482 (1.2); 2.6424 (0.7); 2.6301 (0.6); 2.6253 (0.7); 2.3054 (0.8); 2.2925 (0.7); 2.2837 (1.0); 2.2709 (0.7); 2.2604 (0.9); 2.2466 (1.5); 2.1944 (2.8); 2.1809 (0.7); 2.1470 (0.6); 2.1326 (0.6); 1.9610 (0.6); 1.9506 (0.8); 1.9401 (1.1); 1.9321 (1.6); 1.9207 (1.4); 1.9135 (1.9); 1.8926 (1.4); 1.8741 (0.9); 1.7018 (14.9); 1.6984 (16.0); 1.5592 (1.1); 1.5035 (0.9); 1.3329 (0.6); 1.2843 (1.0); 1.2562 (3.6); 1.2133 (0.7); 1.1942 (0.5); 1.1206 (4.0); 1.1144 (4.0); 1.1020 (8.3); 1.0958 (8.0); 1.0833 (3.8); 1.0771 (3.7); 0.9470 (0.6); 0.9284 (1.2); 0.9101 (0.6); 0.8802 (0.9); 0.0079 (3.7); -0.0002 (132.2); -0.0085 (4.6) |
| I-29 | ¹H-NMR(400.0 MHz, CDCl3): |
| | δ= 7.5188 (1.6); 7.3096 (0.8); 7.2599 (281.0); 7.2101 (0.8); 7.1813 (2.0); 7.1731 (3.2); 7.1672 (3.1); 7.1619 (3.1); 7.1562 (3.3); 7.1480 (2.1); 6.9959 (2.0); 6.9141 (1.1); 6.9086 (1.2); 6.8981 (1.1); 6.8926 (2.2); 6.8871 (2.1); 6.8817 (1.0); 6.8767 (0.8); 6.8708 (1.4); 6.8655 (1.4); 6.8596 (1.1); 6.8305 (1.3); 6.1736 (1.3); 6.1472 (2.0); 6.1305 (1.6); 6.1218 (1.4); 6.1041 (2.2); 6.0786 (1.5); 5.5442 (2.3); 5.5356 (2.9); 5.5011 (2.1); 5.4925 (2.5); 5.3800 (2.5); 5.3728 (2.3); 5.3534 (2.4); 5.3460 (2.2); 4.5082 (1.1); 4.5019 (1.1); 4.4943 (1.5); 4.4860 (1.9); 4.4792 (1.5); 4.4713 (1.6); 4.4651 (1.8); 4.4517 (1.0); 4.4394 (0.9); 4.1486 (1.1); 4.1307 (3.4); 4.1128 (3.5); 4.1025 (1.0); 4.0950 (1.4); 4.0888 (1.0); 4.0783 (2.2); 4.0644 (2.0); 4.0539 (1.4); 4.0398 (1.2); 3.9349 (1.1); 3.9253 (1.2); 3.9181 (2.6); 3.8997 (2.9); 3.8869 (1.2); 3.8750 (2.9); 3.8567 (2.5); 3.7302 (0.6); 3.7180 (0.6); 3.5469 (1.2); 3.5381 (0.9); 3.5350 (1.3); 3.5237 (1.0); 3.4990 (1.2); 3.4930 (1.0); 3.4825 (2.4); 3.4761 (1.9); 3.4659 (1.9); 3.4586 (1.9); 3.4538 (2.0); 3.4417 (1.4); 3.4372 (1.9); 3.4205 (1.0); 3.4019 (0.5); 3.3281 (4.2); 3.2850 (3.8); 2.7233 (0.5); 2.7124 (0.5); 2.7060 (0.6); 2.7005 (0.6); 2.6890 (1.2); 2.6832 (0.7); 2.6775 (0.8); 2.6713 (1.2); 2.6659 (0.8); 2.6599 (0.8); 2.6481 (0.6); 2.6373 (0.6); 2.2910 (0.7); 2.2840 (1.3); 2.2606 (1.1); 2.2466 (3.0); 2.2271 (1.2); 2.2184 (0.8); 2.2036 (1.3); 2.1944 (5.5); 2.1741 (0.5); 2.1671 (0.7); 2.1305 (0.9); 2.1011 (0.7); 2.0438 (16.0); 1.9565 (0.8); 1.9502 (0.8); 1.9385 (1.6); 1.9315 (1.6); 1.9147 (2.0); 1.8986 (1.7); 1.8926 (1.6); 1.8805 (1.0); 1.8748 (1.0); 1.8567 (0.5); 1.6134 (0.9); 1.5964 (1.2); 1.5798 (1.3); 1.5602 (1.2); 1.5436 (0.9); 1.5039 (0.8); 1.4094 (0.6); 1.3908 (0.8); 1.3709 (0.8); 1.3531 (0.6); 1.3331 (1.0); 1.2844 (1.6); 1.2766 (5.4); 1.2587 (13.8); 1.2409 (5.0); 1.2324 (1.0); 1.2133 (1.4); 1.1943 (1.1); 1.1231 (3.8); 1.1172 (3.8); 1.1045 (7.7); 1.0986 (7.3); 1.0858 (3.8); 1.0800 (3.6); 0.9468 (1.6); 0.9284 (3.1); 0.9100 (1.4); 0.8968 (0.6); 0.8801 (1.5); 0.8625 (0.7); 0.0080 (3.4); -0.0002 (116.8); -0.0085 (3.9) |
| I-30 | ¹H-NMR(400.0 MHz, CDCl3): |
| | δ= 7.5185 (2.5); 7.3218 (0.5); 7.3181 (0.6); 7.3094 (0.9); 7.3021 (0.5); 7.2957 (0.6); 7.2941 (0.5); 7.2902 (0.7); 7.2886 (0.7); 7.2862 (0.8); 7.2854 (0.8); 7.2822 (1.0); 7.2790 (1.1); 7.2782 (1.1); 7.2759 (1.4); 7.2750 (1.5); 7.2743 (1.6); 7.2734 (1.7); 7.2727 (1.8); 7.2711 (2.2); 7.2703 (2.3); 7.2694 (2.4); 7.2678 (3.3); 7.2670 (3.6); 7.2596 (455.4); 7.2279 (0.7); 7.2103 (0.6); 7.1464 (2.3); 6.9956 (2.7); 6.8685 (1.6); 4.5296 (0.8); 4.1304 (0.8); 4.1125 (0.8); 3.9728 (0.7); 3.7768 (0.5); 3.7375 (0.6); 3.1965 (1.0); 3.1528 (0.8); 2.0436 (2.6); 1.6706 (6.3); 1.6102 (16.0); 1.2842 (0.9); 1.2763 (1.1); 1.2584 (3.4); 1.2406 (1.0); 0.8801 (0.6); 0.1460 (0.6); 0.0102 (1.0); 0.0079 (6.0); 0.0063 (2.0); 0.0054 (2.2); 0.0046 (2.6); -0.0002 (201.3); -0.0052 (3.5); -0.0060 (2.7); -0.0068 (2.2); -0.0085 (5.9); -0.0116 (0.6); -0.0124 (0.5); -0.1496 (0.6) |

In Analogie zu den oben angeführten und an entsprechender Stelle zitierten Herstellungsbeispielen und unter Berücksichtigung der allgemeinen Angaben zur Herstellung von substituierten Isoxazolincarboxamiden kann man die nachfolgend genannten Verbindungen erhalten:
Tabelle 2.1: Erfindungsgemäße Verbindungen 2.1-1 bis 2.1-390 der allgemeinen Formel (I.1), worin Z-(C=W)-N(R¹¹)-R¹² wie nachfolgend definiert ist.

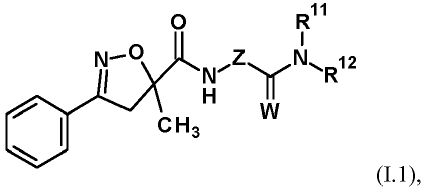

**Tabelle 2.1**

| **Nr.** | 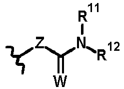 | **Nr.** | 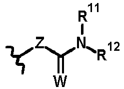 |
|---|---|---|---|
| 2.1-1 | 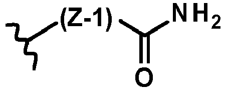 | 2.1-2 | 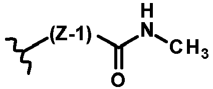 |
| 2.1-3 | 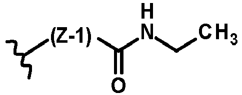 | 2.1-4 | 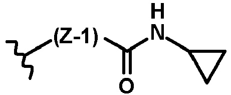 |
| 2.1-5 | 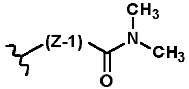 | 2.1-6 | 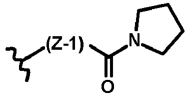 |
| 2.1-7 | 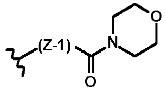 | 2.1-8 | 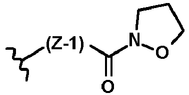 |
| 2.1-9 | 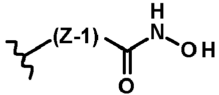 | 2.1-10 | 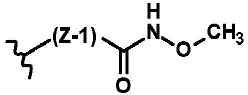 |
| 2.1-11 | 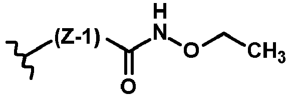 | 2.1-12 | 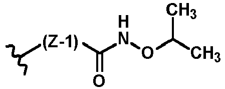 |
| 2.1-13 | 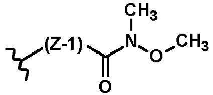 | 2.1-14 | 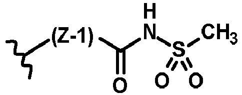 |
| 2.1-15 | 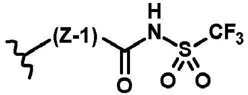 | 2.1-16 | 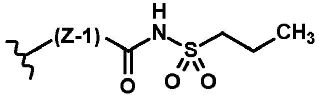 |
| 2.1-17 | 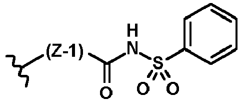 | 2.1-18 | 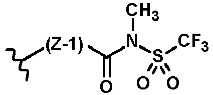 |
| 2.1-19 | 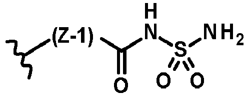 | 2.1-20 | 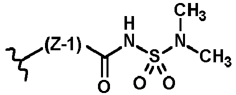 |
| 2.1-21 | 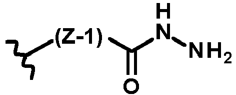 | 2.1-22 | 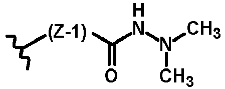 |
| 2.1-23 | 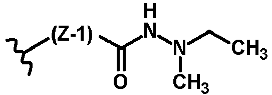 | 2.1-24 | 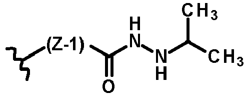 |
| 2.1-25 | 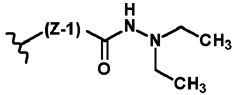 | 2.1-26 | 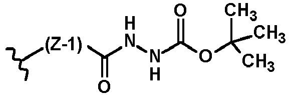 |
| 2.1-27 | 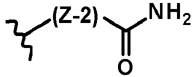 | 2.1-28 | 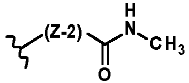 |
| 2.1-29 | 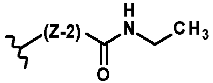 | 2.1-30 | 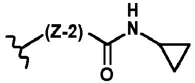 |
| 2.1-31 | 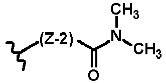 | 2.1-32 | 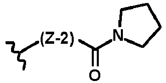 |
| 2.1-33 | 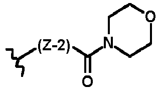 | 2.1-34 | 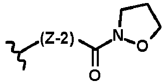 |
| 2.1-35 | 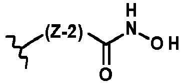 | 2.1-36 | 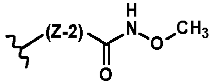 |
| 2.1-37 | 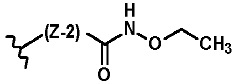 | 2.1-38 | 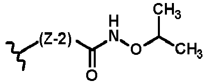 |
| 2.1-39 | 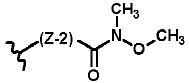 | 2.1-40 | 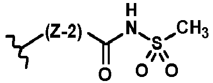 |
| 2.1-41 | 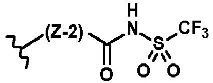 | 2.1-42 | 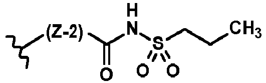 |
| 2.1-43 | 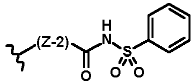 | 2.1-44 | 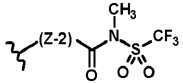 |
| 2.1-45 | 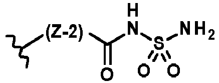 | 2.1-46 | 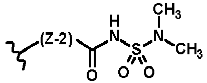 |
| 2.1-47 | 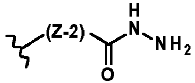 | 2.1-48 | 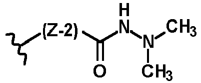 |
| 2.1-49 | 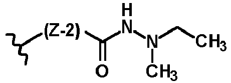 | 2.1-50 | 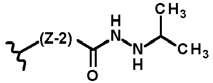 |
| 2.1-51 | 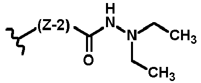 | 2.1-52 | 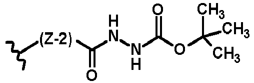 |
| 2.1-53 | 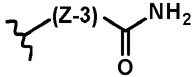 | 2.1-54 | 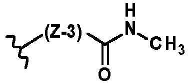 |
| 2.1-55 | 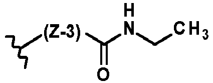 | 2.1-56 | 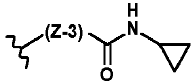 |
| 2.1-57 | 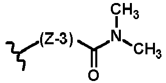 | 2.1-58 | 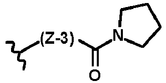 |
| 2.1-59 | 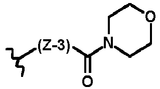 | 2.1-60 | 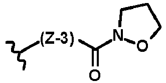 |
| 2.1-61 | 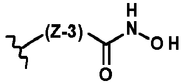 | 2.1-62 | 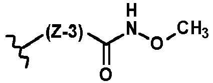 |
| 2.1-63 | 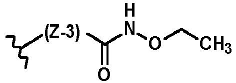 | 2.1-64 | 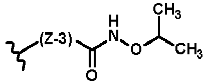 |
| 2.1-65 | 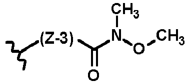 | 2.1-66 | 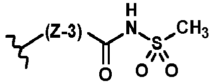 |
| 2.1-67 | 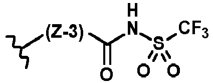 | 2.1-68 | 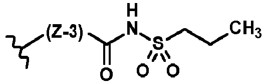 |
| 2.1-69 | 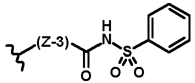 | 2.1-70 | 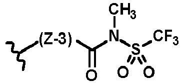 |
| 2.1-71 | 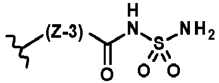 | 2.1-72 | 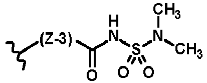 |
| 2.1-73 | 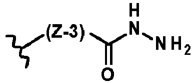 | 2.1-74 | 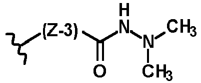 |
| 2.1-75 | 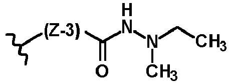 | 2.1-76 | 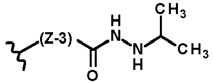 |
| 2.1-77 | 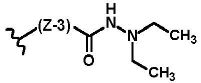 | 2.1-78 | 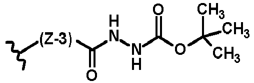 |
| 2.1-79 | 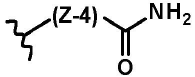 | 2.1-80 | 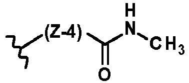 |
| 2.1-81 | 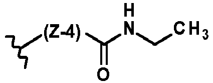 | 2.1-82 | 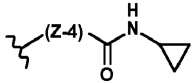 |
| 2.1-83 | 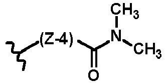 | 2.1-84 | 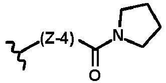 |
| 2.1-85 | 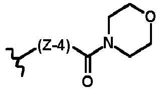 | 2.1-86 | 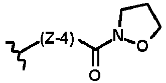 |
| 2.1-87 | 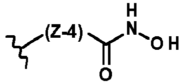 | 2.1-88 | 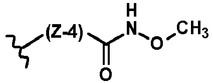 |
| 2.1-89 | 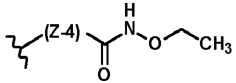 | 2.1-90 | 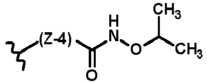 |
| 2.1-91 | 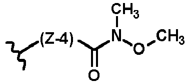 | 2.1-92 | 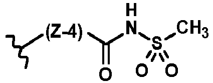 |
| 2.1-93 | 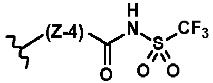 | 2.1-94 | 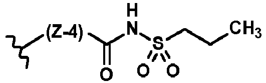 |
| 2.1-95 | 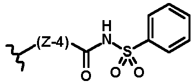 | 2.1-96 | 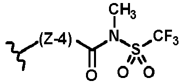 |
| 2.1-97 | 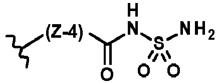 | 2.1-98 | 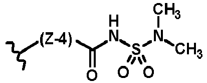 |
| 2.1-99 | 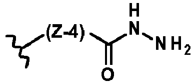 | 2.1-100 | 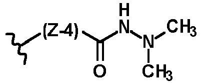 |
| 2.1-101 | 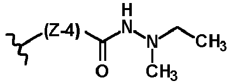 | 2.1-102 | 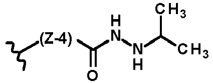 |
| 2.1-103 | 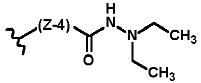 | 2.1-104 | 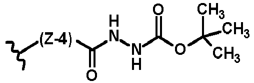 |
| 2.1-105 | 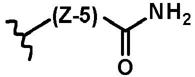 | 2.1-106 | 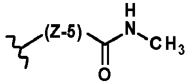 |
| 2.1-107 | 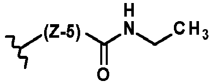 | 2.1-108 | 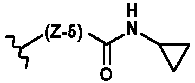 |
| 2.1-109 | 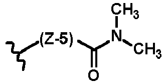 | 2.1-110 | 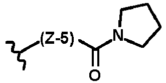 |
| 2.1-111 | 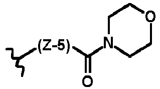 | 2.1-112 | 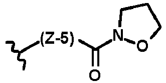 |
| 2.1-113 | 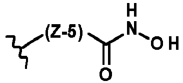 | 2.1-114 | 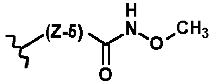 |
| 2.1-115 | 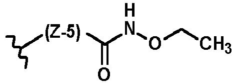 | 2.1-116 | 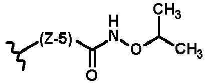 |
| 2.1-117 | 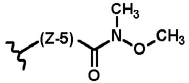 | 2.1-118 | 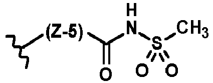 |
| 2.1-119 | 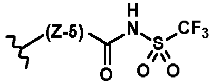 | 2.1-120 | 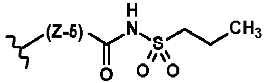 |
| 2.1-121 | 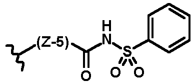 | 2.1-122 | 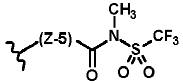 |
| 2.1-123 | 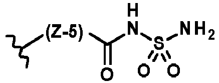 | 2.1-124 | 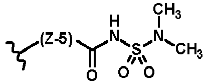 |
| 2.1-125 | 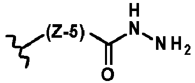 | 2.1-126 | 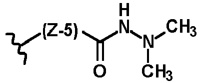 |
| 2.1-127 | 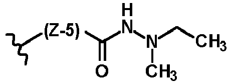 | 2.1-128 | 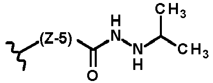 |
| 2.1-129 | 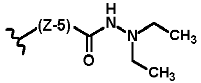 | 2.1-130 | 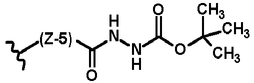 |
| 2.1-131 | 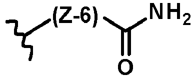 | 2.1-132 | 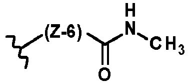 |
| 2.1-133 | 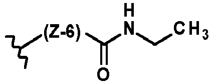 | 2.1-134 | 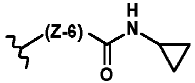 |
| 2.1-135 | 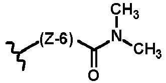 | 2.1-136 | 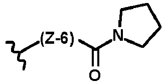 |
| 2.1-137 | 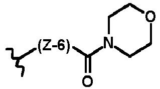 | 2.1-138 | 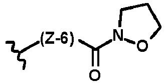 |
| 2.1-139 | 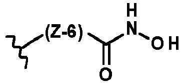 | 2.1-140 | 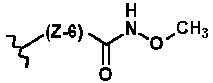 |
| 2.1-141 | 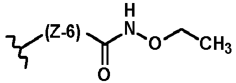 | 2.1-142 | 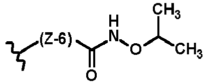 |
| 2.1-143 | 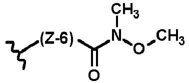 | 2.1-144 | 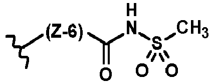 |
| 2.1-145 | 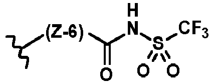 | 2.1-146 | 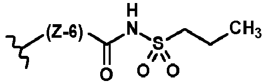 |
| 2.1-147 | 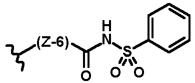 | 2.1-148 | 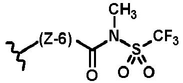 |
| 2.1-149 | 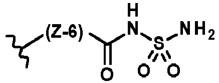 | 2.1-150 | 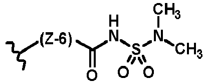 |
| 2.1-151 | 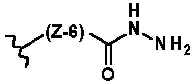 | 2.1-152 | 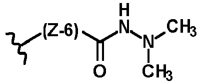 |
| 2.1-153 | 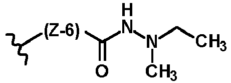 | 2.1-154 | 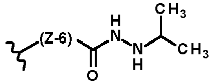 |
| 2.1-155 | 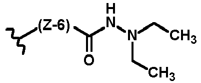 | 2.1-156 | 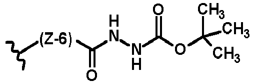 |
| 2.1-157 | 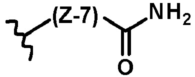 | 2.1-158 | 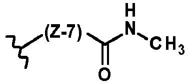 |
| 2.1-159 | 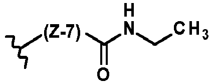 | 2.1-160 | 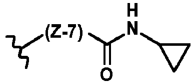 |
| 2.1-161 | 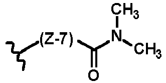 | 2.1-162 | 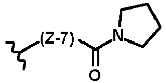 |
| 2.1-163 | 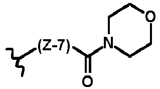 | 2.1-164 | 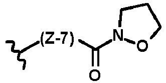 |
| 2.1-165 | 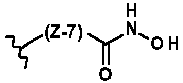 | 2.1-166 | 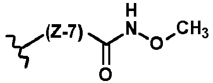 |
| 2.1-167 | 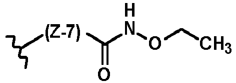 | 2.1-168 | 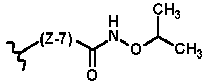 |
| 2.1-169 | 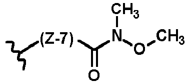 | 2.1-170 | 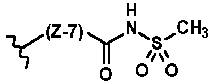 |
| 2.1-171 | 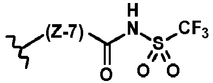 | 2.1-172 | 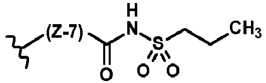 |
| 2.1-173 | 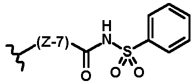 | 2.1-174 | 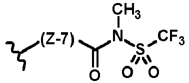 |
| 2.1-175 | 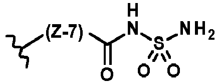 | 2.1-176 | 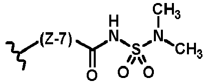 |
| 2.1-177 | 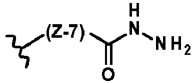 | 2.1-178 | 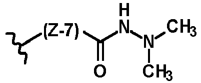 |
| 2.1-179 | 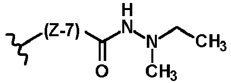 | 2.1-180 | 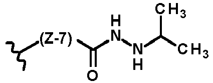 |
| 2.1-181 | 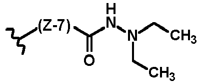 | 2.1-182 | 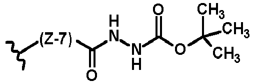 |
| 2.1-183 | 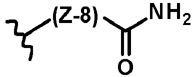 | 2.1-184 | 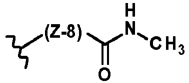 |
| 2.1-185 | 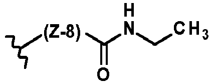 | 2.1-186 | 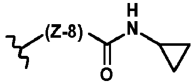 |
| 2.1-187 | 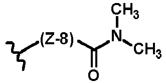 | 2.1-188 | 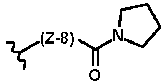 |
| 2.1-189 | 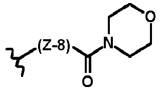 | 2.1-190 | 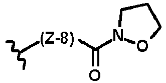 |
| 2.1-191 | 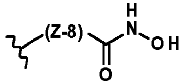 | 2.1-192 | 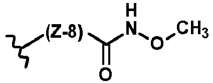 |
| 2.1-193 | 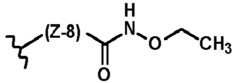 | 2.1-194 | 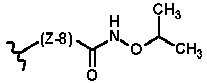 |
| 2.1-195 | 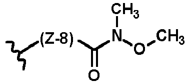 | 2.1-196 | 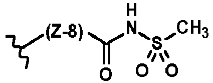 |
| 2.1-197 | 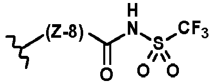 | 2.1-198 | 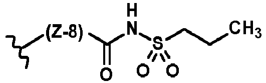 |
| 2.1-199 | 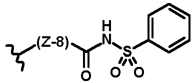 | 2.1-200 | 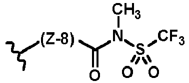 |
| 2.1-201 | 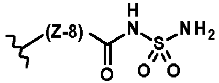 | 2.1-202 | 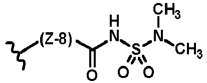 |
| 2.1-203 | 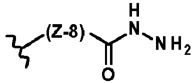 | 2.1-204 | 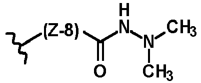 |
| 2.1-205 | 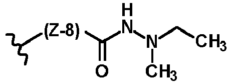 | 2.1-206 | 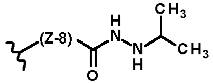 |
| 2.1-207 | 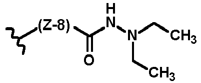 | 2.1-208 | 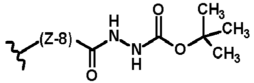 |
| 2.1-209 | 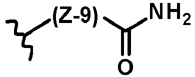 | 2.1-210 | 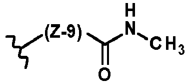 |
| 2.1-211 | 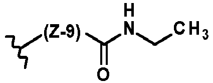 | 2.1-212 | 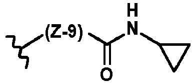 |
| 2.1-213 | 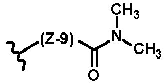 | 2.1-214 | 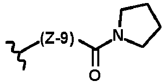 |
| 2.1-215 | 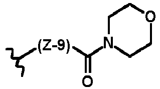 | 2.1-216 | 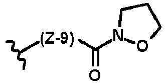 |
| 2.1-217 | 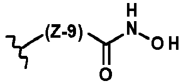 | 2.1-218 | 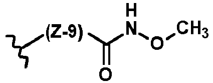 |
| 2.1-219 | 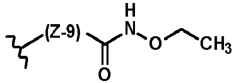 | 2.1-220 | 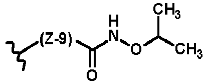 |
| 2.1-221 | 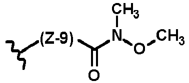 | 2.1-222 | 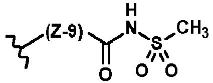 |
| 2.1-223 | 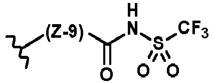 | 2.1-224 | 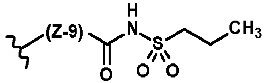 |
| 2.1-225 | 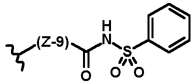 | 2.1-226 | 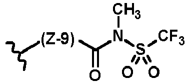 |
| 2.1-227 | 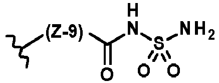 | 2.1-228 | 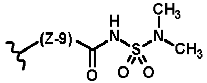 |
| 2.1-229 | 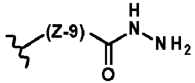 | 2.1-230 | 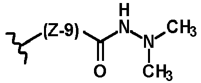 |
| 2.1-231 | 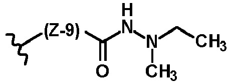 | 2.1-232 | 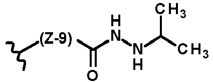 |
| 2.1-233 | 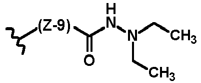 | 2.1-234 | 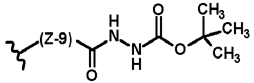 |
| 2.1-235 | 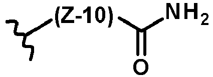 | 2.1-236 | 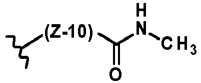 |
| 2.1-237 | 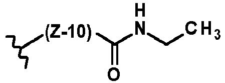 | 2.1-238 | 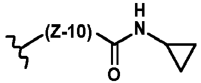 |
| 2.1-239 | 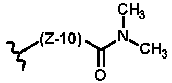 | 2.1-240 | 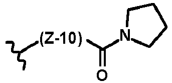 |
| 2.1-241 | 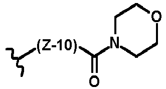 | 2.1-242 | 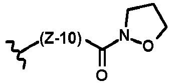 |
| 2.1-243 | 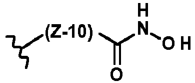 | 2.1-244 | 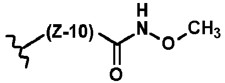 |
| 2.1-245 | 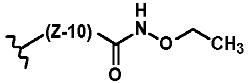 | 2.1-246 | 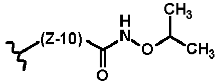 |
| 2.1-247 | 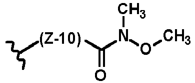 | 2.1-248 | 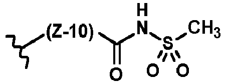 |
| 2.1-249 | 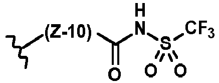 | 2.1-250 | 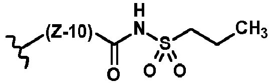 |
| 2.1-251 | 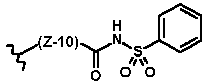 | 2.1-252 | 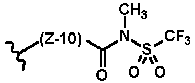 |
| 2.1-253 | 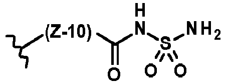 | 2.1-254 | 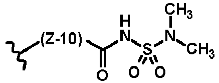 |
| 2.1-255 | 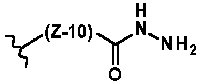 | 2.1-256 | 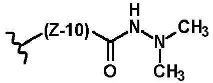 |
| 2.1-257 | 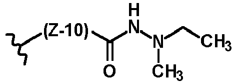 | 2.1-258 | 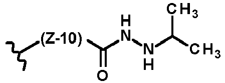 |
| 2.1-259 | 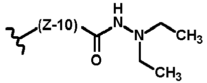 | 2.1-260 | 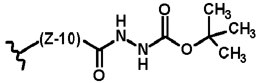 |
| 2.1-261 | 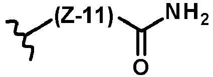 | 2.1-262 | 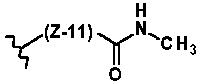 |
| 2.1-263 | 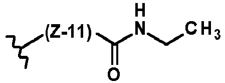 | 2.1-264 | 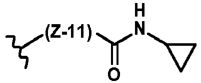 |
| 2.1-265 | 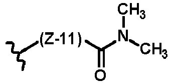 | 2.1-266 | 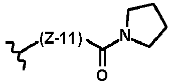 |
| 2.1-267 | 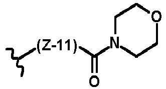 | 2.1-268 | 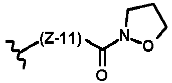 |
| 2.1-269 | 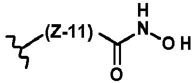 | 2.1-270 | 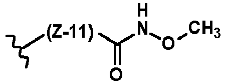 |
| 2.1-271 | 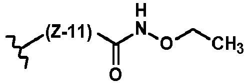 | 2.1-272 | 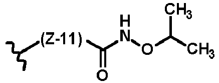 |
| 2.1-273 | 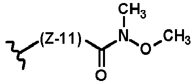 | 2.1-274 | 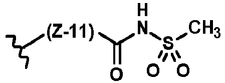 |
| 2.1-275 | 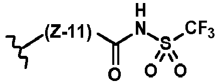 | 2.1-276 | 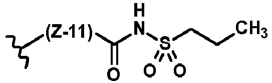 |
| 2.1-277 | 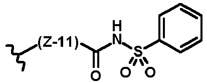 | 2.1-278 | 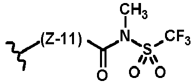 |
| 2.1-279 | 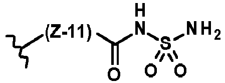 | 2.1-280 | 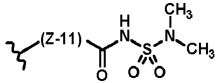 |
| 2.1-281 | 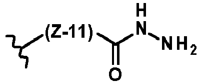 | 2.1-282 | 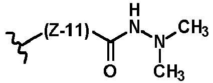 |
| 2.1-283 | 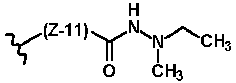 | 2.1-284 | 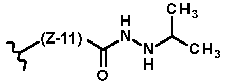 |
| 2.1-285 | 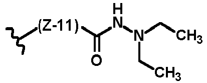 | 2.1-286 | 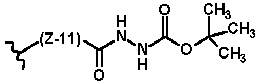 |
| 2.1-287 | 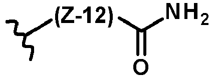 | 2.1-288 | 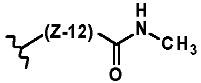 |
| 2.1-289 | 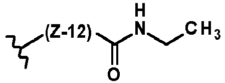 | 2.1-290 | 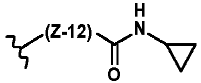 |
| 2.1-291 | 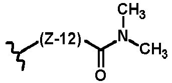 | 2.1-292 | 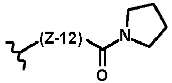 |
| 2.1-293 | 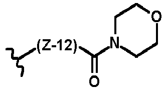 | 2.1-294 | 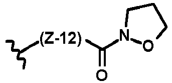 |
| 2.1-295 | 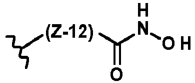 | 2.1-296 | 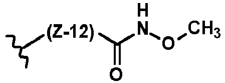 |
| 2.1-297 | 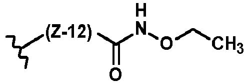 | 2.1-298 | 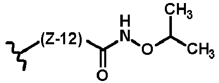 |
| 2.1-299 | 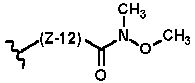 | 2.1-300 | 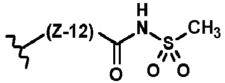 |
| 2.1-301 | 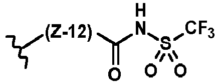 | 2.1-302 | 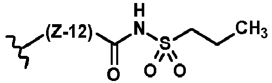 |
| 2.1-303 | 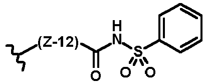 | 2.1-304 | 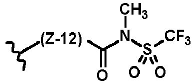 |
| 2.1-305 | 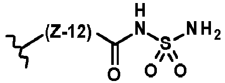 | 2.1-306 | 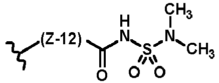 |
| 2.1-307 | 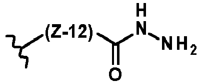 | 2.1-308 | 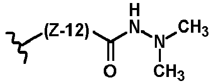 |
| 2.1-309 | 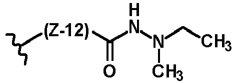 | 2.1-310 | 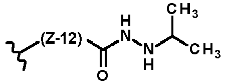 |
| 2.1-311 | 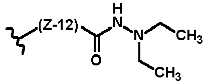 | 2.1-312 | 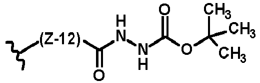 |
| 2.1-313 | 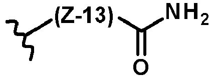 | 2.1-314 | 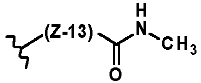 |
| 2.1-315 | 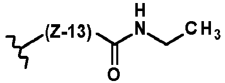 | 2.1-316 | 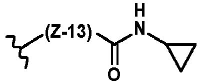 |
| 2.1-317 | 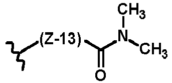 | 2.1-318 | 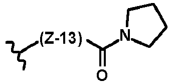 |
| 2.1-319 | 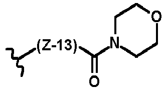 | 2.1-320 | 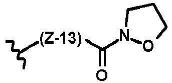 |
| 2.1-321 | 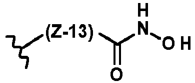 | 2.1-322 | 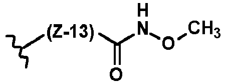 |
| 2.1-323 | 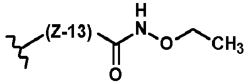 | 2.1-324 | 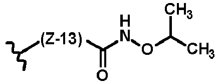 |
| 2.1-325 | 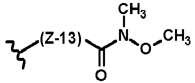 | 2.1-326 | 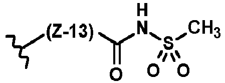 |
| 2.1-327 | 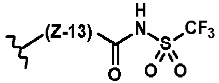 | 2.1-328 | 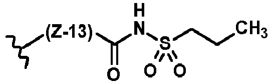 |
| 2.1-329 | 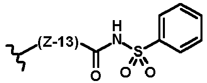 | 2.1-330 | 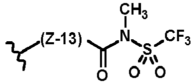 |
| 2.1-331 | 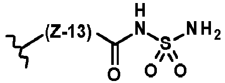 | 2.1-332 | 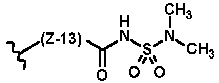 |
| 2.1-333 | 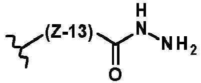 | 2.1-334 | 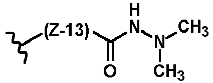 |
| 2.1-335 | 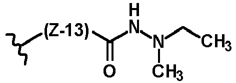 | 2.1-336 | 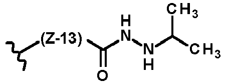 |
| 2.1-337 | 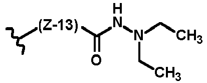 | 2.1-338 | 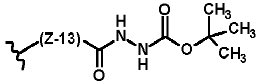 |
| 2.1-339 | 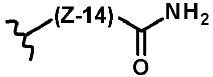 | 2.1-340 | 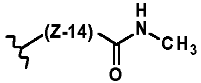 |
| 2.1-341 | 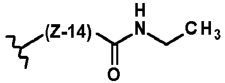 | 2.1-342 | 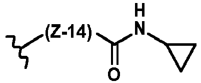 |
| 2.1-343 | 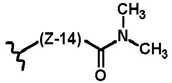 | 2.1-344 | 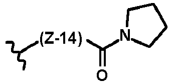 |
| 2.1-345 | 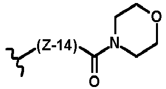 | 2.1-346 | 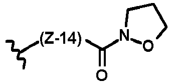 |
| 2.1-347 | 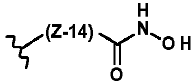 | 2.1-348 | 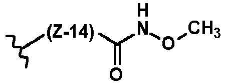 |
| 2.1-349 | 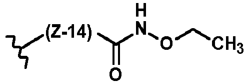 | 2.1-350 | 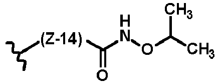 |
| 2.1-351 | 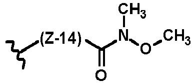 | 2.1-352 | 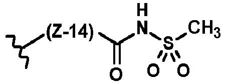 |
| 2.1-353 | 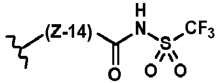 | 2.1-354 | 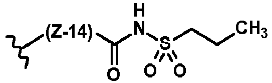 |
| 2.1-355 | 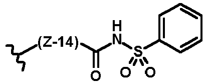 | 2.1-356 | 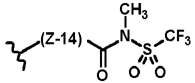 |
| 2.1-357 | 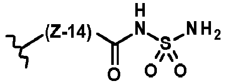 | 2.1-358 | 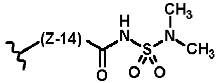 |
| 2.1-359 | 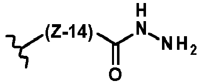 | 2.1-360 | 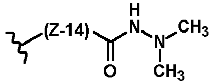 |
| 2.1-361 | 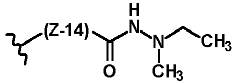 | 2.1-362 | 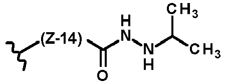 |
| 2.1-363 | 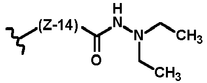 | 2.1-364 | 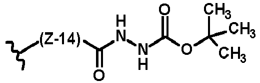 |
| 2.1-365 | 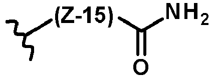 | 2.1-366 | 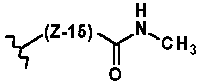 |
| 2.1-367 | 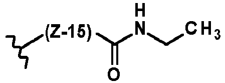 | 2.1-368 | 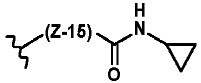 |
| 2.1-369 | 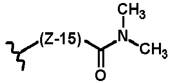 | 2.1-370 | 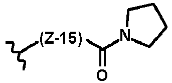 |
| 2.1-371 | 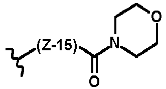 | 2.1-372 | 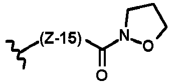 |
| 2.1-373 | 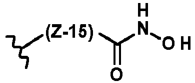 | 2.1-374 | 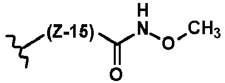 |
| 2.1-375 | 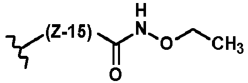 | 2.1-376 | 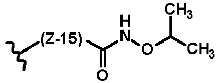 |
| 2.1-377 | 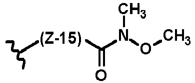 | 2.1-378 | 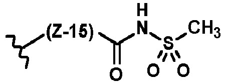 |
| 2.1-379 | 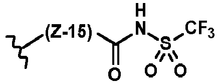 | 2.1-380 | 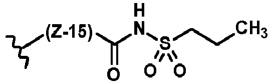 |
| 2.1-381 | 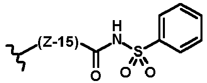 | 2.1-382 | 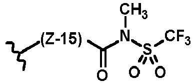 |
| 2.1-383 | 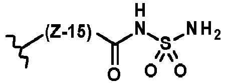 | 2.1-384 | 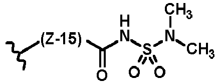 |
| 2.1-385 | 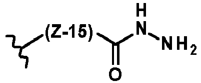 | 2.1-386 | 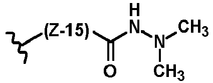 |
| 2.1-387 | 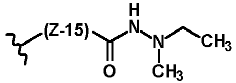 | 2.1-388 | 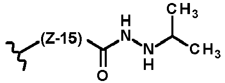 |
| 2.1-389 | 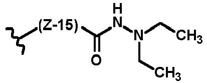 | 2.1-390 | 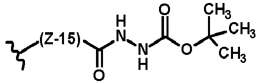 |

Tabelle 2.2: Erfindungsgemäße Verbindungen 2.2-1 bis 2.2-390 der allgemeinen Formel (I.2), worin Z-(C=W)-N(R¹¹)-R¹² wie in Tabelle 2.1 definiert ist.

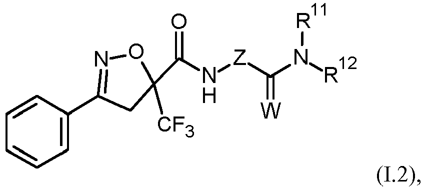

Tabelle 2.3: Erfindungsgemäße Verbindungen 2.3-1 bis 2.3-390 der allgemeinen Formel (I.3), worin Z-(C=W)-N(R¹¹)-R¹² wie in Tabelle 2.1 definiert ist.

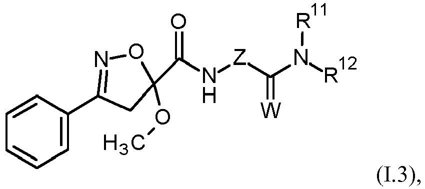

Tabelle 2.4: Erfindungsgemäße Verbindungen 2.4-1 bis 2.4-390 der allgemeinen Formel (I.4), worin Z-(C=W)-N(R¹¹)-R¹² wie in Tabelle 2.1 definiert ist.

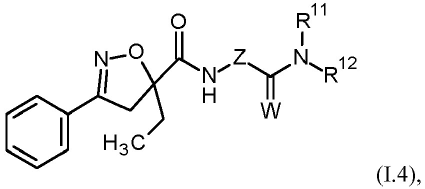

Tabelle 2.5: Erfindungsgemäße Verbindungen 2.5-1 bis 2.5-390 der allgemeinen Formel (I.5), worin Z-(C=W)-N(R¹¹)-R¹² wie in Tabelle 2.1 definiert ist.

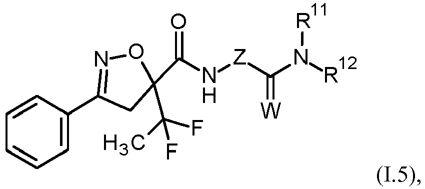

Tabelle 2.6: Erfindungsgemäße Verbindungen 2.6-1 bis 2.6-390 der allgemeinen Formel (I.6), worin Z-(C=W)-N(R¹¹)-R¹² wie in Tabelle 2.1 definiert ist.

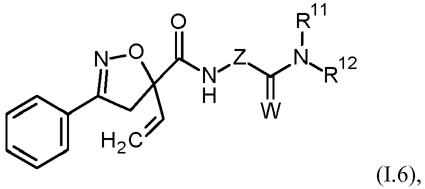

Tabelle 2.7: Erfindungsgemäße Verbindungen 2.7-1 bis 2.7-390 der allgemeinen Formel (I.7), worin Z-(C=W)-N(R¹¹)-R¹² wie in Tabelle 2.1 definiert ist.

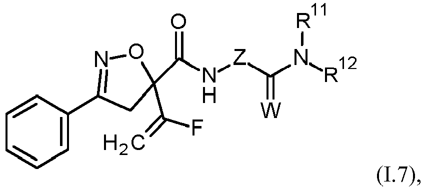

Tabelle 2.8: Erfindungsgemäße Verbindungen 2.8-1 bis 2.8-390 der allgemeinen Formel (I.8), worin Z-(C=W)-N(R¹¹)-R¹² wie in Tabelle 2.1 definiert ist.

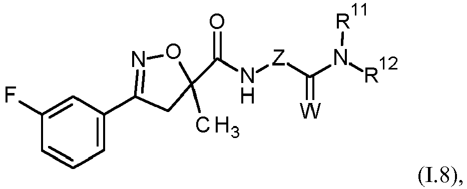

Tabelle 2.9: Erfindungsgemäße Verbindungen 2.9-1 bis 2.9-390 der allgemeinen Formel (I.9), worin Z-(C=W)-N(R¹¹)-R¹² wie in Tabelle 2.1 definiert ist.

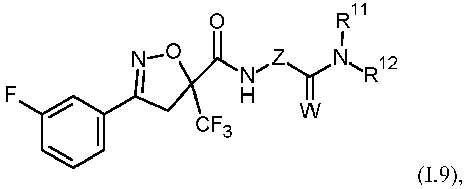

Tabelle 2.10: Erfindungsgemäße Verbindungen 2.10-1 bis 2.10-390 der allgemeinen Formel (I.10), worin Z-(C=W)-N(R¹¹)-R¹² wie in Tabelle 2.1 definiert ist.

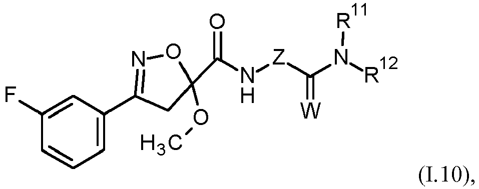

Tabelle 2.11: Erfindungsgemäße Verbindungen 2.11-1 bis 2.11-390 der allgemeinen Formel (I.11), worin Z-(C=W)-N(R¹¹)-R¹² wie in Tabelle 2.1 definiert ist.

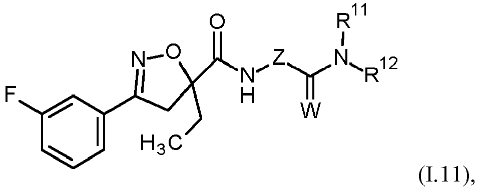

Tabelle 2.12: Erfindungsgemäße Verbindungen 2.12-1 bis 2.12-390 der allgemeinen Formel (I.12), worin Z-(C=W)-N(R¹¹)-R¹² wie in Tabelle 2.1 definiert ist.

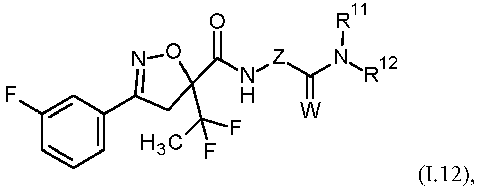

Tabelle 2.13: Erfindungsgemäße Verbindungen 2.13-1 bis 2.13-390 der allgemeinen Formel (I.13), worin Z-(C=W)-N(R¹¹)-R¹² wie in Tabelle 2.1 definiert ist.

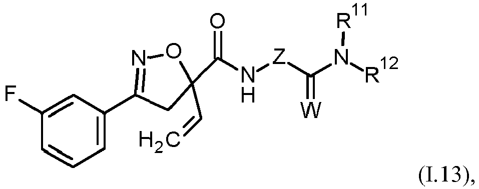

Tabelle 2.14: Erfindungsgemäße Verbindungen 2.14-1 bis 2.14-390 der allgemeinen Formel (I.14), worin Z-(C=W)-N(R¹¹)-R¹² wie in Tabelle 2.1 definiert ist.

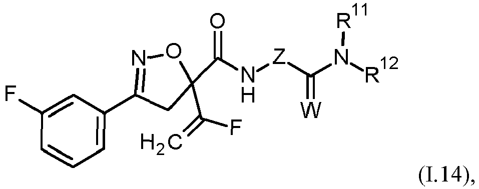

Tabelle 2.15: Erfindungsgemäße Verbindungen 2.15-1 bis 2.15-390 der allgemeinen Formel (I.15), worin Z-(C=W)-N(R¹¹)-R¹² wie in Tabelle 2.1 definiert ist.

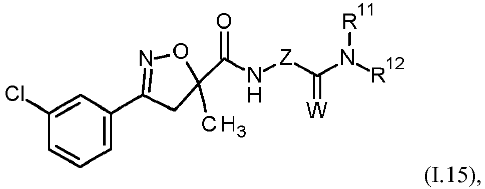

Tabelle 2.16: Erfindungsgemäße Verbindungen 2.16-1 bis 2.16-390 der allgemeinen Formel (I.16), worin Z-(C=W)-N(R¹¹)-R¹² wie in Tabelle 2.1 definiert ist.

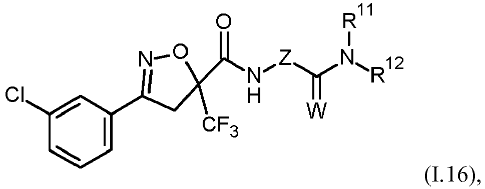

Tabelle 2.17: Erfindungsgemäße Verbindungen 2.17-1 bis 2.17-390 der allgemeinen Formel (I.17), worin Z-(C=W)-N(R¹¹)-R¹² wie in Tabelle 2.1 definiert ist.

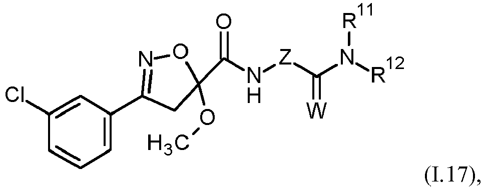

Tabelle 2.18: Erfindungsgemäße Verbindungen 2.18-1 bis 2.18-390 der allgemeinen Formel (I.18), worin Z-(C=W)-N(R¹¹)-R¹² wie in Tabelle 2.1 definiert ist.

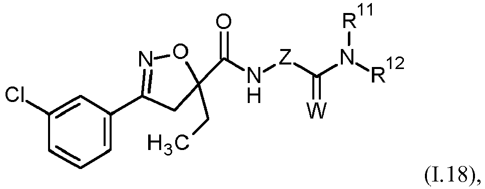

Tabelle 2.19: Erfindungsgemäße Verbindungen 2.19-1 bis 2.19-390 der allgemeinen Formel (I.19), worin Z-(C=W)-N(R¹¹)-R¹² wie in Tabelle 2.1 definiert ist.

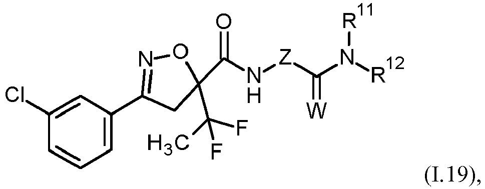

Tabelle 2.20: Erfindungsgemäße Verbindungen 2.20-1 bis 2.20-390 der allgemeinen Formel (I.20), worin Z-(C=W)-N(R¹¹)-R¹² wie in Tabelle 2.1 definiert ist.

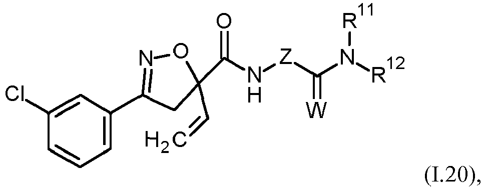

Tabelle 2.21: Erfindungsgemäße Verbindungen 2.21-1 bis 2.21-390 der allgemeinen Formel (1.21), worin Z-(C=W)-N(R¹¹)-R¹² wie in Tabelle 2.1 definiert ist.

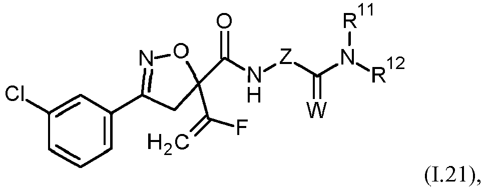

Tabelle 2.22: Erfindungsgemäße Verbindungen 2.22-1 bis 2.22-390 der allgemeinen Formel (I.22), worin Z-(C=W)-N(R¹¹)-R¹² wie in Tabelle 2.1 definiert ist.

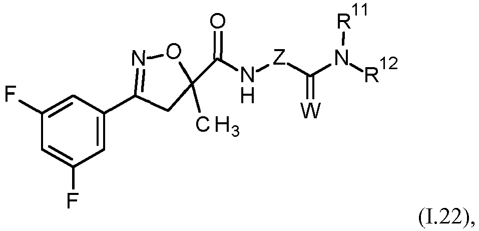

Tabelle 2.23: Erfindungsgemäße Verbindungen 2.23-1 bis 2.23-390 der allgemeinen Formel (I.23), worin Z-(C=W)-N(R¹¹)-R¹² wie in Tabelle 2.1 definiert ist.

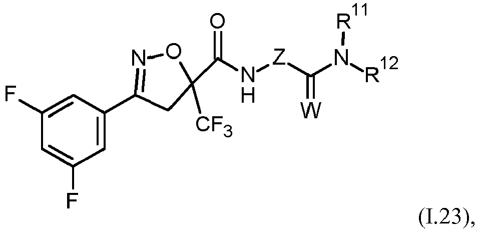

Tabelle 2.24: Erfindungsgemäße Verbindungen 2.24-1 bis 2.24-390 der allgemeinen Formel (I.24), worin Z-(C=W)-N(R¹¹)-R¹² wie in Tabelle 2.1 definiert ist.

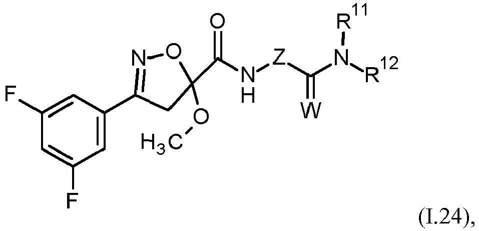

Tabelle 2.25: Erfindungsgemäße Verbindungen 2.25-1 bis 2.25-390 der allgemeinen Formel (I.25), worin Z-(C=W)-N(R¹¹)-R¹² wie in Tabelle 2.1 definiert ist.

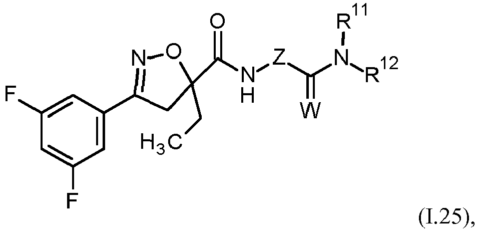

Tabelle 2.26: Erfindungsgemäße Verbindungen 2.26-1 bis 2.26-390 der allgemeinen Formel (I.26), worin Z-(C=W)-N(R¹¹)-R¹² wie in Tabelle 2.1 definiert ist.

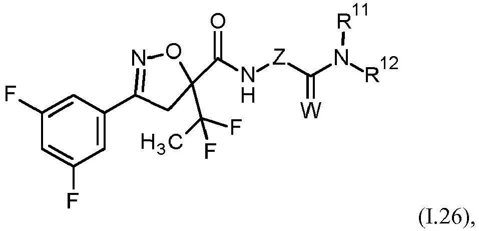

Tabelle 2.27: Erfindungsgemäße Verbindungen 2.27-1 bis 2.27-390 der allgemeinen Formel (I.27), worin Z-(C=W)-N(R¹¹)-R¹² wie in Tabelle 2.1 definiert ist.

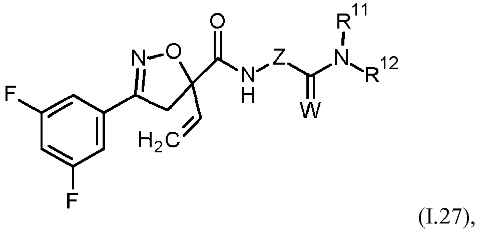

Tabelle 2.28: Erfindungsgemäße Verbindungen 2.28-1 bis 2.28-390 der allgemeinen Formel (I.28), worin Z-(C=W)-N(R¹¹)-R¹² wie in Tabelle 2.1 definiert ist.

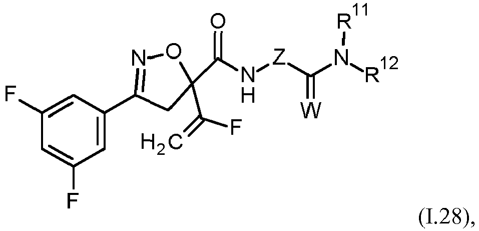

Tabelle 2.29: Erfindungsgemäße Verbindungen 2.29-1 bis 2.29-390 der allgemeinen Formel (I.29), worin Z-(C=W)-N(R¹¹)-R¹² wie in Tabelle 2.1 definiert ist.

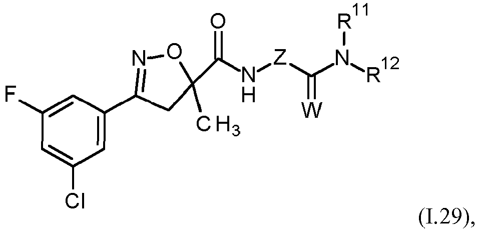

Tabelle 2.30: Erfindungsgemäße Verbindungen 2.30-1 bis 2.30-390 der allgemeinen Formel (I.30), worin Z-(C=W)-N(R¹¹)-R¹² wie in Tabelle 2.1 definiert ist.

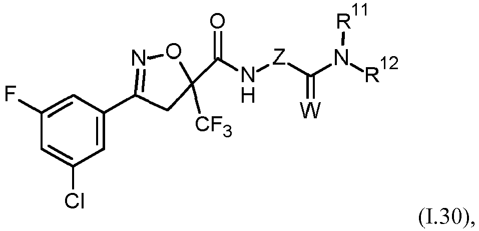

Tabelle 2.31: Erfindungsgemäße Verbindungen 2.31-1 bis 2.31-390 der allgemeinen Formel (I.31), worin Z-(C=W)-N(R¹¹)-R¹² wie in Tabelle 2.1 definiert ist.

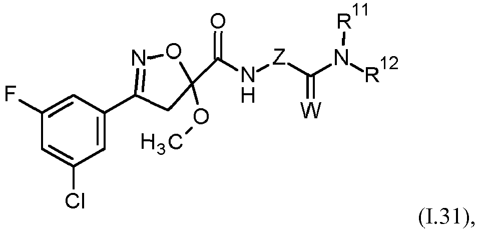

Tabelle 2.32: Erfindungsgemäße Verbindungen 2.32-1 bis 2.32-390 der allgemeinen Formel (I.32), worin Z-(C=W)-N(R¹¹)-R¹² wie in Tabelle 2.1 definiert ist.

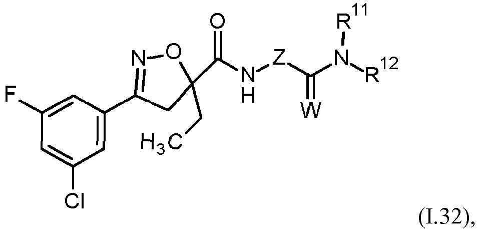

Tabelle 2.33: Erfindungsgemäße Verbindungen 2.33-1 bis 2.33-390 der allgemeinen Formel (I.33), worin Z-(C=W)-N(R¹¹)-R¹² wie in Tabelle 2.1 definiert ist.

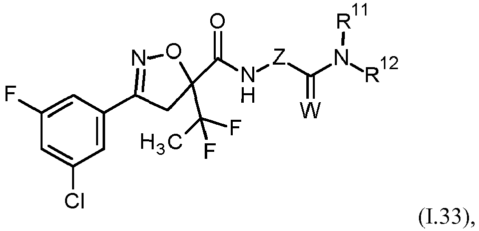

Tabelle 2.34: Erfindungsgemäße Verbindungen 2.34-1 bis 2.34-390 der allgemeinen Formel (I.34), worin Z-(C=W)-N(R¹¹)-R¹² wie in Tabelle 2.1 definiert ist.

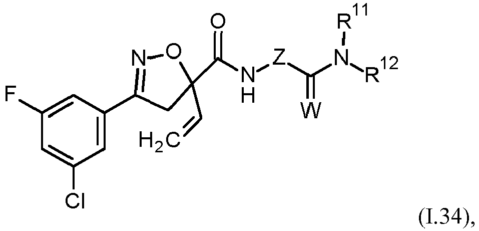

Tabelle 2.35: Erfindungsgemäße Verbindungen 2.35-1 bis 2.35-390 der allgemeinen Formel (I.35), worin Z-(C=W)-N(R¹¹)-R¹² wie in Tabelle 2.1 definiert ist.

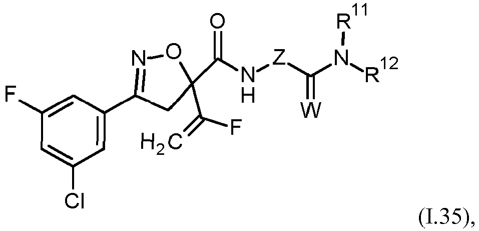

Tabelle 2.36: Erfindungsgemäße Verbindungen 2.36-1 bis 2.36-390 der allgemeinen Formel (I.36), worin Z-(C=W)-N(R¹¹)-R¹² wie in Tabelle 2.1 definiert ist.

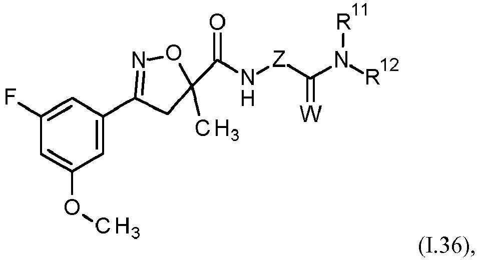

Tabelle 2.37: Erfindungsgemäße Verbindungen 2.37-1 bis 2.37-390 der allgemeinen Formel (I.37), worin Z-(C=W)-N(R¹¹)-R¹² wie in Tabelle 2.1 definiert ist.

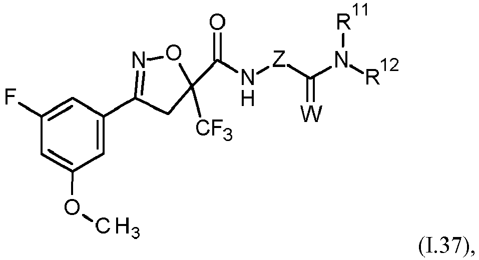

Tabelle 2.38: Erfindungsgemäße Verbindungen 2.38-1 bis 2.38-390 der allgemeinen Formel (I.38), worin Z-(C=W)-N(R¹¹)-R¹² wie in Tabelle 2.1 definiert ist.

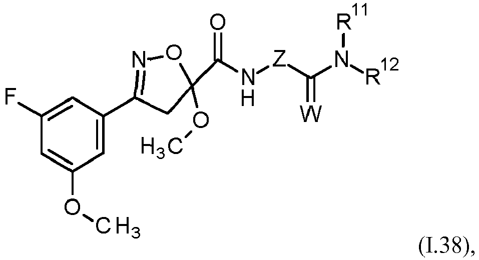

Tabelle 2.39: Erfindungsgemäße Verbindungen 2.39-1 bis 2.39-390 der allgemeinen Formel (I.39), worin Z-(C=W)-N(R¹¹)-R¹² wie in Tabelle 2.1 definiert ist.

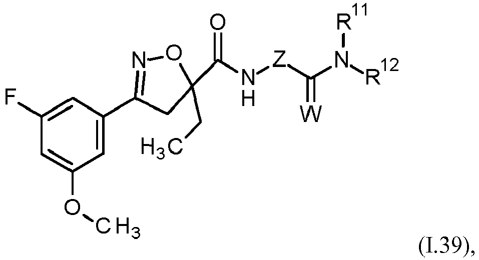

Tabelle 2.40: Erfindungsgemäße Verbindungen 2.40-1 bis 2.40-390 der allgemeinen Formel (I.40), worin Z-(C=W)-N(R¹¹)-R¹² wie in Tabelle 2.1 definiert ist.

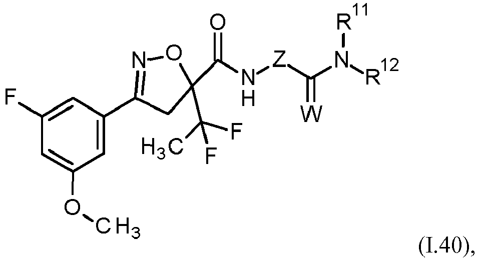

Tabelle 2.41: Erfindungsgemäße Verbindungen 2.41-1 bis 2.41-390 der allgemeinen Formel (1.41), worin Z-(C=W)-N(R¹¹)-R¹² wie in Tabelle 2.1 definiert ist.

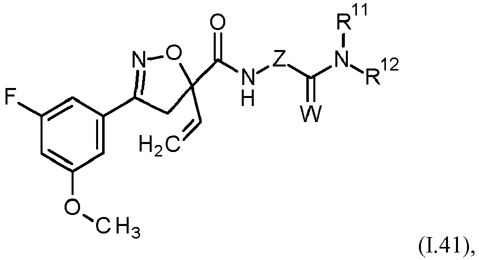

Tabelle 2.42: Erfindungsgemäße Verbindungen 2.42-1 bis 2.42-390 der allgemeinen Formel (I.42), worin Z-(C=W)-N(R¹¹)-R¹² wie in Tabelle 2.1 definiert ist.

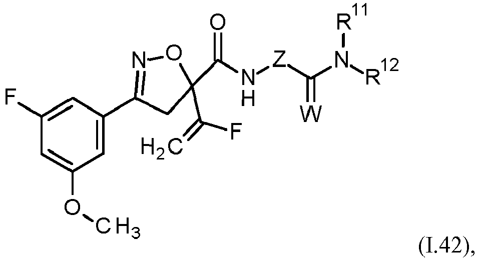

Tabelle 2.43: Erfindungsgemäße Verbindungen 2.43-1 bis 2.43-390 der allgemeinen Formel (I.43), worin Z-(C=W)-N(R¹¹)-R¹² wie in Tabelle 2.1 definiert ist.

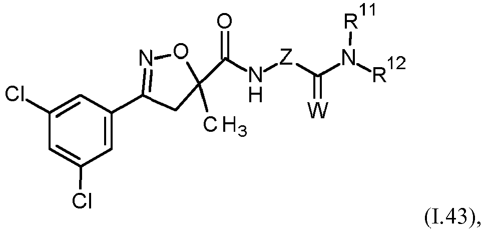

Tabelle 2.44: Erfindungsgemäße Verbindungen 2.44-1 bis 2.44-390 der allgemeinen Formel (I.44), worin Z-(C=W)-N(R¹¹)-R¹² wie in Tabelle 2.1 definiert ist.

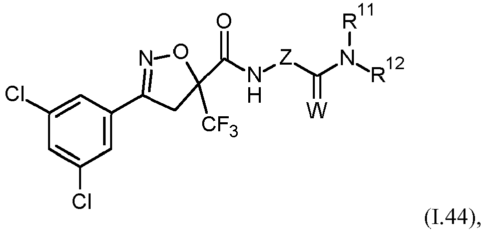

Tabelle 2.45: Erfindungsgemäße Verbindungen 2.45-1 bis 2.45-390 der allgemeinen Formel (I.45), worin Z-(C=W)-N(R¹¹)-R¹² wie in Tabelle 2.1 definiert ist.

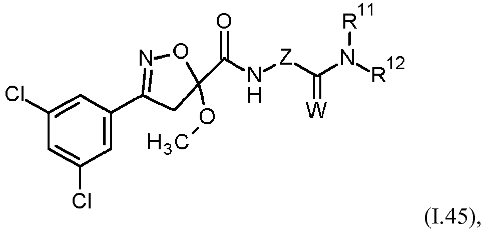

Tabelle 2.46: Erfindungsgemäße Verbindungen 2.46-1 bis 2.46-390 der allgemeinen Formel (I.46), worin Z-(C=W)-N(R¹¹)-R¹² wie in Tabelle 2.1 definiert ist.

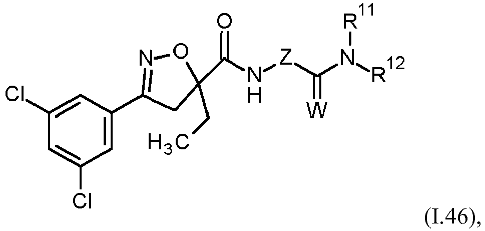

Tabelle 2.47: Erfindungsgemäße Verbindungen 2.47 bis 2.47-390 der allgemeinen Formel (I.47), worin Z-(C=W)-N(R¹¹)-R¹² wie in Tabelle 2.1 definiert ist.

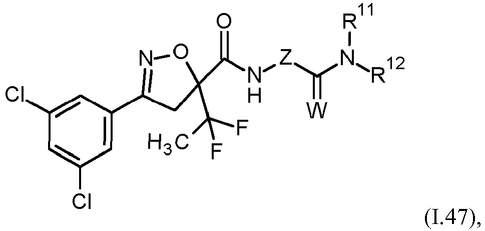

Tabelle 2.48: Erfindungsgemäße Verbindungen 2.48-1 bis 2.48-390 der allgemeinen Formel (I.48), worin Z-(C=W)-N(R¹¹)-R¹² wie in Tabelle 2.1 definiert ist.

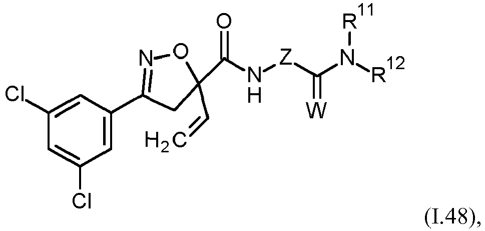

Tabelle 2.49: Erfindungsgemäße Verbindungen 2.49-1 bis 2.49-390 der allgemeinen Formel (I.49), worin Z-(C=W)-N(R¹¹)-R¹² wie in Tabelle 2.1 definiert ist.

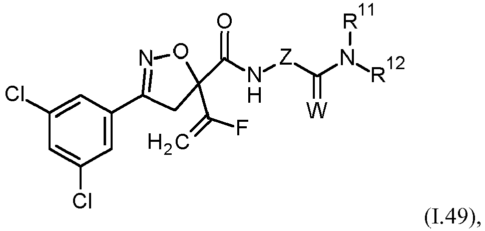

Tabelle 2.50: Erfindungsgemäße Verbindungen 2.50-1 bis 2.50-390 der allgemeinen Formel (I.50), worin Z-(C=W)-N(R¹¹)-R¹² wie in Tabelle 2.1 definiert ist.

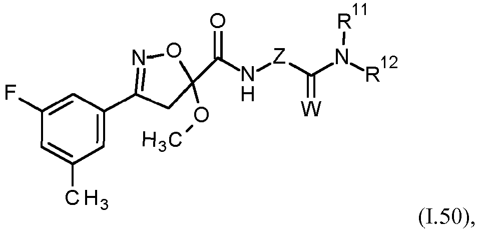

Tabelle 2.51: Erfindungsgemäße Verbindungen 2.51-1 bis 2.51-390 der allgemeinen Formel (I.51), worin Z-(C=W)-N(R¹¹)-R¹² wie in Tabelle 2.1 definiert ist.

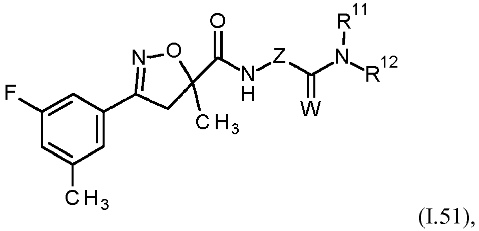

Tabelle 2.52: Erfindungsgemäße Verbindungen 2.52-1 bis 2.52-390 der allgemeinen Formel (I.52), worin Z-(C=W)-N(R¹¹)-R¹² wie in Tabelle 2.1 definiert ist.

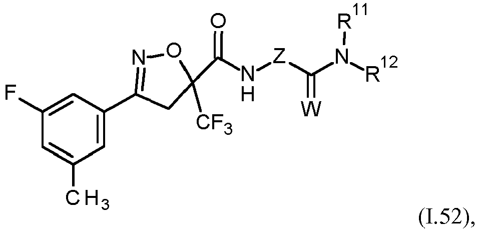

Tabelle 2.53: Erfindungsgemäße Verbindungen 2.53-1 bis 2.53-390 der allgemeinen Formel (I.53), worin Z-(C=W)-N(R¹¹)-R¹² wie in Tabelle 2.1 definiert ist.

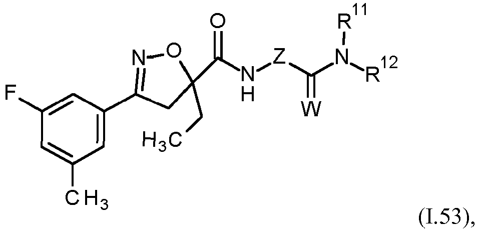

Tabelle 2.54: Erfindungsgemäße Verbindungen 2.54-1 bis 2.54-390 der allgemeinen Formel (I.54), worin Z-(C=W)-N(R¹¹)-R¹² wie in Tabelle 2.1 definiert ist.

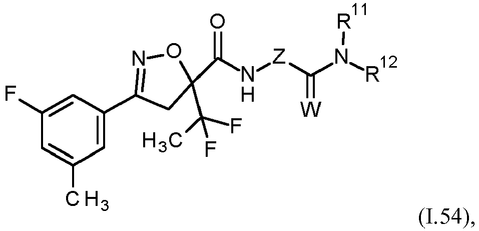

Tabelle 2.55: Erfindungsgemäße Verbindungen 2.55-1 bis 2.55-390 der allgemeinen Formel (I.55), worin Z-(C=W)-N(R¹¹)-R¹² wie in Tabelle 2.1 definiert ist.

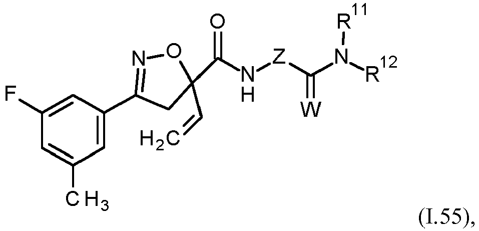

Tabelle 2.56: Erfindungsgemäße Verbindungen 2.56-1 bis 2.56-390 der allgemeinen Formel (I.56), worin Z-(C=W)-N(R¹¹)-R¹² wie in Tabelle 2.1 definiert ist.

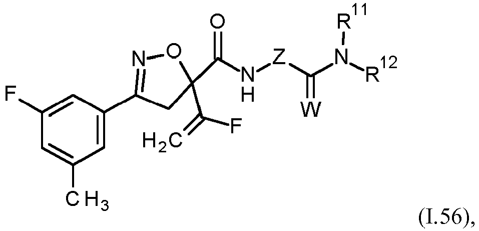

### B. Formulierungsbeispiele

### 1. Stäubemittel

Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man
75 Gew.-Teile einer Verbindung der Formel (I),

| | | |
|---|---|---|
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man
25 Gew.-Teile einer Verbindung der Formel (I),

| | | |
|---|---|---|
| 5 | " | 2,2'-dinaphthylmethan-6,6'-disulfonsaures Natrium, |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Versuchsbeschreibung

1. Herbizide Wirkung und Kulturverträglichkeit gegen Schadpflanzen im Vorauflauf Samen von mono- bzw. dikotylen Unkraut und Kulturpflanzen werden in Kunststoff- oder Holzfasertöpfen ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

In den nachfolgenden Tabellen werden folgende Abkürzungen verwendet:

### Unerwünschte Pflanzen / Weeds:

| | | | |
|---|---|---|---|
| ABUTH: | *Abutilon theophrasti* | ALOMY: | *Alopecurus myosuroides* |
| AMARE: | *Amaranthus retroflexus* | AVEFA: | *Avena fatua* |
| CYPES: | *Cyperus esculentus* | ECHCG: | *Echinochloa crus-galli* |
| HORMU: | *Hordeum murinum* | LOLRI: | *Lolium rigidum* |
| PHBPU: | *Pharbitis purpurea* | POLCO: | *Polygonum convolvulus* |
| SETVI: | *Setaria viridis* | STEME: | *Stellaria media* |
| VERPE: | *Veronica persica* | VIOTR: | *Viola tricolor* |

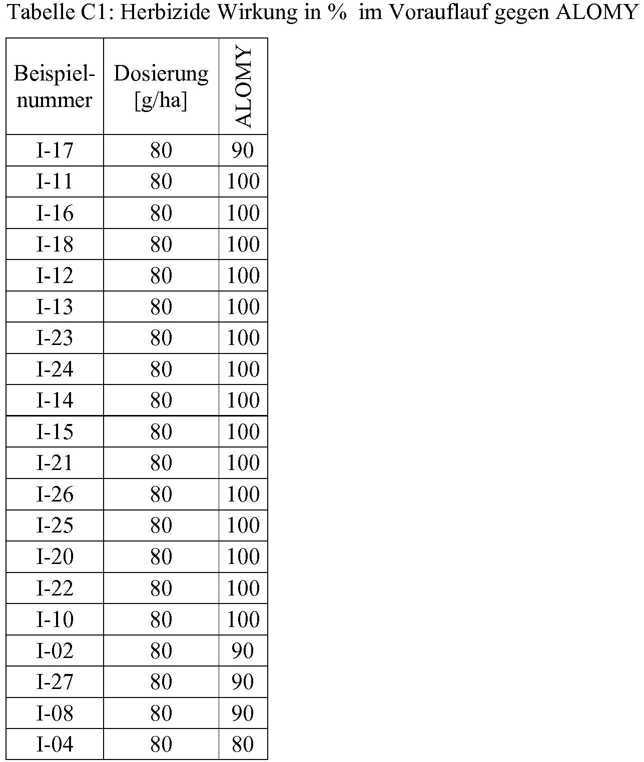

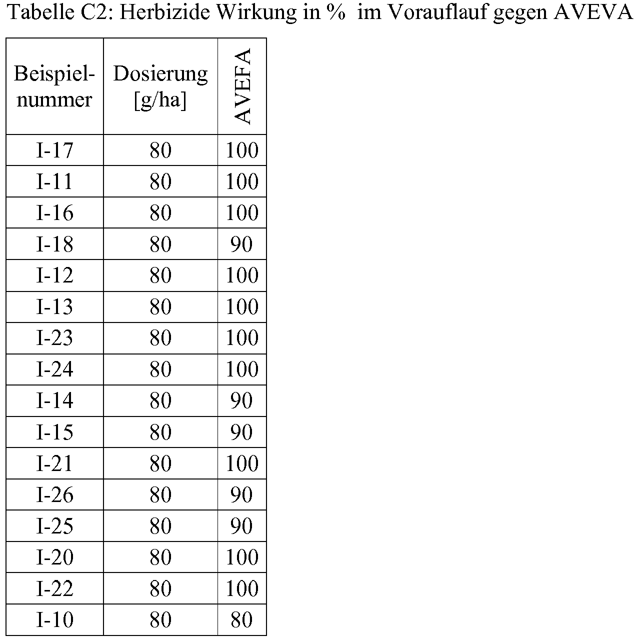

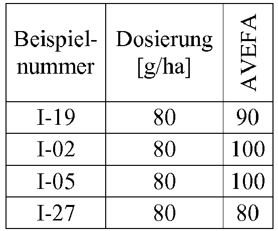

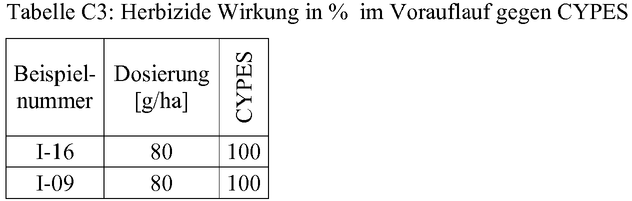

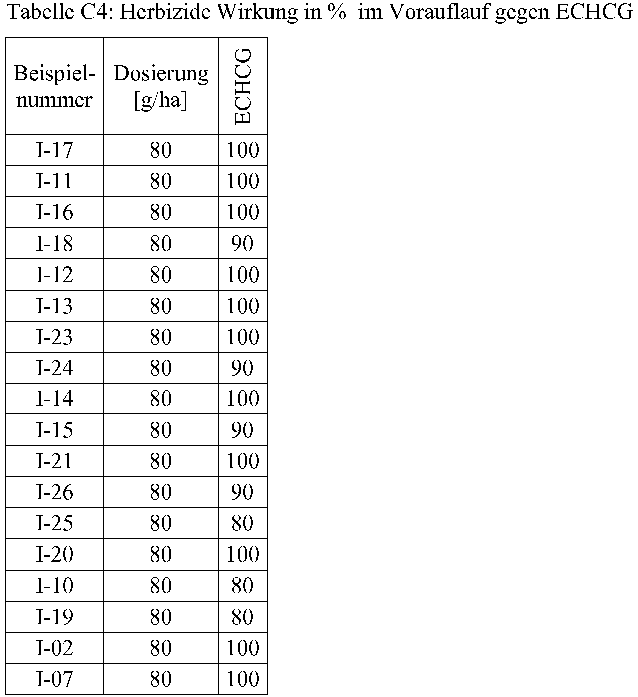

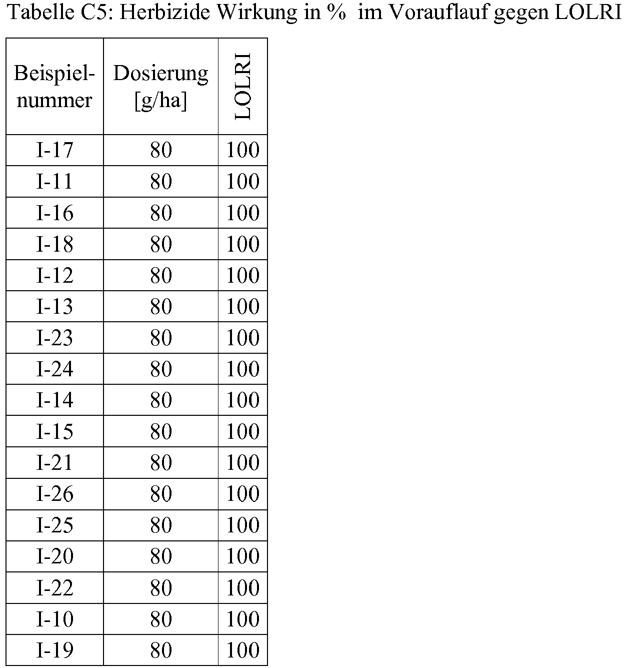

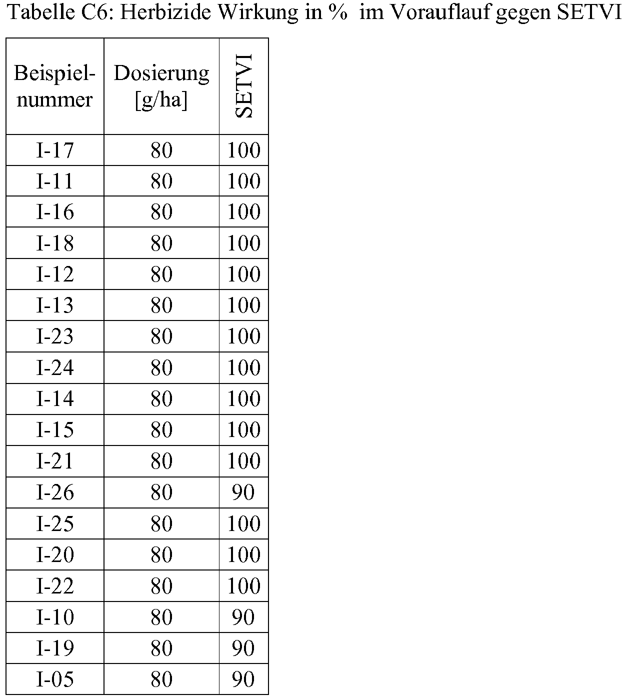

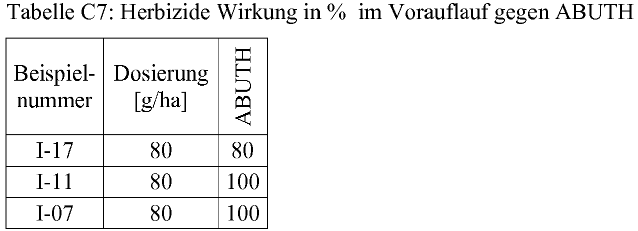

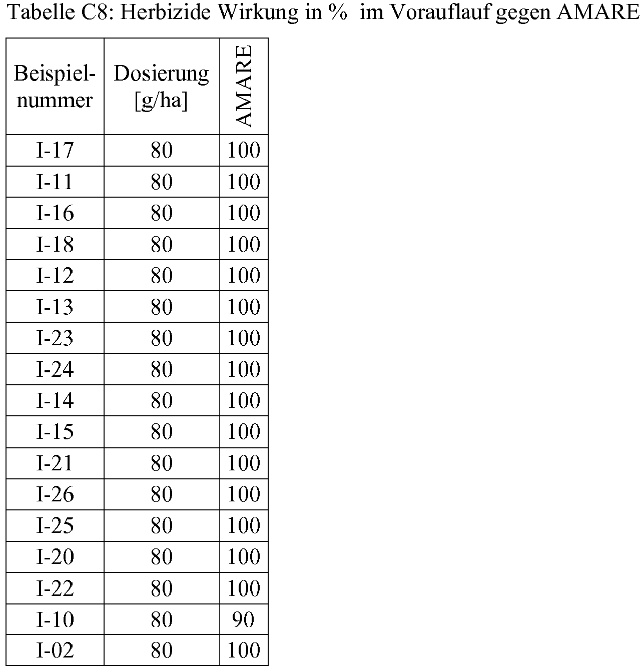

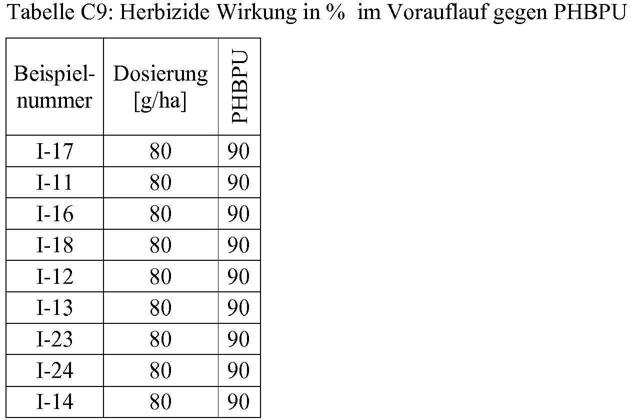

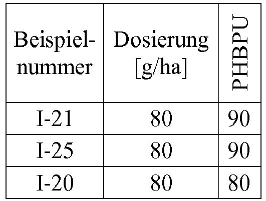

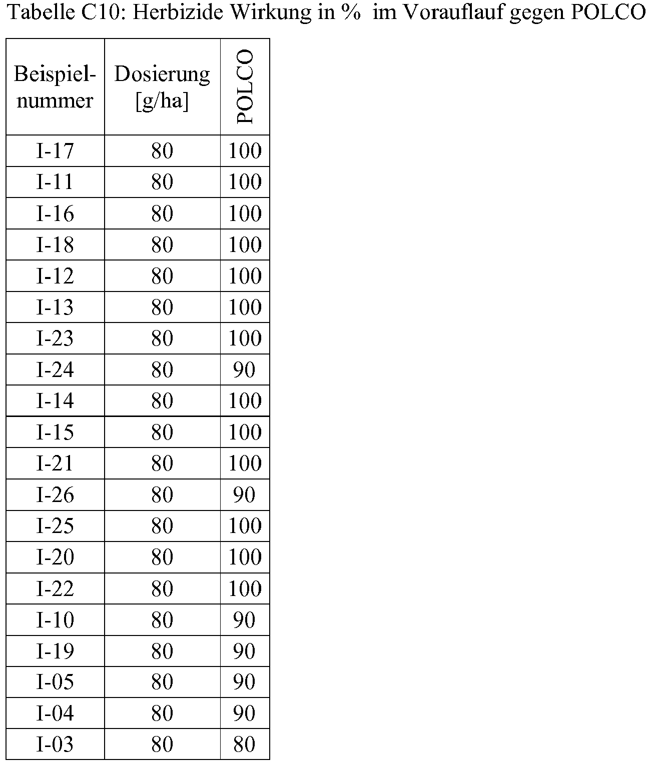

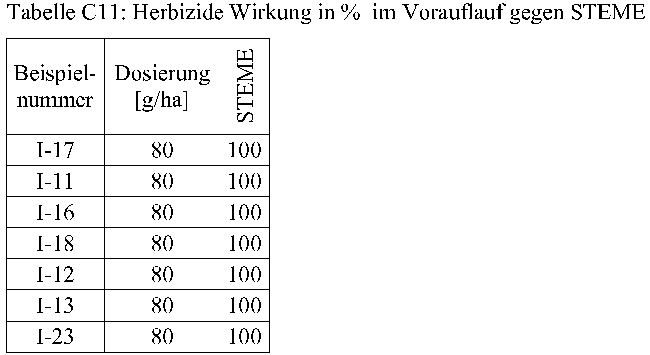

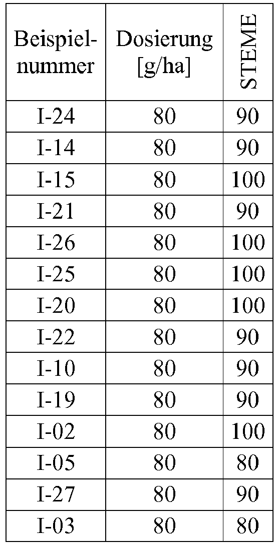

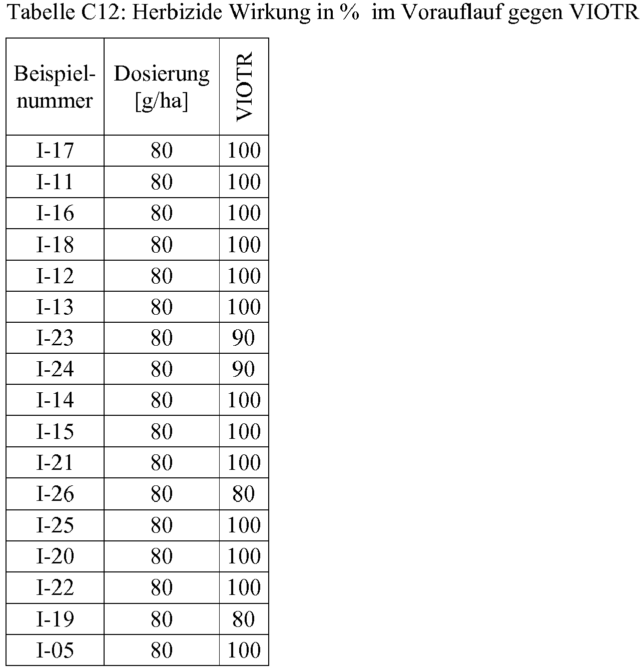

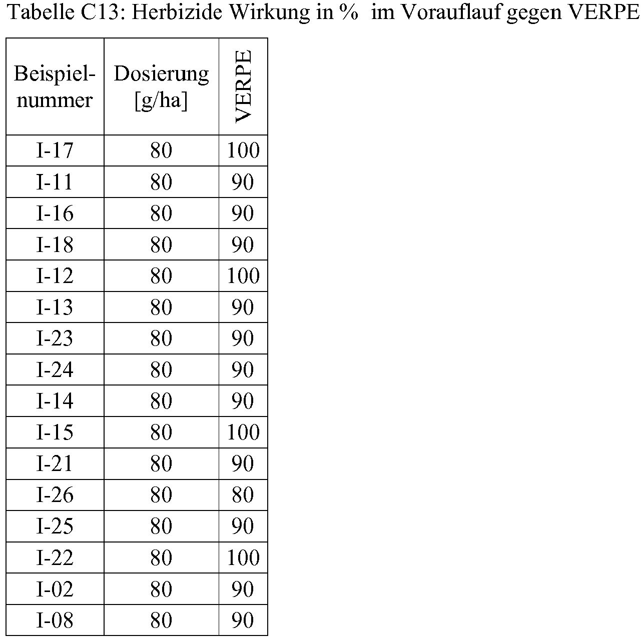

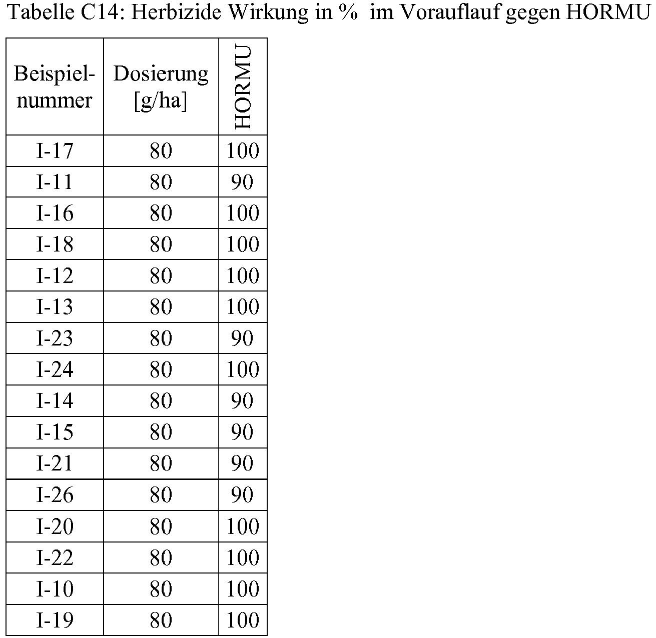

Wie die Ergebnisse zeigen, weisen erfindungsgemäße Verbindungen, wie beispielsweise die Verbindungen Nr. I-02 und andere Verbindungen aus den Tabellen (I-05, I-10, I-11, I-12, I-13, I-14, I-15, I-16, I-17, I-18, I-19, I-20, I-21, I-22, I-23, I-24, I-25, I-26, I-27, I-28, I-29) bei Behandlung im Vorauflauf eine sehr gute Wirkung (80% bis 100% herbizide Wirkung) gegen Schadpflanzen wie *Abutilon theophrasti, Alopecurus myosuroides, Amaranthus retroflexus, Avena fatua, Cyperus esculentus, Echinochloa crus-galli, Hordeum murinum, Lolium rigidum, Pharbitis purpurea, Polygonum convolvulus, Setaria viridis, Stellaria media, Veronica persica* und *Viola tricolor* bei einer Aufwandmenge von 0.08 kg Aktivsubstanz oder weniger pro Hektar auf.

### 2. Herbizide Wirkung und Kulturverträglichkeit gegen Schadpflanzen im Nachauflauf

Samen von mono- bzw. dikotylen Unkraut- bzw. Kulturpflanzen werden in Kunststoff- oder Holzfasertöpfen in sandigem Lehmboden ausgelegt, mit Erde abgedeckt und im Gewächshaus unter kontrollierten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion unter Zusatz von 0,5% Additiv mit einer Wasseraufwandmenge von umgerechnet 600 l/ha auf die grünen Pflanzenteile gesprüht. Nach ca. 3 Wochen Standzeit der Versuchspflanzen im Gewächshaus, unter optimalen Wachstumsbedingungen, wird die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

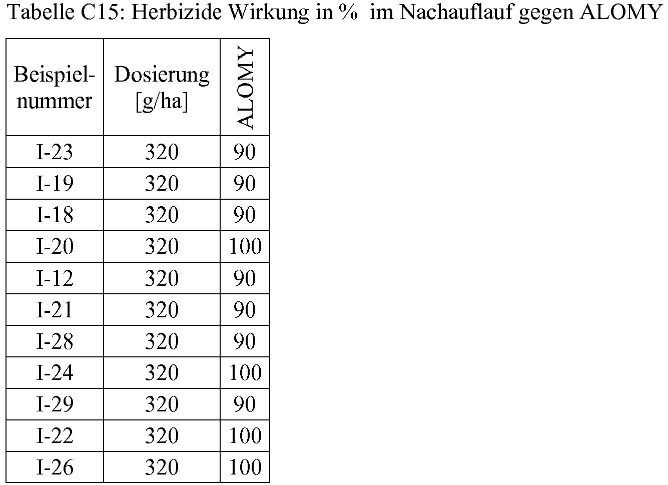

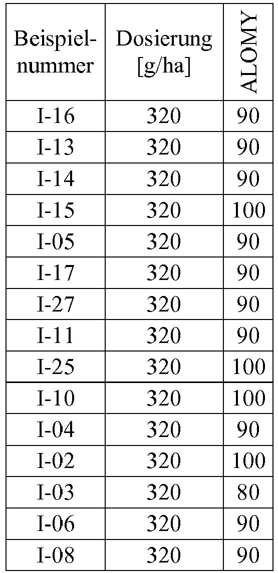

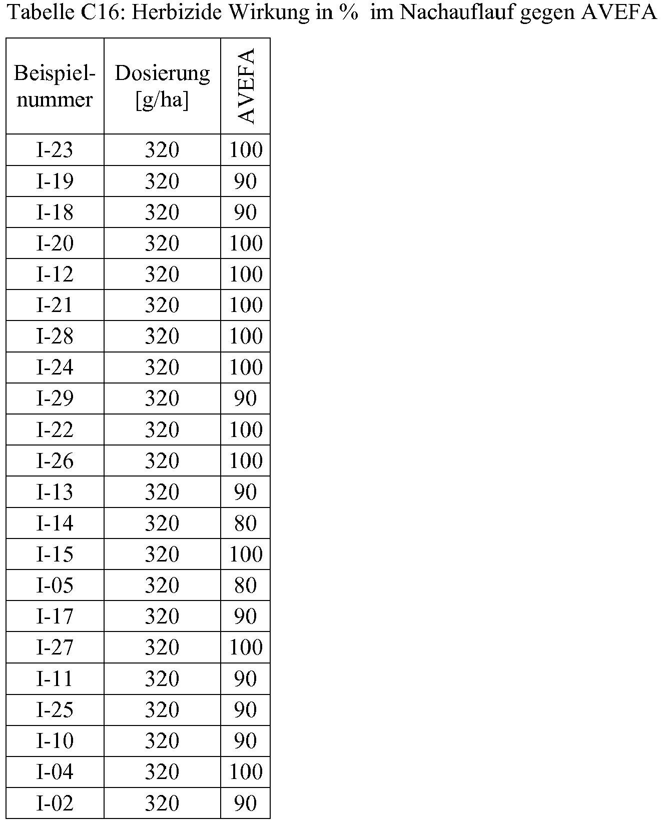

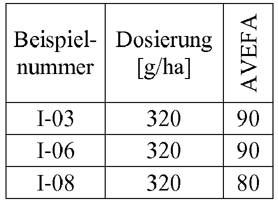

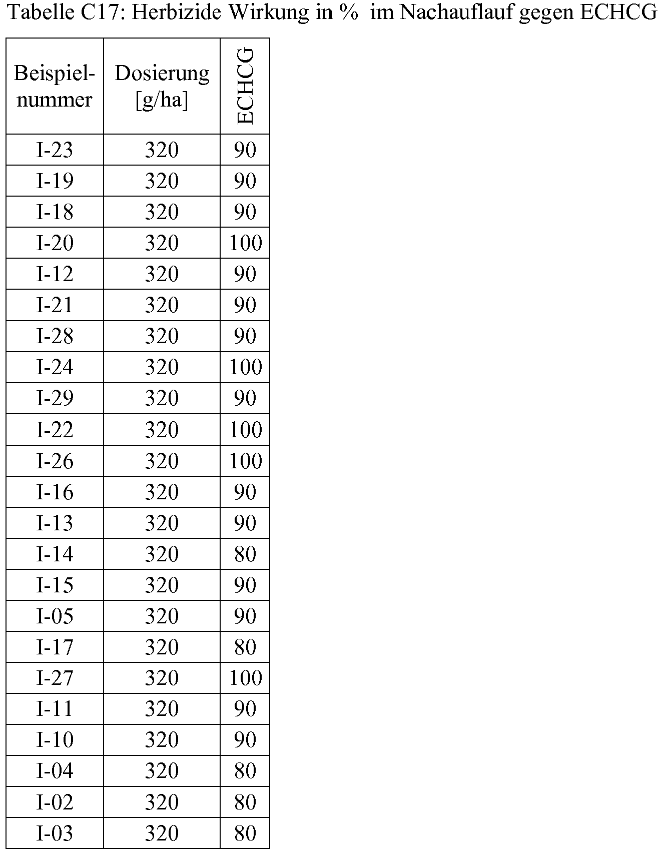

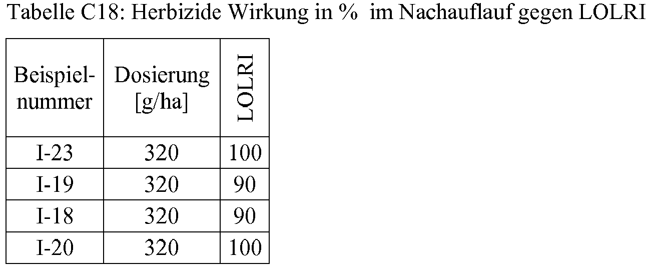

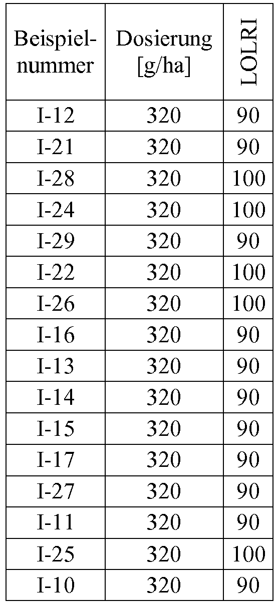

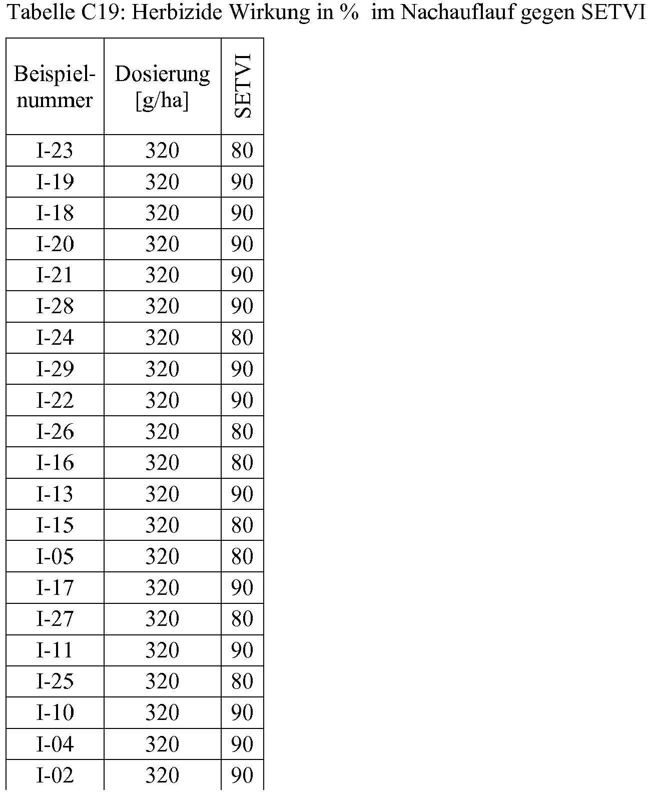

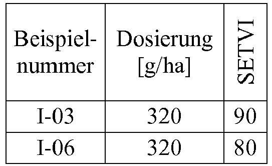

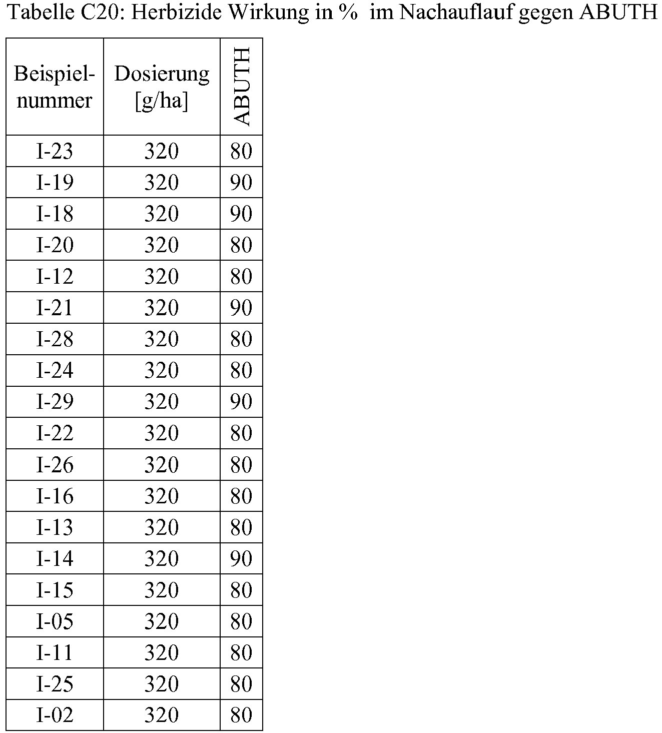

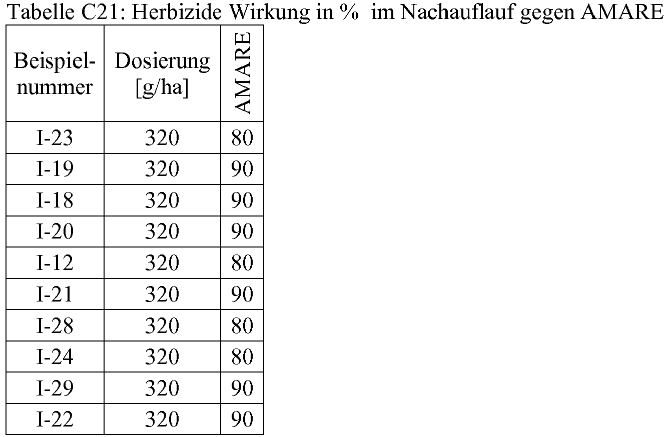

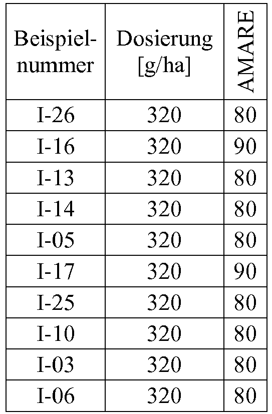

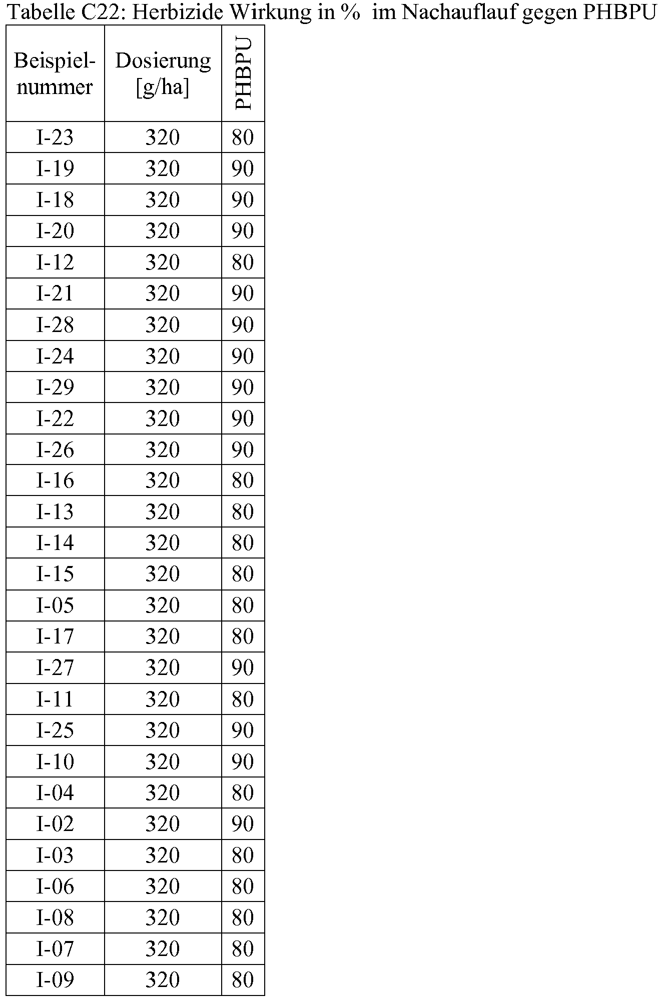

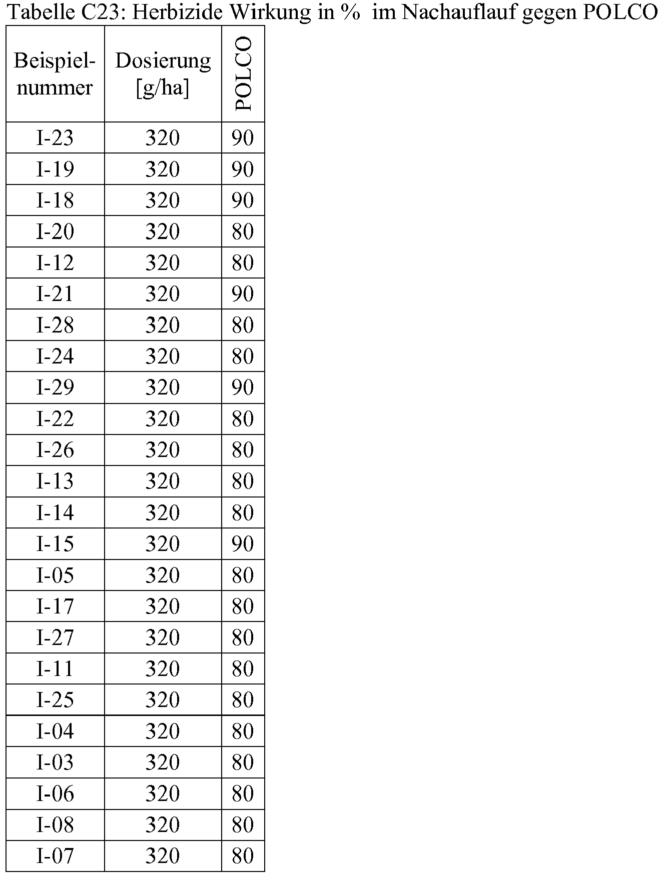

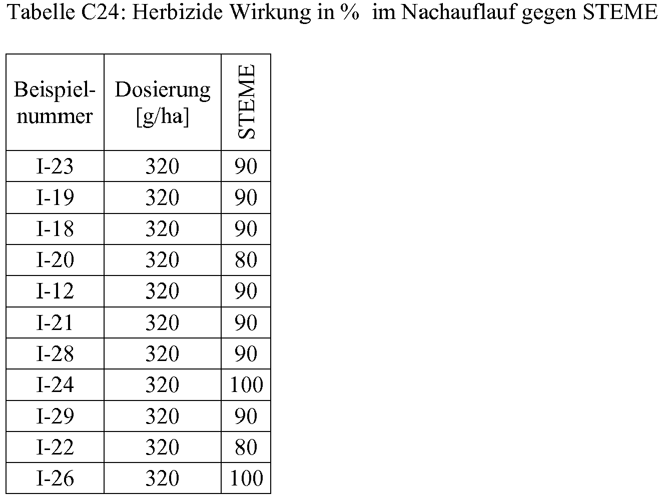

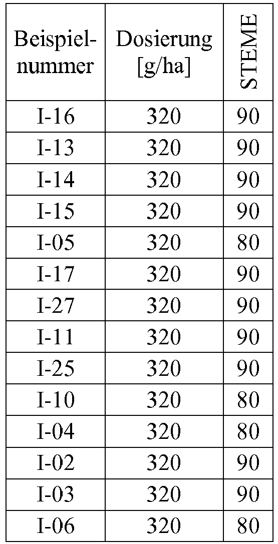

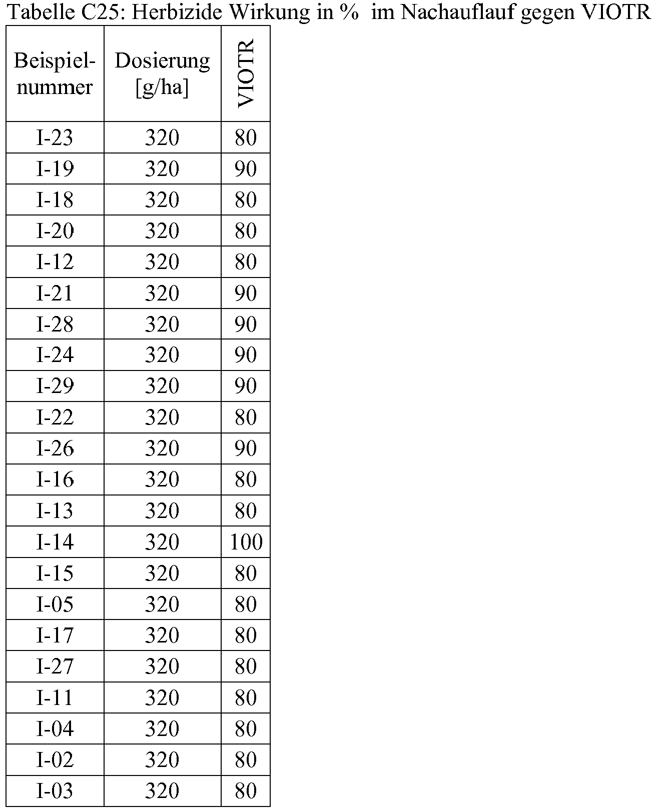

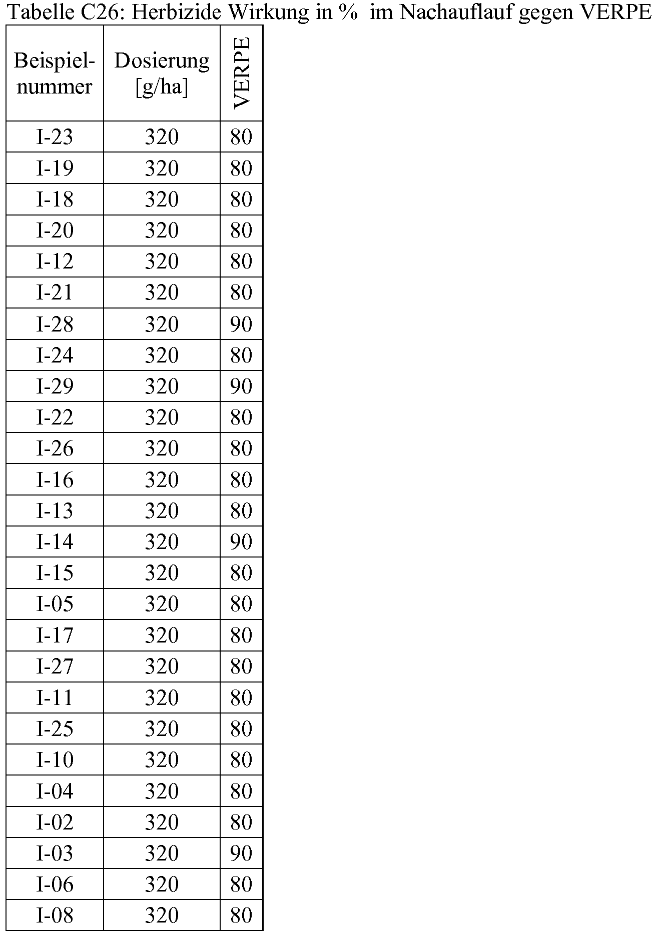

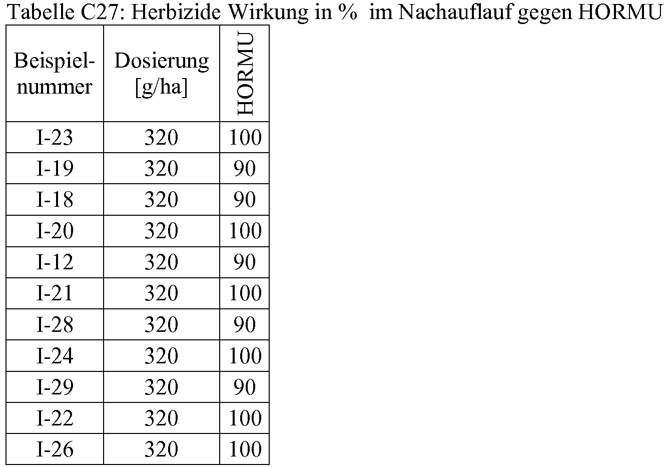

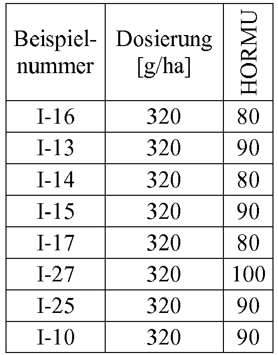

Wie die Ergebnisse zeigen, weisen erfindungsgemäße Verbindungen, wie beispielsweise die Verbindungen Nr. I-02 und andere Verbindungen aus den Tabellen (I-04, I-10, I-15, I-18, I-19, I-20, I-21, I-21, I-22, I-23, I-24, I-26, I-27, I-28, I-29) bei Behandlung im Nachauflauf eine sehr gute Wirkung (80% bis 100% herbizide Wirkung) gegen Schadpflanzen wie *Abutilon theophrasti, Alopecurus myosuroides, Amaranthus retroflexus, Avena fatua, Echinochloa crus-galli, Hordeum murinum, Lolium rigidum, Pharbitis purpurea, Polygonum convolvulus, Setaria viridis, Stellaria media, Veronica persica* und *Viola tricolor* bei einer Aufwandmenge von 0.32 kg Aktivsubstanz oder weniger pro Hektar auf.

## Patentansprüche

1. 3-Phenylisoxazolin-5-carboxamide und -5-thioamide der allgemeinen Formel (I) 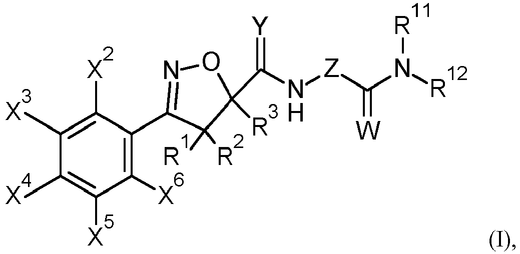 und deren agrochemisch verträglichen Salze, in welchen
R¹ und R² unabhängig voneinander jeweils Wasserstoff, Halogen oder Cyano, oder durch jeweils m Reste aus der Gruppe bestehend aus Halogen und Cyano substituiertes (C₁-C₄)-Alkyl und (C₁-C₄)-Alkoxy bedeuten;
R³ durch jeweils m Reste aus der Gruppe bestehend aus Halogen, Cyano, (C₁-C₅)-Alkoxy und Hydroxy substituiertes (C₁-C₅)-Alkyl, (C₃-C₆)-Cycloalkyl, (C₂-C₅)-Alkenyl, (C₂-C₅)-Alkinyl oder (C₁-C₅)-Alkoxy bedeutet;
Y Sauerstoff oder Schwefel bedeutet;
W Sauerstoff oder Schwefel bedeutet;
Z für einen vollständig gesättigten oder teilweise gesättigten Furanring steht, der mit k Resten der Gruppe R¹⁰ substituiert ist 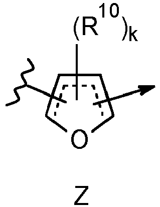 worin der Pfeil jeweils für eine Bindung zur Gruppe C=W der Formel (I) steht;
R¹⁰ Halogen, Cyano oder CO₂R₇ bedeutet, oder durch jeweils m Reste aus der Gruppe bestehend aus Fluor und Chlor substituiertes (C₁-C₂)-Alkyl oder (C₁-C₂)-Alkoxy bedeutet;
R¹¹, R¹² unabhängig voneinander Wasserstoff, Cyano, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, gegebenenfalls substituiertes Aryl, gegebenenfalls substituiertes Heteroaryl und gegebenenfalls substituiertes Heterocyclyl bedeuten, oder durch jeweils m Reste aus der Gruppe bestehend aus Halogen, Cyano, Nitro, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, gegebenenfalls substituiertes Aryl, gegebenenfalls substituiertes Heteroaryl und gegebenenfalls substituiertes Heterocyclyl, substituiertes (C₁-C₁₂)-Alkyl, (C₃-C₈)-Cycloalkyl, (C₃-C₇)-Cycloalkyl-(C₁-C₇)-Alkyl, (C₂-C₁₂)-Alkenyl, (C₅-C₇)-Cycloalkenyl oder (C₂-C₁₂)-Alkinyl bedeuten; oder
R¹¹ und R¹² mit dem Stickstoffatom, an das sie gebunden sind, einen gegebenenfalls ein- bis sechsfach durch Reste aus der Gruppe bestehend aus Halogen, Cyano, Nitro, (C₁-C₆)-Alkyl, Halogen-(C₁-C₆)-Alkyl, Oxo, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸ substituierten, gesättigten, teilweise oder vollständig ungesättigten fünf-, sechs- oder siebengliedrigen Ring bilden, der neben diesem Stickstoffatom r Kohlenstoffatome, n Sauerstoffatome, p Schwefelatome und p Elemente aus der Gruppe bestehend aus NR⁷ und NCOR⁷ als Ringatome enthält;
X², X⁴ und X⁶ unabhängig voneinander jeweils Wasserstoff, Halogen oder Cyano bedeuten, oder jeweils durch m Reste aus der Gruppe bestehend aus Fluor, Chlor, Brom und (C₁-C₂)-Alkoxy substituiertes (C₁-C₂)-Alkyl bedeuten;
X³ und X⁵ unabhängig voneinander Wasserstoff, Fluor, Chlor, Brom, Iod, Hydroxy, Cyano, Nitro, S(O)ₙR⁶ oder CO₂R⁷ bedeuten, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl bedeuten;
R⁵ durch jeweils m Reste aus der Gruppe bestehend aus Halogen, Cyano und Hydroxy substituiertes (C₁-C₈)-Alkyl, (C₃-C₆)-Cycloalkyl oder Aryl bedeutet;
R⁶ Wasserstoff oder R⁵ bedeutet;
R⁷ Wasserstoff, oder durch jeweils m Reste aus der Gruppe bestehend aus Halogen, Cyano und (C₁-C₂)-Alkoxy substituiertes (C₁-C₆)-Alkyl, (C₃-C₆)-Cycloalkyl, (C₃-C₄)-Alkenyl oder (C₃-C₄)-Alkinyl bedeutet;
R⁸ Wasserstoff, oder durch jeweils m Reste aus der Gruppe bestehend aus Halogen, Cyano und (C₁-C₂)-Alkoxy substituiertes (C₁-C₈)-Alkyl, (C₃-C₆)-Cycloalkyl, (C₃-C₈)-Alkenyl oder (C₃-C₈)-Alkinyl bedeutet;
k die Laufzahl 0, 1 oder 2 beträgt;
m die Laufzahl 0, 1, 2, 3, 4 oder 5 beträgt;
n die Laufzahl 0, 1 oder 2 beträgt;
p die Laufzahl 0 oder 1 beträgt; und
r die Laufzahl 3, 4, 5 oder 6 beträgt.

2. Verbindungen der allgemeinen Formel (I) gemäß Anspruch 1, wobei R¹ und R² unabhängig voneinander jeweils Wasserstoff, Fluor, Chlor oder Cyano,
oder
durch jeweils m Reste aus der Gruppe bestehend aus Fluor, Chlor, Brom und Cyano substituiertes (C₁-C₃)-Alkyl oder (C₁-C₃)-Alkoxy bedeuten.

3. Verbindungen der allgemeinen Formel (I) gemäß Anspruch 1 oder 2, wobei R³ durch jeweils m Reste aus der Gruppe bestehend aus Fluor, Chlor, Brom, Cyano, (C₁-C₄)-Alkoxy und Hydroxy substituiertes (C₁-C₄)-Alkyl, (C₃-C₅)-Cycloalkyl, (C₂-C₄)-Alkenyl, (C₂-C₄)-Alkinyl oder (C₁-C₄)-Alkoxy bedeutet.

4. Verbindungen der allgemeinen Formel (I) gemäß einem der Ansprüche 1 bis 3, wobei Y Sauerstoff bedeutet.

5. Verbindungen der allgemeinen Formel (I) gemäß einem der Ansprüche 1 bis 4, wobei W Sauerstoff bedeutet.

6. Verbindungen der allgemeinen Formel (I) gemäß einem der Ansprüche 1 bis 5, wobei Z für eine Gruppe Z-1 bis Z-33 steht, wobei Z-1 bis Z-33 folgende Bedeutung haben: 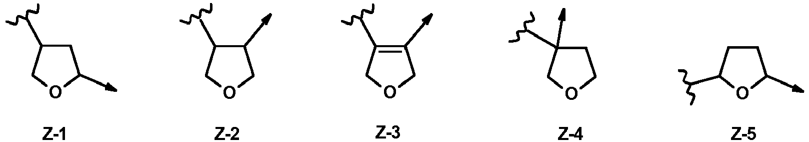 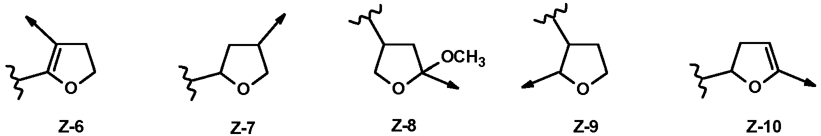 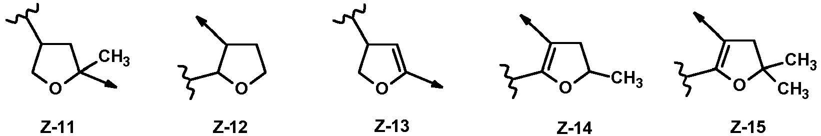 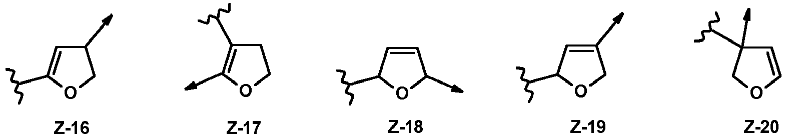 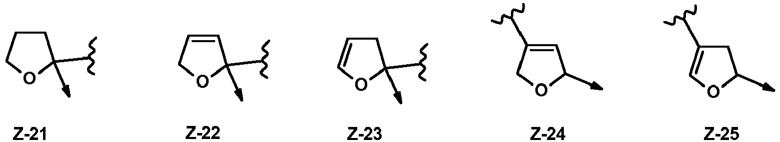 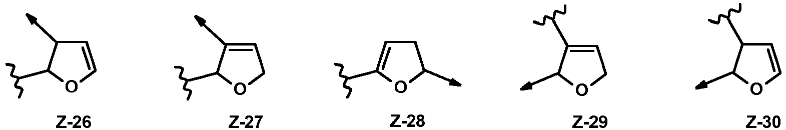 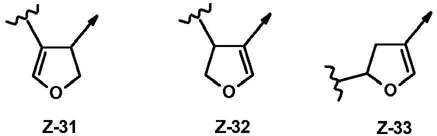 worin der Pfeil jeweils für eine Bindung zur Gruppe C=W der Formel (I) steht.

7. Verbindungen der allgemeinen Formel (I) gemäß einem der Ansprüche 1 bis 6, wobei R¹⁰ Fluor, Chlor, Cyano, CO₂H, CO₂CH₃ oder CO₂CH₂CH₃,
oder
durch jeweils m Reste aus der Gruppe bestehend aus Fluor und Chlor substituiertes (C₁-C₂)-Alkyl oder (C₁-C₂)-Alkoxy bedeutet.

8. Verbindungen der allgemeinen Formel (I) gemäß einem der Ansprüche 1 bis 7, wobei R¹¹ Wasserstoff,
oder
durch jeweils m Reste aus der Gruppe bestehend aus Fluor und Chlor substituiertes (C₁-C₃)-Alkyl oder (C₃-C₆)-Cycloalkyl bedeutet.

9. Verbindungen der allgemeinen Formel (I) gemäß einem der Ansprüche 1 bis 8, wobei R¹² Wasserstoff, Cyano oder Hydroxy,
oder
durch jeweils m Reste aus der Gruppe bestehend aus Fluor, Chlor, Brom, Cyano und Hydroxy substituiertes OR⁷, S(O)ₙR⁵, NR⁶R⁸, NR⁶CO₂R⁸, (C₁-C₆)-Alkyl, (C₃-C₆)-Cycloalkyl, (C₂-C₃)-Alkenyl oder (C₂-C₃)-Alkinyl bedeutet.

10. Verbindungen der allgemeinen Formel (I) gemäß einem der Ansprüche 1 bis 9, wobei R¹¹ und R¹² mit dem Stickstoffatom, an das sie gebunden sind, einen gesättigten, teilweise oder vollständig ungesättigten fünf- oder sechsgliedrigen Ring bilden, der neben diesem Stickstoffatom r Kohlenstoffatome, n Sauerstoffatome und p Elemente aus der Gruppe bestehend aus NR⁷ und NCOR⁷ als Ringatome enthält, wobei ein Kohlenstoffatom p Oxogruppen trägt.

11. Verbindungen der allgemeinen Formel (I) gemäß einem der Ansprüche 1 bis 10, wobei X², X⁴ und X⁶ unabhängig voneinander jeweils Wasserstoff, Fluor, Chlor, Brom oder Cyano,
oder
jeweils durch m Reste aus der Gruppe bestehend aus Fluor und Chlor substituiertes Methyl oder Methoxy bedeuten.

12. Verbindungen der allgemeinen Formel (I) gemäß einem der Ansprüche 1 bis 11,
wobei X³ und X⁵ unabhängig voneinander Wasserstoff, Fluor, Chlor, Brom, Hydroxy oder Cyano,
oder
jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl bedeuten.

13. Verbindungen der allgemeinen Formel (I) gemäß einem der Ansprüche 1 bis 12, wobei R⁵ durch jeweils m Reste aus der Gruppe bestehend aus Fluor, Chlor und Hydroxy substituiertes (C₁-C₆)-Alkyl, (C₃-C₆)-Cycloalkyl oder Aryl bedeutet.

14. Verbindungen der allgemeinen Formel (I) gemäß einem der Ansprüche 1 bis 13, wobei R⁷ Wasserstoff,
oder
durch jeweils m Reste aus der Gruppe bestehend aus Fluor, Chlor und (C₁-C₂)-Alkoxy substituiertes (C₁-C₆)-Alkyl oder (C₃-C₆)-Cycloalkyl bedeutet.

15. Verbindungen der allgemeinen Formel (I) gemäß einem der Ansprüche 1 bis 14, wobei R⁸ Wasserstoff,
oder
durch jeweils m Reste aus der Gruppe bestehend aus Fluor, Chlor und (C₁-C₂)-Alkoxy substituiertes (C₁-C₆)-Alkyl, (C₃-C₆)-Cycloalkyl oder Aryl bedeutet.

16. Verbindungen der allgemeinen Formel (I) gemäß einem der Ansprüche 1 bis 15, wobei die Laufzahl m 0, 1, 2, oder 3 beträgt.

17. Verbindungen der allgemeinen Formel (I) gemäß einem der Ansprüche 1 bis 16, in welchen
R¹ und R² jeweils Wasserstoff bedeuten;
R³ durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy bedeutet;
Y Sauerstoff bedeutet;
W Sauerstoff bedeutet;
Z für eine Gruppe Z-1 bis Z-4 steht, wobei Z-1 bis Z-4 folgende Bedeutung haben: 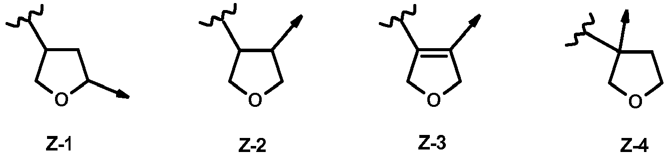
worin der Pfeil jeweils für eine Bindung zur Gruppe C=W der Formel (I) steht;
R¹¹ Wasserstoff, oder durch jeweils m Reste aus der Gruppe bestehend aus Fluor und Chlor substituiertes (C₁-C₃)-Alkyl oder (C₃-C₆)-Cycloalkyl bedeutet;
R¹² Wasserstoff, Cyano oder Hydroxy, oder durch jeweils m Reste aus der Gruppe bestehend aus Fluor, Chlor, Brom, Cyano und Hydroxy substituiertes OR⁷, S(O)ₙR⁵, NR⁶R⁸, NR⁶CO₂R⁸, (C₁-C₆)-Alkyl, (C₃-C₆)-Cycloalkyl, (C₂-C₃)-Alkenyl oder (C₂-C₃)-Alkinyl bedeutet; oder
R¹¹ und R¹² mit dem Stickstoffatom, an das sie gebunden sind, einen gesättigten, teilweise oder vollständig ungesättigten fünf- oder sechsgliedrigen Ring bilden, der neben diesem Stickstoffatom r Kohlenstoffatome, n Sauerstoffatome und p Elemente aus der Gruppe bestehend aus NR⁷ und NCOR⁷ als Ringatome enthält, wobei ein Kohlenstoffatom p Oxogruppen trägt;
X², X⁴ und X⁶ unabhängig voneinander jeweils Wasserstoff oder Fluor bedeuten;
X³ und X⁵ unabhängig voneinander Wasserstoff, Fluor, Chlor, Brom, Hydroxy oder Cyano, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl bedeuten;
R⁵ durch jeweils m Reste aus der Gruppe bestehend aus Fluor, Chlor und Hydroxy substituiertes (C₁-C₆)-Alkyl, (C₃-C₆)-Cycloalkyl oder Aryl bedeutet;
R⁶ Wasserstoff oder R⁵ bedeutet;
R⁷ Wasserstoff, oder durch jeweils m Reste aus der Gruppe bestehend aus Fluor, Chlor und (C₁-C₂)-Alkoxy substituiertes (C₁-C₆)-Alkyl oder (C₃-C₆)-Cycloalkyl bedeutet;
R⁸ Wasserstoff, oder durch jeweils m Reste aus der Gruppe bestehend aus Fluor, Chlor und (C₁-C₂)-Alkoxy substituiertes (C₁-C₆)-Alkyl, (C₃-C₆)-Cycloalkyl oder Aryl bedeutet;
m die Laufzahl 0, 1, 2 oder 3 beträgt;
n die Laufzahl 0, 1 oder 2 beträgt;
p die Laufzahl 0 oder 1 beträgt; und
r die Laufzahl 3, 4, oder 5 beträgt.

18. Herbizides Mittel oder pflanzenwachstumsregulierendes Mittel, **dadurch gekennzeichnet, dass** es eine oder mehrere Verbindungen der allgemeinen Formel (i) oder deren Salze nach einem der Ansprüche 1 bis 17 enthält.

19. Herbizide Mittel nach Anspruch 18, die ferner ein Formulierungshilfsmitteln enthält.

20. Herbizide Mittel nach Anspruch 18 oder 19 enthaltend mindestens einen weiteren Wirkstoff aus der Gruppe der Insektizide, Akarizide, Herbizide, Fungizide, Safenern und/oder Wachstumsregulatoren.

21. Herbizide Mittel nach Anspruch 19 oder 20 enthaltend einen Safener.

22. Herbizide Mittel nach Anspruch 21, worin der Safener ausgewählt ist aus der Gruppe bestehend aus Mefenpyr-diethyl, Cyprosulfamid, Isoxadifen-ethyl, Cloquintocet-mexyl, Benoxacor und Dichlormid.

23. Verfahren zur Bekämpfung unerwünschter Pflanzen, **dadurch gekennzeichnet, dass** man eine wirksame Menge mindestens einer Verbindung der Formel (I) gemäß einem der Ansprüche 1 bis 17 oder eines herbiziden Mittels nach einem der Ansprüche 18 bis 22 auf die Pflanzen oder auf den Ort des unerwünschten Pflanzenwachstums appliziert.

24. Verwendung von Verbindungen der Formel (I) gemäß einem der Ansprüche 1 bis 17 oder von herbiziden Mitteln nach einem der Ansprüche 18 bis 22 zur Bekämpfung unerwünschter Pflanzen.

25. Verwendung nach Anspruch 24, **dadurch gekennzeichnet, dass** die Verbindungen der Formel (I) zur Bekämpfung unerwünschter Pflanzen in Kulturen von Nutzpflanzen eingesetzt werden.

26. Verwendung nach Anspruch 25, **dadurch gekennzeichnet, dass** die Nutzpflanzen transgene Nutzpflanzen sind.

## Claims

1. 3-Phenylisoxazoline-5-carboxamides and -5-thioamides of the general formula (I) 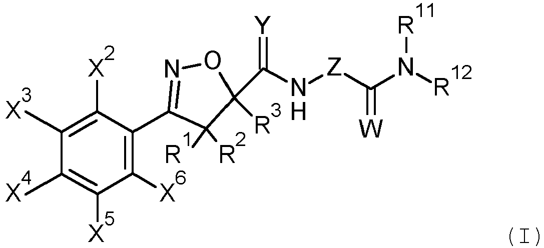 and the agrochemically acceptable salts thereof in which
R¹ and R² independently of one another each represent hydrogen, halogen or cyano, or represent (C₁-C₄)-alkyl or (C₁-C₄) -alkoxy, each of which is substituted by m radicals from the group consisting of halogen and cyano;
R³ represents (C₁-C₅) -alkyl, (C₃-C₆) -cycloalkyl, (C₂-C₅) -alkenyl, (C₂-C₅) -alkynyl or (C₁-C₅)-alkoxy, each of which is substituted by m radicals from the group consisting of halogen, cyano, (C₁-C₅) -alkoxy and hydroxy;
Y represents oxygen or sulfur;
W represents oxygen or sulfur;
Z represents a fully saturated or partially saturated furan ring which is substituted by k radicals from the group R¹⁰ 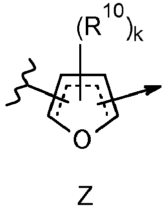 where the arrow in each case denotes a bond to the group C=W of the formula (I);
R¹⁰ represents halogen, cyano or CO₂R₇, or represents (C₁-C₂)-alkyl or (C₁-C₂) -alkoxy, each of which is substituted by m radicals from the group consisting of fluorine and chlorine;
R¹¹, R¹² independently of one another represent hydrogen, cyano, OR⁷, S(O)ₙ R⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, optionally substituted aryl, optionally substituted heteroaryl or optionally substituted heterocyclyl, or represent (C₁-C₁₂) -alkyl, (C₃-C₈)-cycloalkyl, (C₃-C₇) -cycloalkyl- (C₁-C₇)-alkyl, (C₂-C₁₂) -alkenyl, (C₅-C₇)-cycloalkenyl or (C₂-C₁₂) -alkynyl, each of which is substituted by m radicals from the group consisting of halogen, cyano, nitro, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, optionally substituted aryl, optionally substituted heteroaryl or optionally substituted heterocyclyl, or
R¹¹ and R¹² together with the nitrogen atom to which they are attached form a saturated, partially or fully unsaturated five-, six- or seven-membered ring which is optionally mono- to hexasubstituted by radicals from the group consisting of halogen, cyano, nitro, (C₁-C₆) -alkyl, halo- (C₁-C₆) -alkyl, oxo, OR⁷, S(O)ₙ R⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸ and which, in addition to this nitrogen atom, contains r carbon atoms, n oxygen atoms, p sulfur atoms and p elements from the group consisting of NR⁷ and NCOR⁷ as ring atoms;
X², X⁴ and X⁶ independently of one another each represent hydrogen, halogen or cyano,
or
represent (C₁-C₂)-alkyl, in each case substituted by m radicals from the group consisting of fluorine, chlorine, bromine and (C₁-C₂)-alkoxy;
X³ and X⁵ independently of one another represent hydrogen, fluorine, chlorine, bromine, iodine, hydroxy, cyano, nitro, S(O)ₙR⁶ or CO₂R⁷, or represent (C₁-C₃) -alkyl, (C₁-C₃) -alkoxy, (C₃-C₄) -cycloalkyl, (C₂-C₃) -alkenyl or (C₂-C₃)-alkynyl, each of which is substituted by m radicals from the group consisting of fluorine, chlorine and bromine;
R⁵ represents (C₁-C₈) -alkyl, (C₃-C₆) -cycloalkyl or aryl, each of which is substituted by m radicals from the group consisting of halogen, cyano and hydroxy;
R⁶ represents hydrogen or R⁵;
R⁷ represents hydrogen, or represents (C₁-C₆) -alkyl, (C₃-C₆) -cycloalkyl, (C₃-C₄) -alkenyl or (C₃-C₄) -alkynyl, each of which is substituted by m radicals from the group consisting of halogen, cyano and (C₁-C₂)-alkoxy;
R⁸ represents hydrogen, or represents (C₁-C₈) -alkyl, (C₃-C₆) -cycloalkyl, (C₃-C₈) -alkenyl or (C₃-C₈) -alkynyl, each of which is substituted by m radicals from the group consisting of halogen, cyano and (C₁-C₂)-alkoxy;
k is the running number 0, 1 or 2;
m is the running number 0, 1, 2, 3, 4 or 5;
n is the running number 0, 1 or 2;
p is the running number 0 or 1; and
r is the running number 3, 4, 5 or 6.

2. Compounds of the general formula (I) according to Claim 1, where R¹ and R² independently of one another each represent hydrogen, fluorine, chlorine or cyano,
or
represent (C₁-C₃) -alkyl or (C₁-C₃) -alkoxy, each of which is substituted by m radicals from the group consisting of fluorine, chlorine, bromine and cyano.

3. Compounds of the general formula (I) according to Claim 1 or 2, where R³ represents (C₁-C₄)-alkyl, (C₃-C₅) -cycloalkyl, (C₂-C₄) -alkenyl, (C₂-C₄) -alkynyl or (C₁-C₄) -alkoxy, each of which is substituted by m radicals from the group consisting of fluorine, chlorine, bromine, cyano, (C₁-C₄) -alkoxy and hydroxy;

4. Compounds of the general formula (I) according to any of Claims 1 to 3, where Y represents oxygen.

5. Compounds of the general formula (I) according to any of Claims 1 to 4, where W represents oxygen.

6. Compounds of the general formula (I) according to any of Claims 1 to 5, where Z represents a group Z-1 to Z-33, where Z-1 to Z-33 have the following meaning: 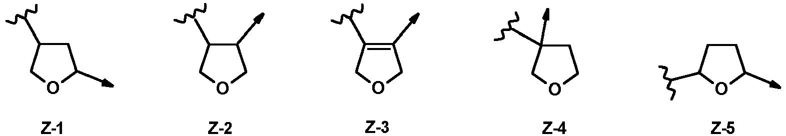 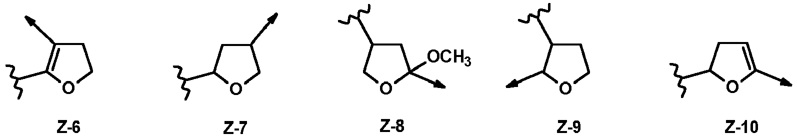 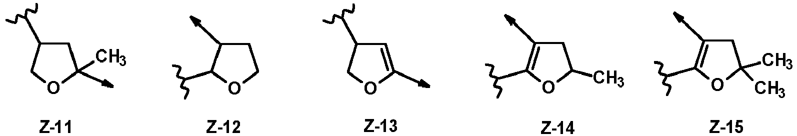 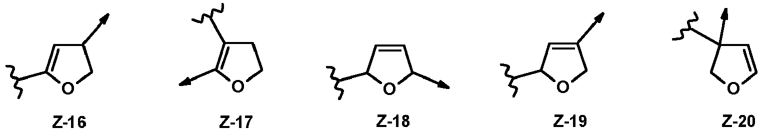 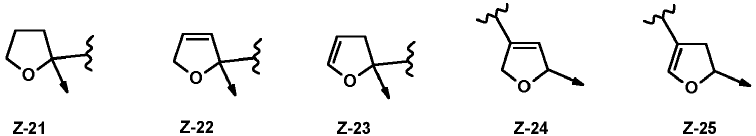 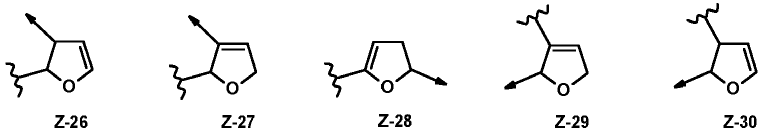 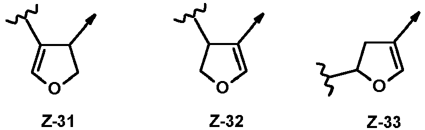 where the arrow in each case denotes a bond to the group C=W of the formula (I).

7. Compounds of the general formula (I) according to any of Claims 1 to 6, where R¹⁰ represents fluorine, chlorine, cyano, CO₂H, CO₂CH₃ or CO₂CH₂CH₃,
or
represents (C₁-C₂) -alkyl or (C₁-C₂) -alkoxy, each of which is substituted by m radicals from the group consisting of fluorine and chlorine.

8. Compounds of the general formula (I) according to any of Claims 1 to 7, where R¹¹ represents hydrogen, or
represents (C₁-C₃)-alkyl or (C₃-C₆)-cycloalkyl, each of which is substituted by m radicals from the group consisting of fluorine and chlorine.

9. Compounds of the general formula (I) according to any of Claims 1 to 8, where R¹² represents hydrogen, cyano or hydroxy,
or
represents OR⁷, S(O)ₙ R⁵, NR⁶R⁸, NR⁶CO₂R⁸, (C₁-C₆) - alkyl, (C₃-C₆) -cycloalkyl, (C₂-C₃) -alkenyl or (C₂-C₃)-alkynyl, each of which is substituted by m radicals from the group consisting of fluorine, chlorine, bromine, cyano and hydroxy.

10. Compounds of the general formula (I) according to any of Claims 1 to 9, where R¹¹ and R¹² together with the nitrogen atom to which they are attached form a saturated, partially or fully unsaturated five- or six-membered ring which, in addition to this nitrogen atom, contains r carbon atoms, n oxygen atoms and p elements from the group consisting of NR⁷ and NCOR⁷ as ring atoms, where a carbon atom carries p oxo groups.

11. Compounds of the general formula (I) according to any of Claims 1 to 10, where X², X⁴ and X⁶ independently of one another each represent hydrogen, fluorine, chlorine, bromine or cyano,
or
represent methyl or methoxy, each of which is substituted by m radicals from the group consisting of fluorine and chlorine.

12. Compounds of the general formula (I) according to any of Claims 1 to 11,
where X³ and X⁵ independently of one another represent hydrogen, fluorine, chlorine, bromine, hydroxy or cyano,
or
represent (C₁-C₃) -alkyl, (C₁-C₃) -alkoxy, (C₃-C₄)-cycloalkyl, (C₂-C₃) -alkenyl or (C₂-C₃) -alkynyl, each of which is substituted by m radicals from the group consisting of fluorine, chlorine and bromine.

13. Compounds of the general formula (I) according to any of Claims 1 to 12, where R⁵ represents (C₁-C₆) - alkyl, (C₃-C₆)-cycloalkyl or aryl, each of which is substituted by m radicals from the group consisting of fluorine, chlorine and hydroxy.

14. Compounds of the general formula (I) according to any of Claims 1 to 13, where R⁷ represents hydrogen, or
represents (C₁-C₆) -alkyl or (C₃-C₆)-cycloalkyl, each of which is substituted by m radicals from the group consisting of fluorine, chlorine and (C₁-C₂) -alkoxy.

15. Compounds of the general formula (I) according to any of Claims 1 to 14, where R⁸ represents hydrogen, or
represents (C₁-C₆) -alkyl, (C₃-C₆) -cycloalkyl or aryl, each of which is substituted by m radicals from the group consisting of fluorine, chlorine and (C₁-C₂) -alkoxy.

16. Compounds of the general formula (I) according to any of Claims 1 to 15, where the running number m is 0, 1, 2 or 3.

17. Compounds of the general formula (I) according to any of Claims 1 to 16 in which
R¹ and R² each represent hydrogen;
R³ represents (C₁-C₃) -alkyl, (C₃-C₄) -cycloalkyl, (C₂-C₃) -alkenyl, (C₂-C₃) -alkynyl or (C₁-C₃)-alkoxy, each of which is substituted by m radicals from the group consisting of fluorine, chlorine, bromine, cyano, (C₁-C₂)-alkoxy and hydroxy;
Y represents oxygen;
W represents oxygen;
Z represents a group Z-1 to Z-4, where Z-1 to Z-4 have the following meaning: 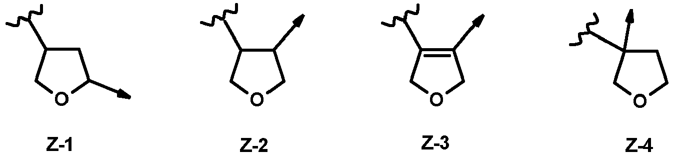
where the arrow in each case denotes a bond to the group C=W of the formula (I);
R¹¹ represents hydrogen, or represents (C₁-C₃) -alkyl or (C₃-C₆) -cycloalkyl, each of which is substituted by m radicals from the group consisting of fluorine and chlorine;
R¹² represents hydrogen, cyano or hydroxy, or represents OR⁷, S(O)ₙ R⁵, NR⁶R⁸, NR⁶CO₂R⁸, (C₁-C₆) -alkyl, (C₃-C₆) -cycloalkyl, (C₂-C₃) -alkenyl or (C₂-C₃) -alkynyl, each of which is substituted by m radicals from the group consisting of fluorine, chlorine, bromine, cyano and hydroxy; or
R¹¹ and R¹² together with the nitrogen atom to which they are attached form a saturated, partially or fully unsaturated five- or six-membered ring which, in addition to this nitrogen atom, contains r carbon atoms, n oxygen atoms and p elements from the group consisting of NR⁷ and NCOR⁷ as ring atoms, where a carbon atom carries p oxo groups;
X², X⁴ and X⁶ independently of one another each represent hydrogen or fluorine;
X³ and X⁵ independently of one another represent hydrogen, fluorine, chlorine, bromine, hydroxy or cyano, or represent (C₁-C₃) -alkyl, (C₁-C₃) -alkoxy, (C₃-C₄) -cycloalkyl, (C₂-C₃) -alkenyl or (C₂-C₃)-alkynyl, each of which is substituted by m radicals from the group consisting of fluorine, chlorine and bromine;
R⁵ represents (C₁-C₆) -alkyl, (C₃-C₆) -cycloalkyl or aryl, each of which is substituted by m radicals from the group consisting of fluorine, clorine and hydroxy;
R⁶ represents hydrogen or R⁵;
R⁷ represents hydrogen, or represents (C₁-C₆)-alkyl or (C₃-C₆)-cycloalkyl, each of which is substituted by m radicals from the group consisting of fluorine, chlorine and (C₁-C₂) -alkoxy;
R⁸ represents hydrogen, or represents (C₁-C₆) -alkyl, (C₃-C₆) -cycloalkyl or aryl, each of which is substituted by m radicals from the group consisting of fluorine, chlorine and (C₁-C₂)-alkoxy;
m is the running number 0, 1, 2 or 3;
n is the running number 0, 1 or 2;
p is the running number 0 or 1; and
r is the running number 3, 4 or 5.

18. Herbicidal composition or plant growth-regulating composition, **characterized in that** it comprises one or more compounds of the general formula (i) or salts thereof according to any of Claims 1 to 17.

19. Herbicidal compositions according to Claim 18, further comprising a formulation auxiliary.

20. Herbicidal compositions according to Claim 18 or 19, comprising at least one further active compound from the group of insecticides, acaricides, herbicides, fungicides, safeners and/or growth regulators.

21. Herbicidal compositions according to Claim 19 or 20, comprising a safener.

22. Herbicidal compositions according to Claim 21, in which the safener is selected from the group consisting of mefenpyr-diethyl, cyprosulfamide, isoxadifen-ethyl, cloquintocet-mexyl, benoxacor and dichlormid.

23. Method of controlling unwanted plants, **characterized in that** an effective amount of at least one compound of the formula (I) according to any of Claims 1 to 17 or of a herbicidal composition according to any of Claims 18 to 22 is applied to the plants or to the site of the unwanted vegetation.

24. Use of compounds of the formula (I) according to any of Claims 1 to 17 or of herbicidal compositions according to any of Claims 18 to 22 for controlling unwanted plants.

25. Use according to Claim 24, **characterized in that** the compounds of the formula (I) are used for controlling unwanted plants in crops of useful plants.

26. Use according to Claim 25, **characterized in that** the useful plants are transgenic useful plants.

## Revendications

1. 3-Phénylisoxazolin-5-carboxamides et 3-phénylisoxazolin-5-thioamides de formule générale (I), 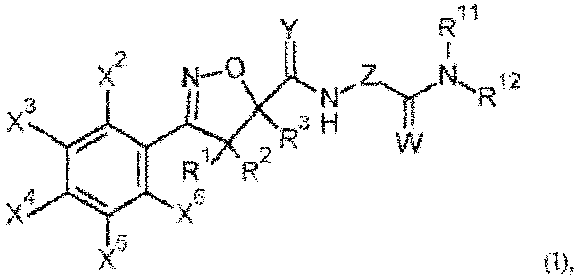 et leurs sels acceptables sur le plan agrochimique, dans lesquels
R¹ et R² signifient indépendamment l'un de l'autre à chaque fois hydrogène, halogène ou cyano, ou (C₁-C₄)-alkyle et (C₁-C₄)-alcoxy substitués par à chaque fois m radicaux du groupe constitué par halogène et cyano ;
R³ signifie (C₁-C₅) -alkyle, (C₃-C₆) -cycloalkyle, (C₂-C₅) -alcényle, (C₂-C₅) -alcynyle ou (C₁-C₅) -alcoxy substitué par à chaque fois m radicaux du groupe constitué par halogène, cyano, (C₁-C₅) -alcoxy et hydroxy ;
Y signifie oxygène ou soufre ;
W signifie oxygène ou soufre ;
Z représente un cycle furanne totalement saturé ou partiellement saturé, qui est substitué par k radicaux du groupe R¹⁰ 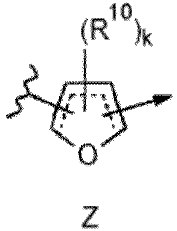 dans lequel la flèche représente à chaque fois une liaison au groupe C=W de la formule (I) ;
R¹⁰ signifie halogène, cyano ou CO₂R₇, ou (C₁-C₂)-alkyle ou (C₁-C₂) -alcoxy substitué par à chaque fois m radicaux du groupe constitué par fluor et chlore ;
R¹¹, R¹² signifient indépendamment l'un de l'autre hydrogène, cyano, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, aryle éventuellement substitué, hétéroaryle éventuellement substitué et hétérocyclyle éventuellement substitué, ou (C₁-C₁₂) -alkyle, (C₃-C₈) -cycloalkyle, (C₃-C₇)-cycloalkyl- (C₁-C₇) -alkyle, (C₂-C₁₂) -alcényle, (C₅-C₇) -cycloalcényle ou (C₂-C₁₂) -alcynyle substitué à chaque fois par m radicaux du groupe constitué par halogène, cyano, nitro, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, aryle éventuellement substitué, hétéroaryle éventuellement substitué et hétérocyclyle éventuellement substitué ; ou
R¹¹ et R¹² forment avec l'atome d'azote auquel ils sont liés, un cycle à cinq, six ou sept chaînons saturé, partiellement ou totalement insaturé éventuellement substitué une à six fois par des radicaux du groupe constitué par halogène, cyano, nitro, (C₁-C₆)-alkyle, halogéno- (C₁-C₆) -alkyle, oxo, OR⁷, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸, qui, outre cet atome d'azote, contient r atomes de carbone, n atomes d'oxygène, p atomes de soufre et p éléments choisis dans le groupe constitué par NR⁷ et NCOR⁷ en tant qu'atomes de cycle ;
X², X⁴ et X⁶ signifient indépendamment les uns des autres à chaque fois hydrogène, halogène ou cyano, ou (C₁-C₂)-alkyle substitué à chaque fois par m radicaux du groupe constitué par fluor, chlore, brome et (C₁-C₂)-alcoxy ;
X³ et X⁵ signifient indépendamment l'un de l'autre hydrogène, fluor, chlore, brome, iode, hydroxy, cyano, nitro, S(O)ₙR⁶ ou CO₂R⁷, ou (C₁-C₃) -alkyle, (C₁-C₃) -alcoxy, (C₃-C₄)-cycloalkyle, (C₂-C₃) -alcényle ou (C₂-C₃) -alcynyle substitué à chaque fois par m radicaux du groupe constitué par fluor, chlore et brome ;
R⁵ signifie (C₁-C₈) -alkyle, (C₃-C₆) -cycloalkyle ou aryle substitué par à chaque fois m radicaux du groupe constitué par halogène, cyano et hydroxy ;
R⁶ signifie hydrogène ou R⁵ ;
R⁷ signifie hydrogène, ou (C₁-C₆)-alkyle, (C₃-C₆)-cycloalkyle, (C₃-C₄)-alcényle ou (C₃-C₄)-alcynyle substitué à chaque fois par m radicaux du groupe constitué par halogène, cyano et (C₁-C₂)-alcoxy ;
R⁸ signifie hydrogène, ou (C₁-C₈)-alkyle, (C₃-C₆)-cycloalkyle, (C₃-C₈)-alcényle ou (C₃-C₈) -alcynyle substitué à chaque fois par m radicaux du groupe constitué par halogène, cyano et (C₁-C₂)-alcoxy ;
k est l'indice 0, 1 ou 2 ;
m est l'indice 0, 1, 2, 3, 4 ou 5 ;
n est l'indice 0, 1 ou 2 ;
p est l'indice 0 ou 1 ; et
r est l'indice 3, 4, 5 ou 6.

2. Composés de formule générale (I) selon la revendication 1, R¹ et R² signifiant indépendamment l'un de l'autre à chaque fois hydrogène, fluor, chlore ou cyano,
ou
(C₁-C₃) -alkyle ou (C₁-C₃) -alcoxy substitué par à chaque fois m radicaux du groupe constitué par fluor, chlore, brome et cyano.

3. Composés de formule générale (I) selon la revendication 1 ou 2, R³ signifiant (C₁-C₄)-alkyle, (C₃-C₅) -cycloalkyle, (C₂-C₄) -alcényle, (C₂-C₄)-alcynyle ou (C₁-C₄)-alcoxy substitué par à chaque fois m radicaux du groupe constitué par fluor, chlore, brome, cyano, (C₁-C₄)-alcoxy et hydroxy.

4. Composés de formule générale (I) selon l'une quelconque des revendications 1 à 3, Y signifiant oxygène.

5. Composés de formule générale (I) selon l'une quelconque des revendications 1 à 4, W signifiant oxygène.

6. Composés de formule générale (I) selon l'une quelconque des revendications 1 à 5, Z représentant un groupe Z-1 à Z-33, Z-1 à Z-33 possédant la signification suivante : 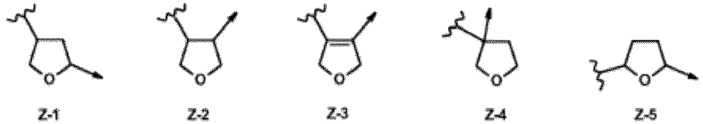 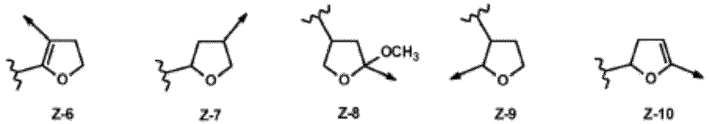 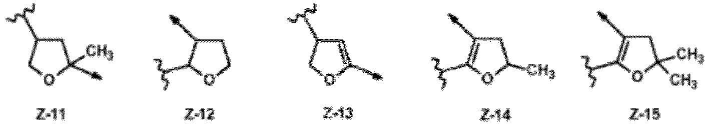 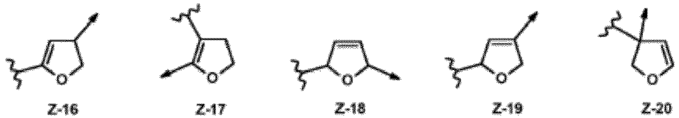 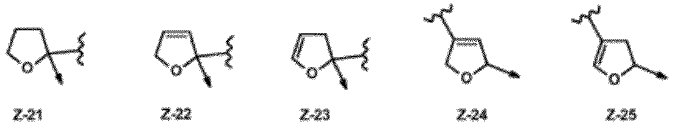 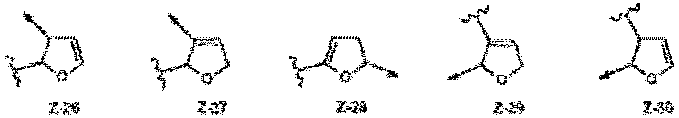 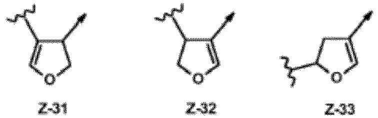 dans lesquels la flèche représente à chaque fois une liaison au groupe C=W de la formule (I).

7. Composés de formule générale (I) selon l'une quelconque des revendications 1 à 6, R¹⁰ signifiant fluor, chlore, cyano, CO₂H, CO₂CH₃ ou CO₂CH₂CH₃,
ou
(C₁-C₂)-alkyle ou (C₁-C₂)-alcoxy substitué à chaque fois par m radicaux du groupe constitué par fluor et chlore.

8. Composés de formule générale (I) selon l'une quelconque des revendications 1 à 7, R¹¹ signifiant hydrogène,
ou
(C₁-C₃) -alkyle ou (C₃-C₆)-cycloalkyle substitué à chaque fois par m radicaux du groupe constitué par fluor et chlore.

9. Composés de formule générale (I) selon l'une quelconque des revendications 1 à 8, R¹² signifiant hydrogène, cyano ou hydroxy,
ou
OR⁷, S(O)ₙR⁵, NR⁶R⁸, NR⁶CO₂R⁸, (C₁-C₆)-alkyle, (C₃-C₆)-cycloalkyle, (C₂-C₃)-alcényle ou (C₂-C₃)-alcynyle substitué à chaque fois par m radicaux du groupe constitué par fluor, chlore, brome, cyano et hydroxy.

10. Composés de formule générale (I) selon l'une quelconque des revendications 1 à 9, R¹¹ et R¹² formant avec l'atome d'azote auquel ils sont liés, un cycle à cinq ou six chaînons saturé, partiellement ou totalement insaturé, qui, outre cet atome d'azote, contient r atomes de carbone, n atomes d'oxygène et p éléments choisis dans le groupe constitué par NR⁷ et NCOR⁷ en tant qu'atomes de cycle, un atome de carbone portant p groupes oxo.

11. Composés de formule générale (I) selon l'une quelconque des revendications 1 à 10, X², X⁴ et X⁶ signifiant indépendamment les uns des autres à chaque fois hydrogène, fluor, chlore, brome ou cyano,
ou
méthyle ou méthoxy substitué à chaque fois par m radicaux du groupe constitué par fluor et chlore.

12. Composés de formule générale (I) selon l'une quelconque des revendications 1 à 11, X³ et X⁵ signifiant indépendamment l'un de l'autre hydrogène, fluor, chlore, brome, hydroxy ou cyano,
ou
(C₁-C₃)-alkyle, (C₁-C₃)-alcoxy, (C₃-C₄)-cycloalkyle, (C₂-C₃)-alcényle ou (C₂-C₃)-alcynyle substitué à chaque fois par m radicaux du groupe constitué par fluor, chlore et brome.

13. Composés de formule générale (I) selon l'une quelconque des revendications 1 à 12, R⁵ signifiant (C₁-C₆)-alkyle, (C₃-C₆)-cycloalkyle ou aryle substitué par à chaque fois m radicaux du groupe constitué par fluor, chlore et hydroxy.

14. Composés de formule générale (I) selon l'une quelconque des revendications 1 à 13, R⁷ signifiant hydrogène,
ou
(C₁-C₆)-alkyle ou (C₃-C₆)-cycloalkyle substitué par à chaque fois m radicaux du groupe constitué par fluor, chlore et (C₁-C₂)-alcoxy.

15. Composés de formule générale (I) selon l'une quelconque des revendications 1 à 14, R⁸ signifiant hydrogène,
ou
(C₁-C₆)-alkyle, (C₃-C₆)-cycloalkyle ou aryle substitué par à chaque fois m radicaux du groupe constitué par fluor, chlore et (C₁-C₂)-alcoxy.

16. Composés de formule générale (I) selon l'une quelconque des revendications 1 à 15, l'indice m étant 0, 1, 2 ou 3.

17. Composés de formule générale (I) selon l'une quelconque des revendications 1 à 16, dans lesquels R¹ et R² signifient à chaque fois hydrogène ;
R³ signifie (C₁-C₃)-alkyle, (C₃-C₄)-cycloalkyle, (C₂-C₃) -alcényle, (C₂-C₃)-alcynyle ou (C₁-C₃)-alcoxy substitué par à chaque fois m radicaux du groupe constitué par fluor, chlore, brome, cyano, (C₁-C₂)-alcoxy et hydroxy ;
Y signifie oxygène ;
W signifie oxygène ;
Z représente un groupe Z-1 à Z-4, Z-1 à Z-4 possédant la signification suivante : 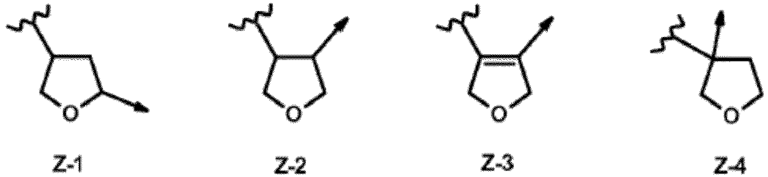 dans lesquels la flèche représente à chaque fois une liaison au groupe C=W de la formule (I) ;
R¹¹ signifie hydrogène, ou (C₁-C₃)-alkyle ou (C₃-C₆)-cycloalkyle substitué par à chaque fois m radicaux du groupe constitué par fluor et chlore ;
R¹² signifie hydrogène, cyano ou hydroxy, ou OR⁷, S(O)ₙR⁵, NR⁶R⁸, NR⁶CO₂R⁸, (C₁-C₆)-alkyle, (C₃-C₆)-cycloalkyle, (C₂-C₃)-alcényle ou (C₂-C₃)-alcynyle substitué à chaque fois par m radicaux du groupe constitué par fluor, chlore, brome, cyano et hydroxy ; ou
R¹¹ et R¹² forment avec l'atome d'azote auquel ils sont liés, un cycle à cinq ou six chaînons saturé, partiellement ou totalement insaturé, qui, outre cet atome d'azote, contient r atomes de carbone, n atomes d'oxygène et p éléments choisis dans le groupe constitué par NR⁷ et NCOR⁷ en tant qu'atomes de cycle, un atome de carbone portant p groupes oxo ;
X², X⁴ et X⁶ signifient indépendamment les uns des autres à chaque fois hydrogène ou fluor ;
X³ et X⁵ signifient indépendamment l'un de l'autre hydrogène, fluor, chlore, brome, hydroxy ou cyano, ou (C₁-C₃)-alkyle, (C₁-C₃)-alcoxy, (C₃-C₄)-cycloalkyle, (C₂-C₃)-alcényle ou (C₂-C₃)-alcynyle substitué à chaque fois par m radicaux du groupe constitué par fluor, chlore et brome ;
R⁵ signifie (C₁-C₆)-alkyle, (C₃-C₆)-cycloalkyle ou aryle substitué par à chaque fois m radicaux du groupe constitué par fluor, chlore et hydroxy ;
R⁶ signifie hydrogène ou R⁵ ;
R⁷ signifie hydrogène, ou (C₁-C₆)-alkyle ou (C₃-C₆)-cycloalkyle substitué à chaque fois par m radicaux du groupe constitué par fluor, chlore et (C₁-C₂)-alcoxy ;
R⁸ signifie hydrogène, ou (C₁-C₆)-alkyle, (C₃-C₆)-cycloalkyle ou aryle substitué à chaque fois par m radicaux du groupe constitué par fluor, chlore et (C₁-C₂)-alcoxy ;
m est l'indice 0, 1, 2, ou 3 ;
n est l'indice 0, 1 ou 2 ;
p est l'indice 0 ou 1 ; et
r est l'indice 3, 4, ou 5.

18. Agent herbicide ou agent de régulation de la croissance végétale, **caractérisé en ce qu'**il contient un ou plusieurs composés de formule générale (i) ou leurs sels selon l'une quelconque des revendications 1 à 17.

19. Agents herbicides selon la revendication 18, qui contiennent en outre un auxiliaire de formulation.

20. Agents herbicides selon la revendication 18 ou 19 contenant au moins un autre principe actif du groupe des insecticides, des acaricides, des herbicides, des fongicides, des phytoprotecteurs et/ou des régulateurs de croissance.

21. Agents herbicides selon la revendication 19 ou 20 contenant un phytoprotecteur.

22. Agents herbicides selon la revendication 21, dans lesquels le phytoprotecteur est choisi dans le groupe constitué par le méfenpyr-diéthyle, le cyprosulfamide, l'isoxadifen-éthyle, le cloquintocet-mexyle, le bénoxacor et le dichlormide.

23. Procédé pour la lutte contre des végétaux indésirables, **caractérisé en ce qu'**on applique une quantité efficace d'au moins un composé de formule (I) selon l'une quelconque des revendications 1 à 17 ou d'un agent herbicide selon l'une quelconque des revendications 18 à 22 sur les végétaux ou sur le lieu de croissance végétale indésirable.

24. Utilisation de composés de formule (I) selon l'une quelconque des revendications 1 à 17 ou d'agents herbicides selon l'une quelconque des revendications 18 à 22 pour la lutte contre des végétaux indésirables.

25. Utilisation selon la revendication 24, **caractérisée en ce que** les composés de formule (I) sont utilisés pour la lutte contre des végétaux indésirables dans des cultures de végétaux utiles.

26. Utilisation selon la revendication 25, **caractérisée en ce que** les végétaux utiles sont des végétaux utiles transgéniques.
